# EUROPEAN PATENT APPLICATION

(11) **EP 1 566 379 A1**
(43) Date of publication of application: **24.08.2005**
(21) Application number: 03769958.4
(22) Date of filing: 29.10.2003
(51) Int. Cl.: C07D 215/22, C07D 239/88, C07D 401/12, C07D 413/12, A61K 31/47, A61K 31/4709, A61K 31/496, A61K 31/517, A61K 31/5377, A61K 31/55, A61P 35/00, A61P 35/02, A61P 37/02, A61P 43/00

(54) **QUINOLINE DERIVATIVES AND QUINAZOLINE DERIVATIVES INHIBITING AUTOPHOSPHORYLATION OF Flt3 AND MEDICINAL COMPOSITIONS CONTAINING THE SAME**

(30) Priority: 29.10.2002 JP 2002314670
(71) Applicant: KIRIN BEER KABUSHIKI KAISHA, Tokyo 104-8288 (JP)
(72) Inventor: MIWA, Atsushi, c/o Kirin Beer Kabushiki Kaisha, Takasaki-shi, Gunma 370-1295 (JP); YOSHINO, Tetsuya, c/o University of Tokyo Hospital, Tokyo 113-8655 (JP); KUROKAWA, Mineo, c/o University of Tokyo Hospital, Tokyo 113-8655 (JP); HIRAI, Hisamaru
(74) Representative: HOFFMANN EITLE
(86) International application number: PCT/JP2003/013848
(87) International publication number: WO 2004/039782

(57) **Abstract**

An objective of the present invention is to provide compounds and pharmaceticals useful for the treatment of disease where the inhibition of autophosphorylation of FMS-like tyrosine kinase 3(Flt3) is therapeutically effective. The present invention relates to a pharmaceutical composition for use in the treatment or prevention of diseases where the inhibition of autophosphorylation of Flt3 therapeutically or prophylactically effective, which comprises a compound represented by formula (I) or a pharmaceutically acceptable salt or solvate thereof: 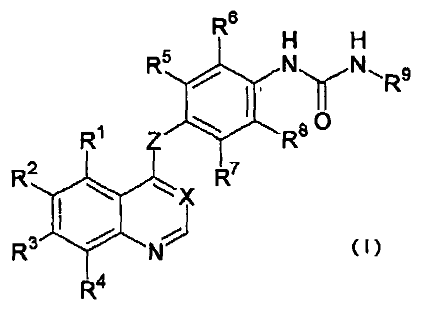 wherein X represents CH or N; Z represents O or S; R¹, R², and R³ represent H, OH, or optionally substituted alkoxy; R⁴ represents H; R⁵, R⁶, R⁷, and R⁸ represent H, Hal, alkyl or the like; and R⁹ represents, e.g., alkyl substituted by t-butyl or the like.

## Description

### BACKGROUND OF THE INVENTION

### Field of the Invention

The present invention relates to quinoline derivatives and quinazoline derivatives for inhibiting the autophosphorylation of Flt3. More particularly, the present invention relates to quinoline derivatives and quinazoline derivatives useful for the treatment of hematopoietic malignancies such as acute myelocytic leukemia and myelodysplastic syndrome, and immunological diseases caused by abnormal proliferation of B cells, dendritic cells, or natural killer cells.

### Background Art

Various intracellular signal transduction systems are involved in controlling cell growth and differentiation. In general, the intracellular signal transduction systems are known to be activated by binding of certain growth factors to receptors on the surface of cell membranes. Such intracellular signal transduction occurred through the activation of receptor-type tyrosine kinase. FMS-like tyrosine kinase 3 (hereinafter referred to as "Flt3"), together with KIT, FMS, and PDGF receptors or the like, is one of proteins belonging to class III of receptor-type tyrosine kinases and is considered to be involved in a hematopoietic system.

Small molecule compounds which inhibit the autophosphorylation of Flt3 were reported in E. Weisberg et al., Cancer Cell, 2002, 1, 433; Kumagai et al., The Japanese Cancer Association (60th) Abst. 611 (2001. 9); Mark Levis et al., Blood, 2002, 99, 3885; Mark Levis et al., Blood, 2001, 98, 885; K-F. Tse et al., Leulemia, 2001, 15, 1001; Louise M. Kelly et al., Cancer Cell, 2002, 1, 421; and WO03/33472.

### SUMMARY OF THE INVENTION

The present inventors have now found that a certain group of quinoline derivatives and quinazoline derivatives have inhibitory activity against autophosphorylation of Flt3 and/or autophosphorylation of somatic cell variants of Flt3 (hereinafter referred to as "Flt3-ITD"). The present inventors have further found that a certain group of quinoline derivatives and quinazoline derivatives have antiproliferative effect against certain Flt3 expressing cells and/or Flt3-ITD expressing cells. The present inventors have also found that a certain group of quinoline derivatives and quinazoline derivatives have antitumor effects against in nonhuman animals to which certain Flt3 expressing cancer cells and/or Flt3-ITD expressing cancer cells have been transplanted. Some of the quinoline derivatives and quinazoline derivatives showed excellent physical stability and/or excellent water solubility and/or metabolic stability. The present invention has been made based on such finding.

An object of the present invention is to provide compounds and pharmaceuticals useful for the treatment and prevention of diseases where the inhibition of autophosphorylatinon of Flt3 and/or Flt3-ITD is therapeutically or prophylactically effective.

According to the present invention, there is provided a pharmaceutical composition for use in the treatment or prevention of diseases where the inhibition of autophosphorylation of FMS-like tyrosine kinase 3 (Flt3) is therapeutically or prophylactically effective, which comprises a compound represented by formula (I) or a pharmaceutically acceptable salt or solvate thereof: 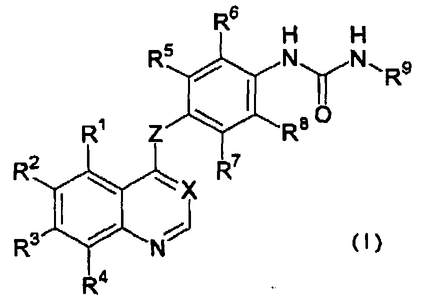 wherein
X represents CH or N,
Z represents O or S,
R¹, R², and R³, which may be the same or different, represent
a hydrogen atom,
hydroxyl,
a halogen atom,
nitro,
cyano,
amino,
C₁₋₆ alkyl,
C₂₋₆ alkenyl,
C₂₋₆ alkynyl,
C₁₋₆ alkoxy,
-(C=O)OR^{c} wherein R^{c} represents a hydrogen atom or C₁₋₄ alkyl, or
-(C=O)NR^{d}R^{e} wherein R^{d} and R^{e}, which may be the same or different, represent a hydrogen atom or C₁₋₄ alkyl,
the C₁₋₆ alkyl, C₂₋₆ alkenyl, C₂₋₆ alkynyl, and C₁₋₆ alkoxy groups, which may be represented by R¹, R², and R³, are optionally substituted by hydroxyl; a halogen atom; C₁₋₆ alkoxy; C₁₋₆ alkylcarbonyl; carboxyl; C₁₋₆ alkoxycarbonyl; -(C=O)-NR¹⁰R¹¹ wherein R¹⁰ and R¹¹, which may be the same or different, represent a hydrogen atom or C₁₋₄ alkyl optionally substituted by hydroxyl, or R¹⁰ and R¹¹ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, a halogen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic group;
one or two hydrogen atoms on the amino group, which may be represented by R¹, R², and R³, are optionally substituted by C₁₋₆ alkyl which is further optionally substituted by hydroxyl or C₁₋₆ alkoxy;
R⁴ represents a hydrogen atom;
all of R⁵, R⁶, R⁷, and R⁸ represent a hydrogen atom, or any one or two of R⁵, R⁶, R⁷, and R⁸ represent a halogen atom, C₁₋₄ alkyl, C₁₋₄ alkoxy, nitro, amino, or hydroxyl with all the remaining groups representing a hydrogen atom, and
R⁹ represents C₁₋₄ alkyl substituted by a substituent selected from the group consisting of a saturated three- to nine-membered (preferably three- to seven-membered) carbocyclic group optionally substituted by C₁₋₄ alkyl, C₁₋₄ alkoxy, or hydroxyl; i-propyl optionally substituted by C₁₋₄ alkyl, C₁₋₄ alkoxy, or hydroxyl; t-butyl optionally substituted by C₁₋₄ alkyl, C₁₋₄ alkoxy, or hydroxyl; C₁₋₄ alkoxy; and -NR^{a}R^{b} wherein R^{a} and R^{b}, which may be the same or different, represent a hydrogen atom or C₁₋₄ alkyl optionally substituted by hydroxyl, or R^{a} and R^{b} may combine with a nitrogen atom attached thereto to form a saturated five- or six-membered heterocyclic group, or R⁹ represents a saturated three- to nine-membered (preferably five- to seven-membered) carbocyclic group optionally substituted by one to three C₁₋₄ alkyl group.

Further, according to the present invention, there are provided compounds represented by formula (Ia) or pharmaceutically acceptable salts or solvates thereof: 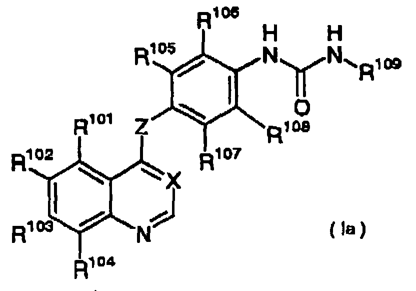 wherein
X represents CH or N,
Z represents O or S,
R¹⁰¹ and R¹⁰⁴ represent a hydrogen atom,
R¹⁰² and R¹⁰³, which may be the same or different, represent
a hydrogen atom,
hydroxyl,
a halogen atom,
nitro,
cyano,
-NR¹¹¹R¹¹² wherein R¹¹¹ and R¹¹², which may be the same or different, represent a hydrogen atom or C₁₋₄ alkyl,
-(C=O)OR¹¹³ wherein R¹¹³ represents a hydrogen atom or C₁₋₄ alkyl,
-(C=O)NR¹¹⁴R¹¹⁵ wherein R¹¹⁴ and R¹¹⁵, which may be the same or different, represent a hydrogen atom or C₁₋₄ alkyl,
C₁₋₆ alkoxy,
C₁₋₆ alkyl,
C₁₋₆ alkenyl, or
C₁₋₆ alkynyl,
the C₁₋₆ alkoxy, C₁₋₆ alkyl, C₁₋₆ alkenyl, or C₁₋₆ alkynyl are optionally substituted by hydroxyl; a halogen atom; C₁₋₄ alkoxy; -NR¹¹⁶R¹¹⁷ wherein R¹¹⁶ and R¹¹⁷, which may be the same or different, represent a hydrogen atom or C₁₋₄ alkyl and the alkyl group is further optionally substituted by hydroxyl or C₁₋₄ alkoxy; or a saturated or unsaturated three- to eight-membered carbocylic or heterocyclic group in which the cyclic group is optionally substituted by hydroxyl, a halogen atom, C₁₋₄ alkyl, or C₁₋₄ alkoxy,
all of R¹⁰⁵, R¹⁰⁶, R¹⁰⁷, and R¹⁰⁸ represent a hydrogen atom, or any one or two of R¹⁰⁵, R¹⁰⁶, R¹⁰⁷, and R¹⁰⁸ represent hydroxyl, C₁₋₄ alkyl, C₁₋₄ alkoxy, amino, nitro, or a halogen atom with all the remaining groups representing a hydrogen atom,
R¹⁰⁹ represents -(CH₂)n-R¹¹⁰ wherein n is 2, 3, or 4, and R¹¹⁰ represents i-propyl optionally substituted by C₁₋₄ alkyl, C₁₋₄ alkoxy, or hydroxyl; t-butyl optionally substituted by C₁₋₄ alkyl, C₁₋₄ alkoxy, or hydroxyl; or a three- to nine-membered saturated carbocyclic group optionally substituted by C₁₋₄ alkyl, C₁₋₄ alkoxy, or hydroxyl.

Furthermore, according to the present invention, there are provided compounds represented by formula (II) or pharmaceutically acceptable salts or solvates thereof: 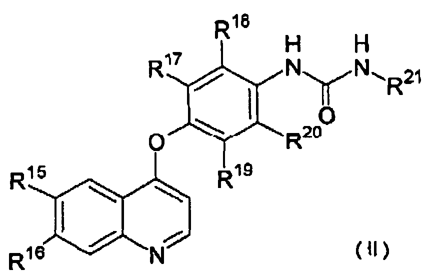 wherein
R¹⁵ and R¹⁶, which may be the same or different, represent -O-(CH₂)r-R²² wherein r is an integer of 0 to 6, -(CH₂)r- is optionally substituted by C₁₋₆ alkyl, hydroxyl, or a halogen atom, and R²² represents a hydrogen atom; hydroxyl; a halogen atom; C₁₋₆ alkoxy; C₁₋₆ alkylcarbonyl; carboxyl; C₁₋₆ alkoxycarbonyl; -(C=O)-NR²³R²⁴ wherein R²³ and R²⁴, which may be the same or different, represent a hydrogen atom or C₁₋₄ alkyl optionally substituted by hydroxyl, or R²³ and R²⁴ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic ring,
all of R¹⁷, R¹⁸, R¹⁹, and R²⁰ represent a hydrogen atom, or any one or two of R¹⁷, R¹⁸, R¹⁹, and R²⁰ represent a halogen atom, C₁₋₄ alkyl, C₁₋₄ alkoxy, nitro, or amino with all the remaining groups representing a hydrogen atom, and
R²¹ represents -(CH₂)t-R⁶¹ wherein t is an integer of 1 to 4 and R⁶¹ represents a saturated three- to seven-membered carbocyclic group; i-propyl optionally substituted by hydroxyl; t-butyl optionally substituted by hydroxyl; C₁₋₄ alkoxy; or -NR⁶²R⁶³ wherein R⁶² and R⁶³, which may be the same or different, represent a hydrogen atom, or C₁₋₄ alkyl optionally substituted by hydroxyl, or R⁶² and R⁶³ may combine with a nitrogen atom attached thereto to form a saturated five- or six-membered heterocyclic group, or R²¹ represents a saturated five- to seven-membered carbocyclic group optionally substituted by one to three C₁₋₄ alkyl groups.

### DETAILED DESCRIPTION OF THE INVENTION

### Compound

The terms "alkyl," "alkoxy," "alkenyl," and "alkynyl" as used herein as a group or a part of a group respectively mean straight chain or branched chain alkyl, alkoxy, alkenyl, and alkynyl.

C₁₋₆ alkyl is preferably C₁₋₄ alkyl.

C₁₋₆ alkoxy is preferably C₁₋₄ alkoxy.

C₂₋₆ alkenyl is preferably C₂₋₄ alkenyl.

C₂₋₆ alkynyl is preferably C₂₋₄ alkynyl.

Examples of C₁₋₆ alkyl include methyl, ethyl, n-propyl, isopropyl, n-butyl, i-butyl, s-butyl, t-butyl, n-pentyl, and n-hexyl.

Examples of C₁₋₆ alkoxy include methoxy, ethoxy, n-propoxy, i-propoxy, n-butoxy, i-butoxy, s-butoxy, and t-butoxy.

Examples of C₂₋₆ alkenyl include allyl, butenyl, pentenyl, and hexenyl.

Examples of C₂₋₆ alkynyl include 2-propynyl, butynyl, pentynyl, and hexynyl.

The expression "alkyl optionally substituted by" as used herein refers to alkyl, in which one or more hydrogen atoms on the alkyl group have been substituted by one or more substituents which may be the same or different, and unsubstituted alkyl. It will be apparent to one skilled in the art that the maximum number of substituents may be determined depending upon the number of substitutable hydrogen atoms on the alkyl group. This is applicable to groups having a substituent other than the alkyl group.

The term "halogen atom" means fluorine, chlorine, bromine, and iodine atoms.

The saturated or unsaturated three- to eight-membered carbocyclic ring is preferably a four- to seven-membered, more preferably five- or six-membered, saturated or unsaturated carbocyclic ring. Examples of saturated or unsaturated three- to eight-membered carbocyclic rings include phenyl, cyclopropyl, cyclobutyl, cyclopentyl, cyclohexyl, and cycloheptyl.

The saturated or unsaturated three- to eight-membered heterocyclic ring contains at least one hetero-atom selected from oxygen, nitrogen, and sulfur atoms. The saturated or unsaturated three- to eight-membered heterocyclic ring preferably contains one, two or three hetero-atoms with the remaining ring-constituting atoms being carbon atoms. The saturated or unsaturated three- to eight-membered heterocyclic ring is preferably a saturated or unsaturated four- to seven-membered heterocyclic ring, more preferably a saturated or unsaturated five- or six-membered heterocyclic ring. Examples of saturated or unsaturated three- to eight-membered heterocyclic groups include thienyl, pyridyl, 1,2,3-triazolyl, thiazolyl, imidazolyl, isoxazolyl, pyrazolyl, piperazinyl, piperazino, piperidyl, piperidino, morpholinyl, morpholino, homopiperazinyl, homopiperazino, thiomorpholinyl, thiomorpholino, tetrahydropyrrolyl, and azepanyl.

The saturated or unsaturated carboxylic and heterocyclic groups may condense with another saturated or unsaturated five- to seven-membered carbocyclic or heterocyclic ring to form a bicyclic group, preferably a saturated or unsaturated nine- to twelve-membered bicyclic carbocyclic or heterocyclic group. Such bicyclic groups include naphthyl, quinolyl, 1,2,3,4-tetrahydroquinolyl, 1,4-benzoxanyl, indanyl, indolyl, 1,2,3,4-tetrahydronaphthyl, and phthalimide.

When the carbocyclic or heterocyclic group is substituted by two C₁₋₆ alkyl groups, the two alkyl groups may combine together to form an alkylene chain, preferably a C₁₋₃ alkylene chain. Carbocyclic or heterocyclic groups having this crosslinked structure include azabicyclo[2.2.2]octanyl, bicyclo[2.2.2]octanyl and norbornanyl.

R¹ preferably represents a hydrogen atom.

R² and R³ preferably represent a group other than a hydrogen atom, more preferably may be the same or different and represent optionally substituted C₁₋₆ alkoxy.

More preferably, R¹ represents a hydrogen atom and both R² and R³ represent unsubstituted C₁₋₆ alkoxy, or alternatively any one of R² and R³ represents substituted C₁₋₆ alkoxy and the other represents unsubstituted C₁₋₆ alkoxy.

Preferably, R² and R³, which may be the same or different, represent -O-(CH₂)p-R¹² wherein p is an integer of 0 to 6, -(CH₂)p- is optionally substituted by C₁₋₆ alkyl, hydroxyl, or a halogen atom, and R¹² represents a hydrogen atom; hydroxyl; a halogen atom; C₁₋₆ alkoxy; C₁₋₆ alkylcarbonyl; carboxyl; C₁₋₆ alkoxycarbonyl; -(C=O)-NR¹³R¹⁴ wherein R¹³ and R¹⁴, which may be the same or different, represent a hydrogen atom or C₁₋₄ alkyl optionally substituted by hydroxyl, or alternatively R¹³ and R¹⁴ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or alternatively, the carbocyclic or heterocyclic group may be a bicyclic group condensed with another saturated or unsaturated five- to seven-membered carbocyclic or heterocyclic ring. When p is 0 (zero), -(CH₂)p- represents a bond. p is preferably an integer of 1 to 4.

More preferably, R¹ represents a hydrogen atom and, at the same time, both R² and R³ represent -O-(CH₂)p-H, or alternatively R¹ represents a hydrogen atom and, at the same time, any one of R² and R³ represents -O-(CH₂)p-H with the other representing -O-(CH₂)p-R¹² wherein R¹² represents a group other than a hydrogen atom.

Preferably, all of R⁵, R⁶, R⁷, and R⁸ represent a hydrogen atom, or alternatively any one or two of R⁵, R⁶, R⁷, and R⁸ represent a halogen atom with all the remaining groups represening a hydrogen atom.

More preferably, all of R⁵, R⁶, R⁷, and R⁸ represent a hydrogen atom; or R⁶ represents a fluorine atom, and R⁵, R⁷, and R⁸ represent a hydrogen atom; or R⁵ represents a halogen atom, C₁₋₄ alkyl, C₁₋₄ alkoxy, nitro, or amino (preferably a halogen atom or C₁₋₄ alkyl or C₁₋₄ alkoxy) and R⁶, R⁷, and R⁸ represent a hydrogen atom; or R⁵ and R⁷ represent a halogen atom, C₁₋₄ alkyl, C₁₋₄ alkoxy, nitro, or amino (preferably a halogen atom or C₁₋₄ alkyl or C₁₋₄ alkoxy), and R⁶ and R⁸ represent a hydrogen atom.

Preferably, R⁹ represents -(CH₂)s-R⁵¹ wherein s is an integer of 1 to 4, and R⁵¹ represents a saturated three- to seven-membered carbocyclic group; i-propyl optionally substituted by hydroxyl; t-butyl optionally substituted by hydroxyl; C₁₋₄ alkoxy; or -NR⁵²R⁵³ wherein R⁵² and R⁵³, which may be the same or different, represent a hydrogen atom, or C₁₋₄ alkyl optionally substituted by hydroxyl, or R⁵² and R⁵³ may combine with a nitrogen atom attached thereto to form a saturated five- or six-membered heterocyclic group, or R⁹ represents a saturated five- to seven-membered carbocyclic group optionally substituted by one to three C₁₋₄ alkyl groups.

More preferably, R⁹ represents -(CH₂)s-R⁵¹, wherein s is an integer of 1 to 4, and R⁵¹ represents a saturated five- to seven-membered carbocyclic group; i-propyl; t-butyl optionally substituted by hydroxyl; C₁₋₄ alkoxy; or -NR⁵²R⁵³ wherein R⁵² and R⁵³, which may be the same or different, represent C₁₋₄ alkyl, or R⁹ represents a five- to seven-membered carbocyclic group optionally substituted by 1 to 3 C₁₋₄ alkyl groups. Most preferably, R⁹ represents -(CH₂)s-R⁵¹, wherein s is an integer of 2 or 3 and R⁵¹ represents a saturated five- to seven-membered carbocyclic group or t-butyl, or R⁹ represents a five- to seven-membered carbocyclic group optionally substituted by 1 to 3 C₁₋₄ alkyls.

In the compounds represented by formula (I), preferably,
X represents CH or N,
Z represents O or S,
R¹, R², and R³, which may be the same or different, represent
a hydrogen atom,
hydroxyl,
a halogen atom,
nitro,
amino,
C₁₋₆ alkyl,
C₂₋₆ alkenyl,
C₂₋₆ alkynyl, or
C₁₋₆ alkoxy,
the C₁₋₆ alkyl, C₂₋₆ alkenyl, C₂₋₆ alkynyl, and C₁₋₆ alkoxy groups, which may be represented by R¹, R², and R³, are optionally substituted by hydroxyl; a halogen atom; C₁₋₆ alkoxy; C₁₋₆ alkylcarbonyl; carboxyl; C₁₋₆ alkoxycarbonyl; -(C=O)-NR¹⁰R¹¹ wherein R¹⁰ and R¹¹, which may be the same or different, represent a hydrogen atom or C₁₋₄ alkyl optionally substituted by hydroxyl, or alternatively R¹⁰ and R¹¹ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or alternatively, the carbocyclic or heterocyclic group may be a bicyclic group condensed with another saturated or unsaturated five- to seven-membered carbocyclic or heterocyclic ring;
one or two hydrogen atoms on the amino group, which may be represented by R¹, R², and R³, are optionally substituted by C₁₋₆ alkyl which is further optionally substituted by hydroxyl or C₁₋₆ alkoxy;
R⁴ represents a hydrogen atom;
all of R⁵, R⁶, R⁷, and R⁸ represent a hydrogen atom, or any one or two of R⁵, R⁶, R⁷, and R⁸ represent a halogen atom, C₁₋₄ alkyl, C₁₋₄ alkoxy, nitro, or amino with all the remaining groups representing a hydrogen atom, and
R⁸ represents C₁₋₄ alkyl substituted by a substituent selected from the group consisting of a saturated three- to seven-membered carbocyclic group; i-propyl optionally substituted by hydroxyl; t-butyl optionally substituted by hydroxyl; C₁₋₄ alkoxy; and -NR^{a}R^{b} wherein R^{a} and R^{b}, which may be the same or different, represent a hydrogen atom or C₁₋₄ alkyl optionally substituted by hydroxyl, or alternatively R^{a} and R^{b} may combine with a nitrogen atom attached thereto to form a saturated five- or six-membered heterocyclic group, or R⁹ represents a saturated five- to seven-membered carbocyclic group optionally substituted by one to three C₁₋₄ alkyl groups.

The compounds represented by formula (I) are preferably compounds represented by formula (Ia).

In formula (Ia), preferably, R¹⁰² and R¹⁰³, which may be the same or different, represent C₁₋₆ alkoxy and the C₁₋₆ alkoxy is optionally substituted by hydroxyl; a halogen atom; C₁₋₄ alkoxy; -NR¹¹⁶R¹¹⁷ wherein R¹¹⁶ and R¹¹⁷, which may be the same or different, represent a hydrogen atom or C₁₋₄ alkyl and the alkyl group is further optionally substituted by hydroxyl or C₁₋₄ alkoxy; or a saturated or unsaturated three- to eight-membered carbocyclic or heterocyclic group in which the cyclic group is optionally substituted by hydroxyl, a halogen atom, C₁₋₄ alkyl, or C₁₋₄ alkoxy.

More preferably, R¹⁰² and R¹⁰³, which may be the same or different, represent C₁₋₆ alkoxy in which the alkoxy group is optionally substituted by a saturated or unsaturated three- to eight-membered carbocyclic or heterocyclic group and the cyclic group is further optionally substituted by hydroxyl, a halogen atom, C₁₋₄ alkyl, or C₁₋₄ alkoxy.

Still more preferably, R¹⁰² and R¹⁰³, which may be the same or different, represent C₁₋₄ alkoxy in which the alkoxy group is optionally substituted by a saturated five- to seven-membered heterocyclic group and the cyclic group is further optionally substituted by C₁₋₄ alkyl.

In formula (Ia), "substituted C₁₋₄ alkoxy" represented by R¹⁰² and R¹⁰³ preferably represents a group represented by 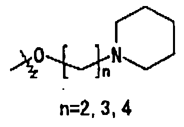 more preferably n is 2,
or a group represented by 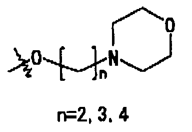 more preferably n is 2.

In formula (Ia), preferably, one of R¹⁰² and R¹⁰³ represents unsubstituted C₁₋₆ alkoxy and the other represents substituted C₁₋₆ alkoxy. More preferably, R¹⁰² represents unsubstituted C₁₋₆ alkoxy (more preferably methoxy) and R¹⁰³ represents substituted C₁₋₆ alkoxy.

In formula (Ia), preferably, X represents CH.

In formula (Ia), preferably, Z represents O.

In formula (Ia), preferably, all of R¹⁰⁵, R¹⁰⁶, R¹⁰⁷, and R¹⁰⁸ represent a hydrogen atom, or alternatively any one or two of R¹⁰⁵, R¹⁰⁶, R¹⁰⁷, and R¹⁰⁸ represent C₁₋₄ alkyl, C₁₋₄ alkoxy, or a halogen atom with all the remaining groups representing a hydrogen atom.

In formula (Ia), more preferably, R¹⁰⁵ represents methoxy, and R¹⁰⁶, R¹⁰⁷, and R¹⁰⁸ represent a hydrogen atom.

In formula (Ia), more preferably, R¹⁰⁵ represents methyl, and R¹⁰⁶, R¹⁰⁷, and R¹⁰⁸ represent a hydrogen atom.

In formula (Ia), more preferably, R¹⁰⁵ represents a halogen atom (still more preferably a chlorine or fluorine atom, most preferably a fluorine atom), and R¹⁰⁶, R¹⁰⁷, and ¹⁰⁸ represent a hydrogen atom.

In formula (Ia), more preferably, all of R¹⁰⁵, R¹⁰⁶, R¹⁰⁷, and R¹⁰⁸ represent a hydrogen atom.

In formula (Ia), preferably, R¹⁰⁹ is a group represented by 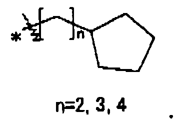 (more preferably, n is 2),
or a group represented by 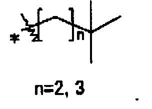 (more preferably, n is 2).

Among the compounds represented by formula (Ia), the following compounds are particularly preferred:
1-(3,3-dimethyl-butyl)-3-{3-fluoro-4-[6-methoxy-7-(2-piperidin-1-yl-ethoxy)-quinolin-4-yloxy]-phenyl}-urea;
1-(2-cyclopentyl-ethyl)-3-{3-fluoro-4-[6-methoxy-7-(2-piperidin-1-yl-ethoxy)-quinolin-4-yloxy]-phenyl}-urea; and
1-(2-cyclopentyl-ethyl)-3-{2-fluoro-4-[6-methoxy-7-(2-piperidin-1-yl-ethoxy)-quinolin-4-yloxy]-phenyl}-urea.

The compounds represented by formula (I) are preferably compounds represented by formula (II).

In formula (II), preferably, both R¹⁵ and R¹⁶ represent -O-(CH₂)r-H, or alternatively any one of R¹⁵ and R¹⁶ represents -O-(CH₂)r-H with the other representing -O-(CH₂)r-R²² wherein R²² represents a group other than a hydrogen atom, preferably optionally substituted amino or an optionally substituted saturated three- to eight-membered heterocyclic group. When r is 0 (zero), -(CH₂)r- represents a bond. -(CH₂)r- is preferably unsubstituted. r is preferably an integer of 1 to 4.

Preferably, all of R¹⁷, R¹⁸, R¹⁹, and R²⁰ represent a hydrogen atom, or alternatively R¹⁸ represents a fluorine atom, and R¹⁷, R¹⁹, and R²⁰ represents a hydrogen atom; or R¹⁷ represents halogen atom, C₁₋₄ alkyl, C₁₋₄ alkoxy, nitro, or amino (preferably a halogen atom, C₁₋₄ alkyl, or C₁₋₄ alkoxy), and R¹⁸, R¹⁹, and R²⁰ represent a hydrogen atom; or R¹⁷ and R¹⁹ represent a halogen atom, C₁₋₄ alkyl, C₁₋₄ alkoxy, nitro, or amino (preferably a halogen atom, C₁₋₄ alkyl, or C₁₋₄ alkoxy), and R¹⁸ and R²⁰ represent a hydrogen atom.

Preferably, R²¹ represents -(CH₂)t-R⁶¹, wherein t is an integer of 1 to 4, and R⁶¹ represents a saturated five- to seven-membered carbocyclic group; i-propyl; t-butyl optionally substituted by hydroxyl; C₁₋₄ alkoxy; or -NR⁶²R⁶³ wherein R⁶² and R⁶³, which may be the same or different, represents C₁₋₄ alkyl, or R²¹ represents a five- to seven-membered carbocyclic group optionally substituted by one to three C₁₋₄ alkyl groups.

More preferably, R²¹ represents -(CH₂)t-R⁶¹, wherein t is an integer of 2 or 3, and R⁶¹ represents a saturated five- to seven-membered carbocyclic group or t-butyl, or R²¹ represents a five- to seven-membered carbocyclic group optionally substituted by one to three C₁₋₄ alkyl groups.

Preferred compounds represented by formula (II) include compounds in which
R¹⁵ and R¹⁶ represent -O-(CH₂)r-H wherein r is an integer of 1 to 4 and the -(CH₂)r- part is unsubstituted, or alternatively any one of R¹⁵ and R¹⁶ represents -O-(CH₂)r-H wherein r is an integer of 1 to 4 and the -(CH₂)r- part is unsubstituted with the other representing -O-(CH₂)r-R²² wherein r is an integer of 1 to 4, the -(CH₂)r- part is unsubstituted, and R²² represents optionally substituted amino or an optionally substituted saturated three- to eight-membered heterocyclic group,
all of R¹⁷, R¹⁸, R¹⁹, and R²⁰ represent a hydrogen atom, or alternatively any one or two of R¹⁷, R¹⁸, R¹⁹, and R²⁰ represent a halogen atom, C₁₋₄ alkyl, C₁₋₄ alkoxy, nitro, or amino with all the remaining groups representing a hydrogen atom, and
R²¹ represents -(CH₂)t-R⁶¹, wherein t is an integer of 1 to 4 and R⁶¹ represents a saturated five- to seven-membered carbocyclic group; i-propyl; t-butyl optionally substituted by hydroxyl; C₁₋₄ alkoxy; or -NR⁶²R⁶³ wherein R⁶² and R⁶³, which may be the same or different, represent C₁₋₄ alkyl, or R²¹ represents a five- to seven-membered carbocyclic group optionally substituted by one to three C₁₋₄ alkyl groups.

More preferred examples of compounds represented by formula (II) include compounds in which
R¹⁵ and R¹⁶ represent -O-(CH₂)r-H wherein r is an integer of 1 to 4 and the -(CN₂)r- part is unsubstituted, or alternatively any one of R¹⁵ and R¹⁶ represents -O-(CH₂)r-H wherein r is an integer of 1 to 4 and the -(CH₂)r- part is unsubstituted with the other representing -O-(CH₂)r-R²² wherein r is an integer of 1 to 4, the -(CH₂)r- part is unsubstituted, and R²² represents optionally substituted amino or an optionally substituted saturated three- to eight-membered heterocyclic group,
all of R¹⁷, R¹⁸, R¹⁹, and R²⁰ represent a hydrogen atom; or R¹⁶ represents a fluorine atom, and R¹⁷, R¹⁹, and R²⁰ represent a hydrogen atom; or R¹⁷ represents a halogen atom, C₁₋₄ alkyl, or C₁₋₄ alkoxy, and R¹⁸, R¹⁹, and R²⁰ represent a hydrogen atom; or R¹⁷ and R¹⁹ represent a halogen atom, C₁₋₄ alkyl, or C₁₋₄ alkoxy, and R¹⁸ and R²⁰ represent a hydrogen atom, and
R²¹ represents -(CH₂)t-R⁶¹, wherein t is an integer of 2 or 3 and R⁶¹ represents a saturated five- to seven-membered carbocyclic group or t-butyl, or R²¹ represents a five- to seven-membered carbocyclic group optionally substituted by one to three C₁₋₄ alkyl groups.

Preferred examples of compounds represented by formula (I) include compounds 1 to 77 described in the working examples.

The compounds represented by formula (I), the compounds represented by formula (Ia), and the compounds represented by formula (II) may form pharmaceutically acceptable salts thereof. Preferred examples of such salts include: alkali metal or alkaline earth metal salts such as sodium salts, potassium salts or calcium salts; hydrohalogenic acid salts such as hydrofluoride salts, hydrochloride salts, hydrobromide salts, or hydroiodide salts; inorganic acid salts such as nitric acid salts, perchloric acid salts, sulfuric acid salts, or phosphoric acid salts; lower alkylsulfonic acid salts such as methanesulfonic acid salts, trifluoromethanesulfonic acid salts, or ethanesulfonic acid salts; arylsulfonic acid salts such as benzenesulfonic acid salts or p-toluenesulfonic acid salts; organic acid salts such as fumaric acid salts, succinic acid salts, citric acid salts, tartaric acid salts, oxalic acid salts, maleic acid salts, acetic acid salts, malic acid salts, lactic acid salts, or ascorbic acid salts; and amino acid salts such as glycine salts, phenylalanine salts, glutamic acid salts, or aspartic acid salts.

The compounds represented by formula (I), the compounds represented by formula (Ia), and the compounds represented by formula (II) may form solvates. Such solvates include, for example, hydrates, alcoholates, for example, ethanolates, and etherates.

### Production of compounds

Compounds of formula (I), compounds of formula (Ia), and compounds of formula (II) may be produced, for example, according to schemes 1 to 11. Starting compounds necessary for the synthesis of the compounds according to the present invention are commercially available or alternatively can be easily produced by conventional methods. In the schemes, R¹ to R⁹ are as defined in formula (I).

A 4-chloroquinoline derivative can be produced according to scheme 1.

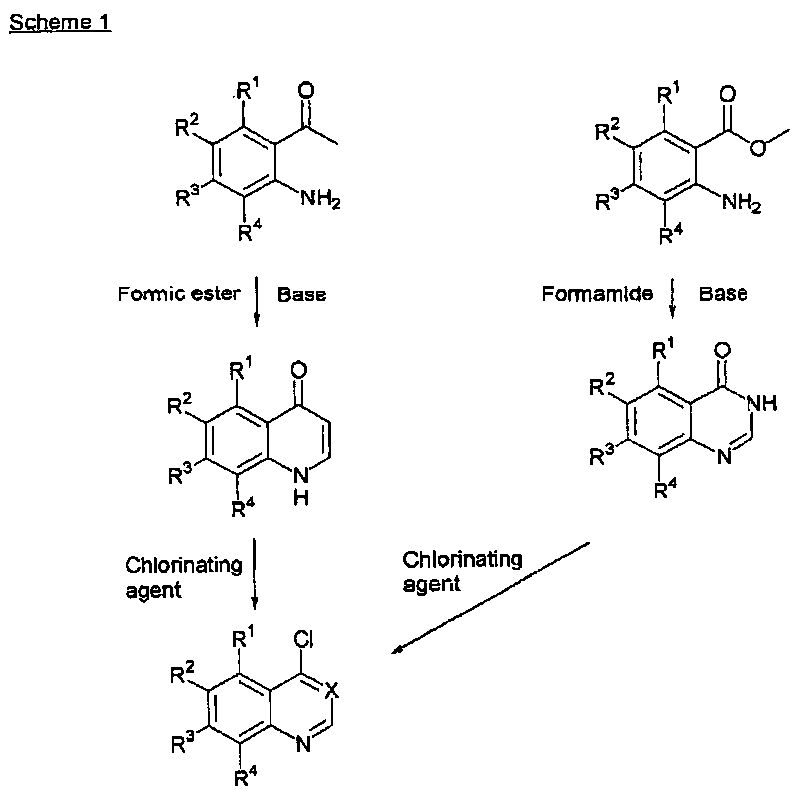

A 4-chloroquinoline derivative may be synthesized by conventional means described, for example, in Org. Synth. Col. Vol. 3, 272 (1955), Acta Chim. Hung., 112, 241 (1983) and WO 98/47873. A quinolone derivative is produced by reacting a 2-aminoacetophenone derivative in a suitable solvent, for example, tetrahydrofuran, with a formic ester, for example, ethyl formate, in the presence of a base, for example, sodium methoxide. A 4-chloroquinoline derivative is produced by allowing the quinolone derivative to react in the presence of a chlorinating agent, for example, phosphorus oxychloride.

Alternatively, the 4-chloroquinazoline derivative may be produced, for example, as follows. A quinazolone derivative is produced by reacting a 2-aminobenzoic ester derivative in a suitable solvent, for example, a mixed solvent composed of N,N-dimethylformamide and methanol, in the presence of formamide and a base, for example, sodium methoxide. A 4-chloroquinazoline derivative is produced by reacting the quinazolone derivative in the presence of a chlorinating agent, for example, phosphorus oxychloride.

A urea derivative having a quinoline or quinazoline ring can be synthesized, for example, according to scheme 2.

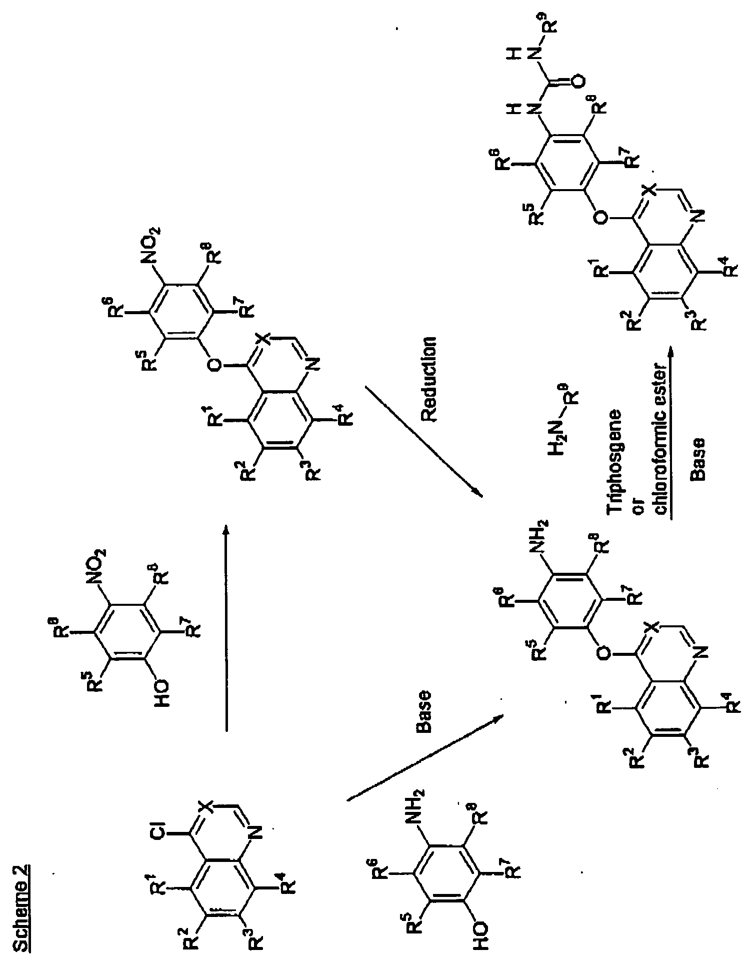

Specifically, a 4-(aminophenoxy)quinoline derivative or a corresponding quinazoline derivative is produced by reacting a nitrophenol derivative with a 4-chloroquinoline derivative or a corresponding quinazoline derivative in a suitable solvent, for example, chlorobenzene to synthesize a 4-(nitrophenoxy)quinoline derivative or a corresponding quinazoline derivative and then allowing a reaction to proceed in a suitable solvent, for example, N,N-dimethylformamide in the presence of a catalyst, for example, palladium hydroxide-carbon or palladium-carbon, under a hydrogen atmosphere. The nitro group may also be reduced by zinc, iron or the like.

Alternatively, a 4-(aminophenoxy)quinoline derivative or a corresponding quinazoline derivative may be produced by reacting an aminophenol derivative with a 4-chloroquinoline derivative or a corresponding quinazoline derivative in a suitable solvent, for example, dimethylsulfoxide in the presence of a base, for example, sodium hydride. The 4-(aminophenoxy)quinazoline derivative may also be produced by dissolving an aminophenol derivative in an aqueous sodium hydroxide solution and reacting the solution with a 4-chloroquinazoline derivative dissolved in a suitable organic solvent, for example, ethyl methyl ketone, in the presence of a phase transfer catalyst, for example, tetra-n-butylammonium chloride or in the absence of a catalyst by a two-phase reaction.

A quinoline- or quinazoline-substituted urea derivative can be produced by dissolving the 4-(aminophenoxy)quinoline derivative or corresponding quinazoline derivative in a suitable solvent, for example, chloroform, adding triphosgene or a chloroformic ester in the presence of a suitable base, for example, triethylamine, and reacting the solution with a suitable alkylamine.

An intermediate for synthesizing a derivative containing a specific substituent in the quinoline ring at its 7-position may be produced, for example, according to scheme 3.

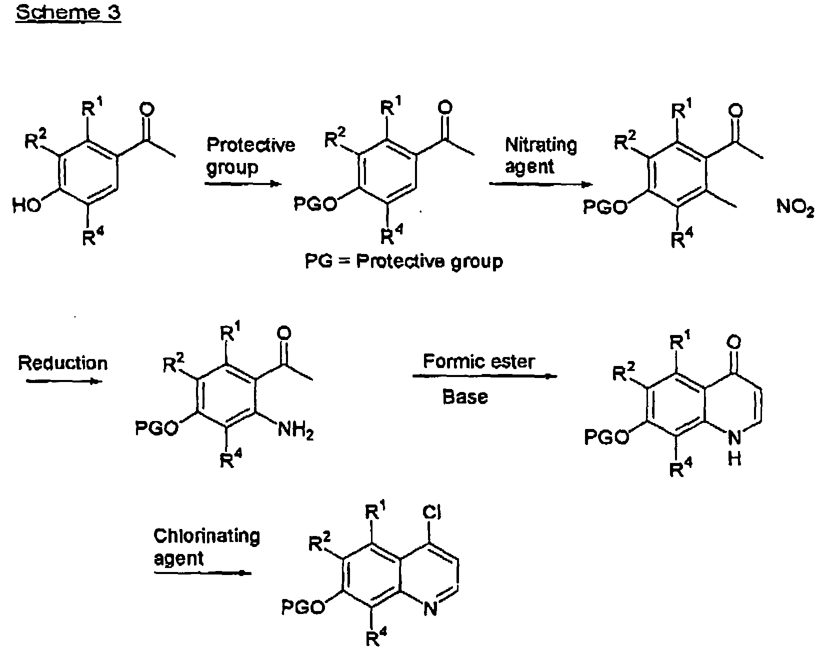

A nitro group can be introduced by protecting a commercially available 4'-hydroxyacetophenone derivative by a suitable substituent, for example, benzyl, and then reacting the protected compound with a nitrating agent, for example, fuming nitric acid-acetic acid. Thereafter, the same procedure as in scheme 1 is repeated. Specifically, the nitro group is reduced to an amino group, and the compound is then reacted with a formic ester in the presence of a base to form a quinolone ring. Subsequently, a 4-chloroquinoline derivative can be produced by reacting the compound with a chlorinating agent. In this chlorination reaction, when phosphorus oxychloride is used as the chlorinating agent, the addition of a base, for example, N,N-diisopropylethylamine, can further improve the yield.

An intermediate for synthesizing a derivative containing a specific substituent in the quinoline ring at its 6-position may be produced in the same manner as in the quinoline ring at its 7-position, except that a 3'-hydroxyacetophenone derivative is used instead of the 4'-hydroxyacetophenone derivative.

An intermediate for synthesizing a derivative containing a specific substituent in the quinazoline ring at its 7-position may be produced, for example, according to scheme 4.

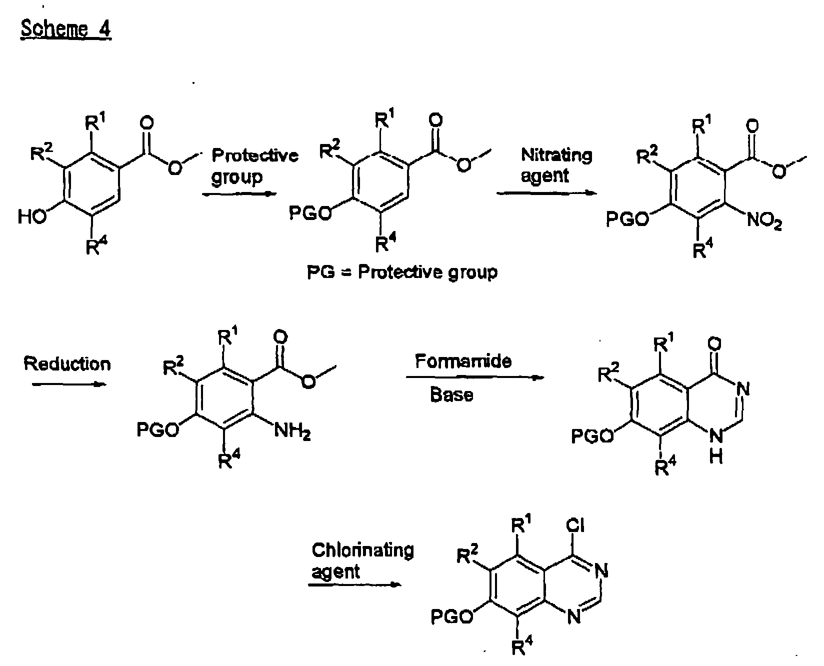

A nitro group can be introduced by protecting a hydroxyl group in a commercially available 4'-hydroxybenzoic ester derivative by a suitable substituent, for example, benzyl, and then reacting the protected compound with a nitrating agent, for example, fuming nitric acid-acetic acid. Thereafter, the same procedure as in scheme 1 is repeated. Specifically, the nitro group is reduced to an amino group, and the compound is then reacted with formamide in the presence of a base to form a quinazolone ring. Subsequently, a 4-chloroquinazoline derivative can be produced by reacting the compound with a chlorinating agent. In this chlorination reaction, when phosphorus oxychloride is used as the chlorinating agent, the addition of a base, for example, N,N-diisopropylethylamine, can further improve the yield.

An intermediate for synthesizing a derivative containing a specific substituent in the quinazoline ring at its 6-position may be produced in the same manner as in the quinazoline ring at its 7-position, except that a 3'-hydroxybenzoic ester derivative is used instead of the 4'-hydroxybenzoic ester derivative.

An aniline derivative containing a specific substituent in the quinoline or quinazoline ring at its 7-position may be produced, for example, according to scheme 5.

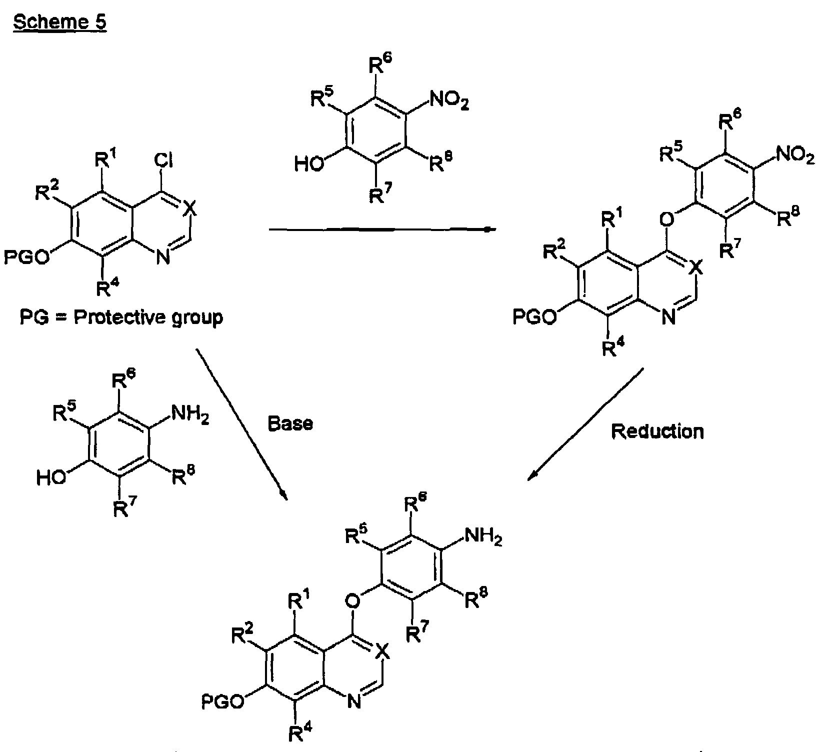

Specifically, a 4-(aminophenoxy)quinoline derivative or a corresponding quinazoline derivative is produced by reacting the 4-chloroquinoline derivative or quinazoline derivative produced in scheme 3 or 4 with a nitrophenol derivative in a suitable solvent, for example, chlorobenzene to synthesize a 4-(nitrophenoxy)quinoline derivative or a corresponding quinazoline derivative and then allowing a reaction to proceed in a suitable solvent, for example, N,N-dimethylformamide in the presence of a catalyst, for example, palladium hydroxide-carbon or palladium-carbon, under a hydrogen atmosphere. The nitro group may also be reduced by zinc, iron or the like. Alternatively, a 4-(aminophenoxy)quinoline derivative or a corresponding quinazoline derivative may be produced by reacting an aminophenol derivative with a 4-chloroquinoline derivative or a corresponding quinazoline derivative in a suitable solvent, for example, dimethylsulfoxide in the presence of a base, for example, sodium hydride. Alternatively, the 4-(aminophenoxy)quinazoline derivative may also be produced by dissolving an aminophenol derivative in an aqueous sodium hydroxide solution and reacting the solution with a 4-chloroquinazoline derivative dissolved in a suitable organic solvent, for example, ethyl methyl ketone, in the presence of a phase transfer catalyst, for example, tetra-n-butylammonium chloride or in the absence of a catalyst by a two-phase reaction. Scheme 5 depicts a process for synthesizing a compound according to the present invention containing a substituent in the quinoline ring or quinazoline ring at its 7-position. A compound according to the present invention containing a substituent in the quinoline ring or quinazoline ring at its 6-position may be synthesized by using, as a starting compound, a quinoline derivative or quinazoline derivative with a protective group introduced into the 6-position thereof.

The quinoline derivative with a protective group introduced into the 6-position used as the starting compound may be synthesized, for example, according to scheme 6 (for details, see Production Examples).

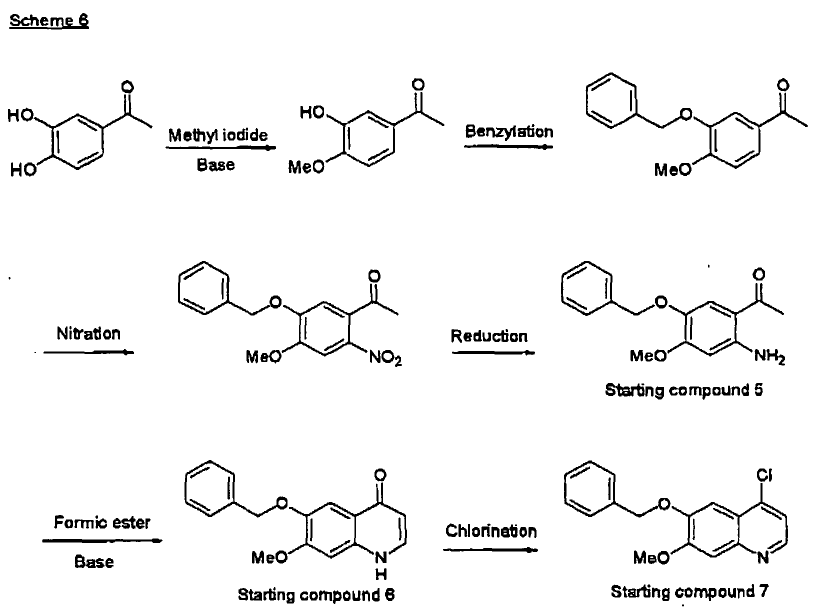

A 4-(quinolylsulfanyl)aniline derivative or a 4-(quinazolinylsulfanyl)aniline derivative (a compound of formula (I) wherein Z = S) can be produced according to scheme 7.

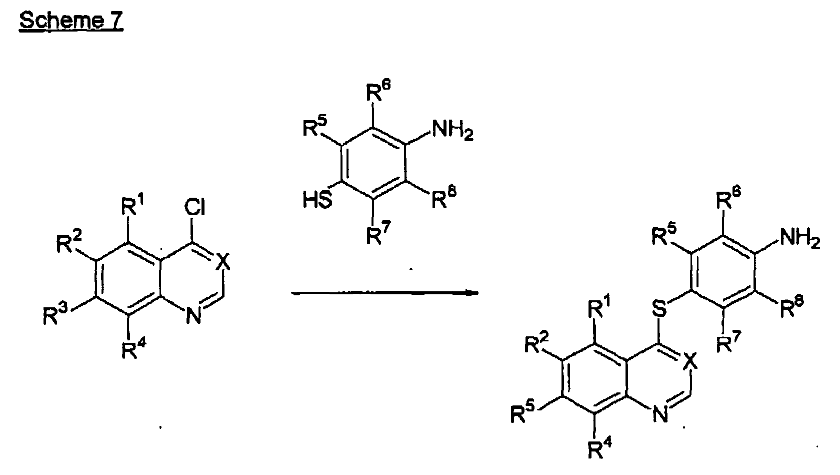

A 4-(quinolylsulfanyl)aniline derivative or a 4-(quinazolinyisulfanyl)aniline derivative may be produced by reacting an aminothiophenol derivative with a 4-chloroquinoline derivative or a corresponding quinazoline derivative in a suitable solvent, for example, chlorobenzene). A urea derivative containing a sulfur atom in the quinoline or quinazoline ring at its 4-position can be produced from this derivative according to scheme 2.

A urea derivative containing a specific substitutent in the quinoline ring or quinazoline ring at its 7-position can be synthesized, for example, according to scheme 8.

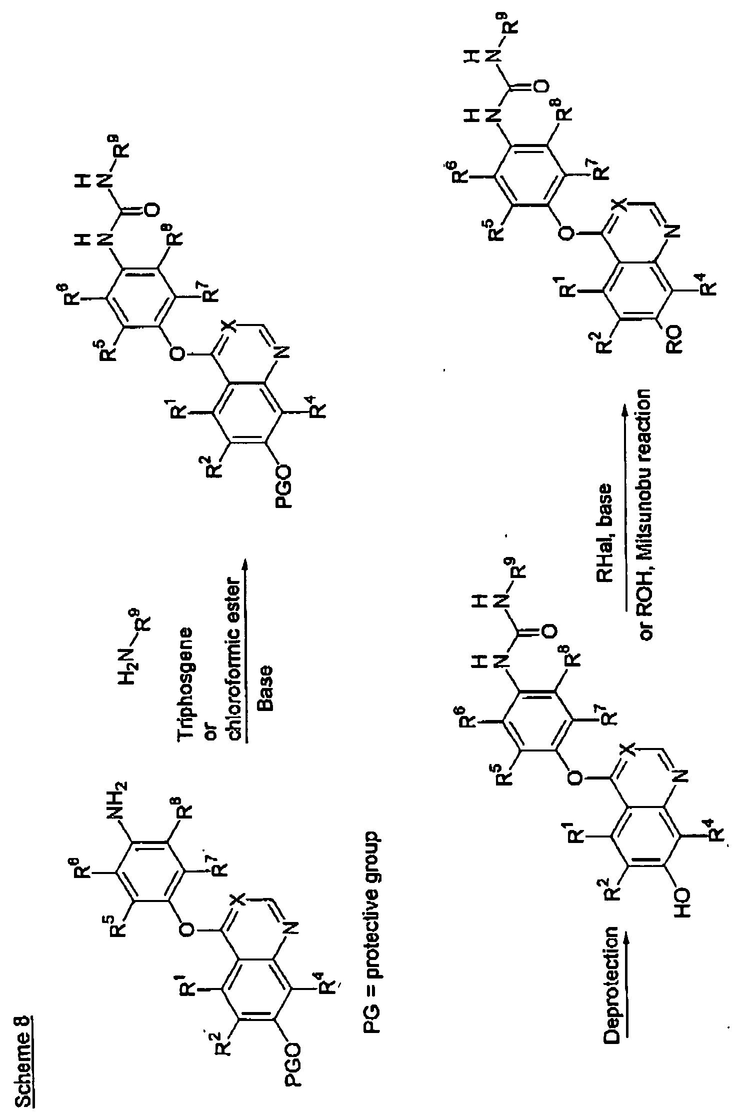

Specifically, a urea derivative containing a protected hydroxyl group in quinoline or quinazoline at its 7-position can be produced by dissolving the 4-(aminophenoxy)quinoline derivative or corresponding quinazoline derivative prepared in scheme 5 in a suitable solvent, for example, chloroform, adding triphosgene or a chloroformic ester in the presence of a suitable base, for example, triethylamine, and reacting the solution with a suitable alkylamine. A 7-hydroxyquinoline derivative or a corresponding quinazoline derivative can be produced by deprotecting the hydroxyl group in the urea derivative under suitable conditions. For example, when the protective group is benzyl, a reaction is allowed to proceed, for example, in N,N-dimethylformamide in the presence of palladium hydroxide-carbon or palladium-carbon under a hydrogen atmosphere. Next, a urea derivative containing a specific substituent in quinoline or quinazoline at its 7-position can be produced by alkylating the 7-hydroxyquinoline derivative or corresponding quinazoline derivative under suitable conditions, for example, by a reaction with an alkyl halide (RHal) in N,N-dimethylformamide in the presence of potassium carbonate, or by a reaction with an alkyl alcohol (ROH) utilizing the Mitsunobu reaction). A urea derivative containing a specific substituent in the quinoline ring or quinazoline ring at its 6-position can be produced in the same manner as in the 7-position.

Alternatively, a urea derivative containing a specific substituent in the quinoline ring or quinazoline ring at its 6- or 7-position may be synthesized, for example, as follows. A quinoline derivative or corresponding quinazoline derivative containing a hydroxyl group in quinoline or quinazoline at its 6- or 7-position can be produced by dissolving a 6,7-dimethoxy-4-(nitrophenoxy)quinoline derivative or corresponding quinazoline derivative in a suitable solvent, for example, chloroform, and heating the solution under reflux in the presence of a suitable Lewis acid, for example, trialuminum chloride. A 4-(nitrophenoxy)quinoline derivative or corresponding quinazoline derivative which has been protected at its 6- or 7-position can be produced by protecting the hydroxyl group in this derivative under suitable conditions and separating and purifying the product. The hydroxyl group may be protected, for example, by a benzyl group. The benzyl group may be introduced by reacting the derivative with benzyl chloride in N,N-dimethylformamide in the presence of potassium carbonate. A 4-(aminophenoxy)quinoline derivative or corresponding quinazoline derivative can be derived from the resultant derivative in the same manner as in the method according to scheme 5. A urea derivative containing a specific substituent in the quinoline or quinazoline ring at its 6- or 7-position can be produced from this derivative in the same manner as in scheme 8 (for more details, see scheme 11).

The urea derivative containing a specific substituent in the quinoline or quinazoline ring at its 7-position may also be synthesized according to the method described in WO 00/43366.

### Use of medicaments/ pharmaceutical composition

Flt3 is reported to be highly expressed in brains, placentae, livers and hematopoietic stem cells (Shibuya et al., Oncogene, 5: 519-524, 1990; O. Rosnet et al., Genomics, 9: 380-385, 1991; O. Rosnet et al., Oncogene, 6: 1641-1650, 1991; O. Ronsnet et al., Oncogene, 6: 1641-1650, 1991; W. Matthews et al., Cell, 65: 1143-1152, 1991).

The analysis of genes using knockout mice has revealed that the destruction of Flt3 genes leads to injury of precursor cells of lymphocytes. It is also reported that destruction of KIT genes simultaneously with the destruction of Flt3 genes causes severe hematopoietic injury involving pancytopenia (K. Mackarehtschian, Immunity, 3: 147-161, 1995).

Further, in knockout mice of FLT3 ligand, a reduction in leukocytic cells in bone marrow, bone marrow progenitor cells and B lymphoid progenitor cells, a deficit of natural killer cells in the spleen, and a reduction in dendritic cells of the spleen, thymus, and lymph nodes are observed (H. J. McKenna et al., BLOOD, 95: 3489-3497, 2000).

Furthermore, in chronic myelocytic leukemia (CML), cases are reported in which, as compared with the chronic phase, the expression of Flt3 is increased after conversion to the acute phase (Iwai, T. et al., Leukemia, 11: 1992-1993. 1997). As described above, it is considered that, upon an enhancement in a signal transduction system as a result of occurrence of an abnormal phenomenon of Flt3, excessive growth and differentiation of haemopoietic cells take place, leading to tumorigenesis , immune disorder and the like of cells.

In recent years, somatic cell variants of Flt3 (Flt3-ITD) were found in patients suffering from acute myelocytic leukemia (AML) (M. Nakao et al., Leukemia, 10: 1911-1918. 1996). In this variation, the repetition of a short base sequence of about ten to several tens of base pairs (internal tandem duplication: ITD) were observed within a given region of exon11/12 corresponding to a juxtamenbrane domain of Flt3. This phenomenon was observed in about 20% of AML patients and about 5% of patients suffering from myelodysplastic syndrome (MDS) (S. Yokota et al., Leukemia, 11: 1605-1609, 1997). Further, mutation of Flt3 genes (FLT3-ITD) was an adverse prognostic factor of AML (H. Kiyoi et al., Blood, 93: 3074-3080, 1999), and, in recurrent AML, gene analysis at the first medical examination and at the time of recurrence had revealed a tendency that, upon recurrence, the frequency of abnormality was increased. It has also become apparent that, in positive cases of FLT3-ITD, the lifetime after recurrence is short (Y. Nakano et al., Br. J. Haematol., 104: 659-664. 1999). Furthermore, there is a report about diseases based on mutation of Flt3 other than Flt3-ITD (British Journal of Haematology, 113, 983-988 (2001)), and compounds represented by formula (I) are also effective for these diseases based on mutation.

Thus, the abnormality of the signal transduction system through Flt3 and/or Flt3-ITD is related to abnormality of growth and differentiation of haemopoietic cells and immune disorder. Accordingly, diseases caused by the abnormality of the signal transduction system through Flt3 and/or Flt3-ITD, particularly an enhancement in signal transfer system through Flt3 and/or Flt3-ITD, can be treated and prevented by inhibiting the autophosphorylation of Flt3.

Compounds of formula (I) inhibited Flt3 autophosphorylation of human leukemic cell line MV4-11 (see Pharmacological Test Example 1). Further, compounds of formula (I) actually inhibited in vitro the growth of human leukemic cell line MV4-11 (Pharmacological Test Example 2).

Compounds of formula (I) actually inhibited in vitro and in vivo the growth of human leukemic cell MOLM13 (Pharmacological Test Examples 3, 4, and 5).

Accordingly, compounds of formula (I) and pharmaceutically acceptable salts and solvates thereof are effective for the treatment and prevention of diseases where the inhibition of autophosphorylation of Flt3 and/or Flt3-ITD is therapeutically or prophylactically effective.

Diseases where the inhibition of autophosphorylation of Flt3 and/or Flt3-ITD is therapeutically or prophylactically effective include hematopoietic malignancies, for example, acute myelocytic leukemia and myelodysplastic syndrome, and immune diseases caused by abnormal growth of B cells, dendritic cells, or natural killer cells.

In a preferred embodiment of the pharmaceutical composition according to the present invention, there is provided a pharmaceutical composition for use in the treatment or prevention of a hematopoietic organ malignant tumor (more preferably an acute myelocytic leukemia or myelodysplastic syndrome), which comprises a compound represented by formula (Ia), a compound represented by formula (II), or a pharmaceutically acceptable salt or solvate thereof.

According to the present invention, there is provided a method for the treatment or prevention of a disease where the inhibition of autophosphorylation of Flt3 and/or Flt3-ITD is therapeutically or prophylactically effective, which comprises the step of administering a compound represented by formula (I) or a pharmaceutically acceptable salt or solvate thereof together with a pharmaceutically acceptable carrier, to a mammal.

Further, according to the present invention, there is provided use of a compound represented by formula (I) or a pharmaceutically acceptable salt or solvate thereof, for the manufacture of a medicament used in the treatment or prevention of diseases where the inhibition of autophosphorylation of Flt3 and/or Flt3-ITD is therapeutically or prophylactically effective.

The compounds according to the present invention can be administered to human and non-human animals either orally or parenterally by administration routes, for example, intravenous administration, intramuscular administration, subcutaneous administration, rectal administration, or percutaneous administration. Therefore, the pharmaceutical composition comprising the compound according to the present invention as an active ingredient is formulated into suitable dosage forms according to the administration routes.

Specifically, oral preparations include tablets, capsules, powders, granules, and syrups, and parental preparations include injections, suppositories, tapes, and ointments.

These various preparations may be prepared by conventional methods, for example, with commonly used excipients, disintegrants, binders, lubricants, colorants, and diluents.

Excipients include, for example, lactose, glucose, corn starch, sorbit, and crystalline cellulose. Disintegrants include, for example, starch, sodium alginate, gelatin powder, calcium carbonate, calcium citrate, and dextrin. Binders include, for example, dimethylcellulose, polyvinyl alcohol, polyvinyl ether, methylcellulose, ethylcellulose, gum arabic, gelatin, hydroxypropylcellulose, and polyvinyl pyrrolidone. Lubricants include, for example, talc, magnesium stearate, polyethylene glycol, and hydrogenated vegetable oils.

In preparing the injections, if necessary, for example, buffers, pH adjustors, stabilizers, tonicity agents, and preservatives may be added.

The content of the compound according to the present invention in the pharmaceutical composition according to the present invention may vary depending upon the dosage form. In general, however, the content is 0.5 to 50% by weight, preferably 1 to 20% by weight, based on the whole composition.

The dose may be appropriately determined in consideration of, for example, the age, weight, sex, difference in diseases, and severity of condition of individual patients, preferably, in the range of 1 to 100 mg/kg. This dose is administered at a time daily or divided doses of several times daily.

The compound according to the present invention may be administered in combination with other medicament, for example, a carcinostatic agent. In this case, the compound according to the present invention may be administered simultaneously with or after or before the administration of other medicament. The type, administration intervals and the like of the carcinostatic agent may be determined depending upon the type of cancer and the condition of patients.

### EXAMPLES

The present invention is further illustrated by the following Examples that are not intended as a limitation of the invention. Starting compounds necessary for the synthesis of Compounds 1 to 77 were produced as described in WO 97/17329, WO 98/47873, WO 00/43366, and Japanese Patent Laid-Open Publication No. 158149/1999. The starting compounds not described in these publications were synthesized as described in the following Production Examples.

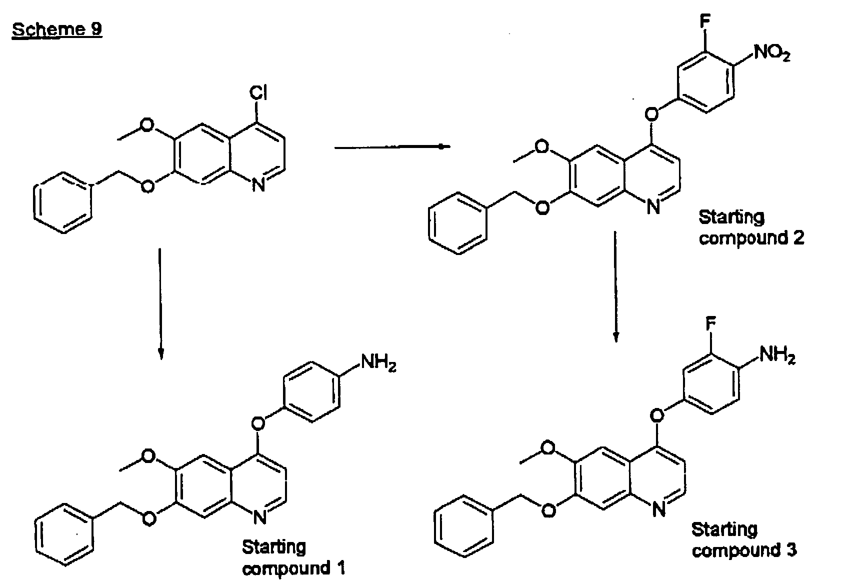

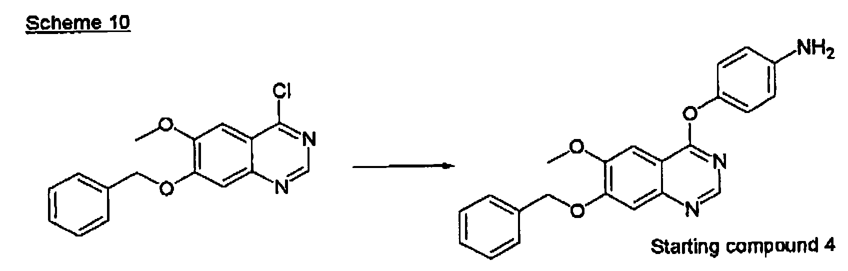

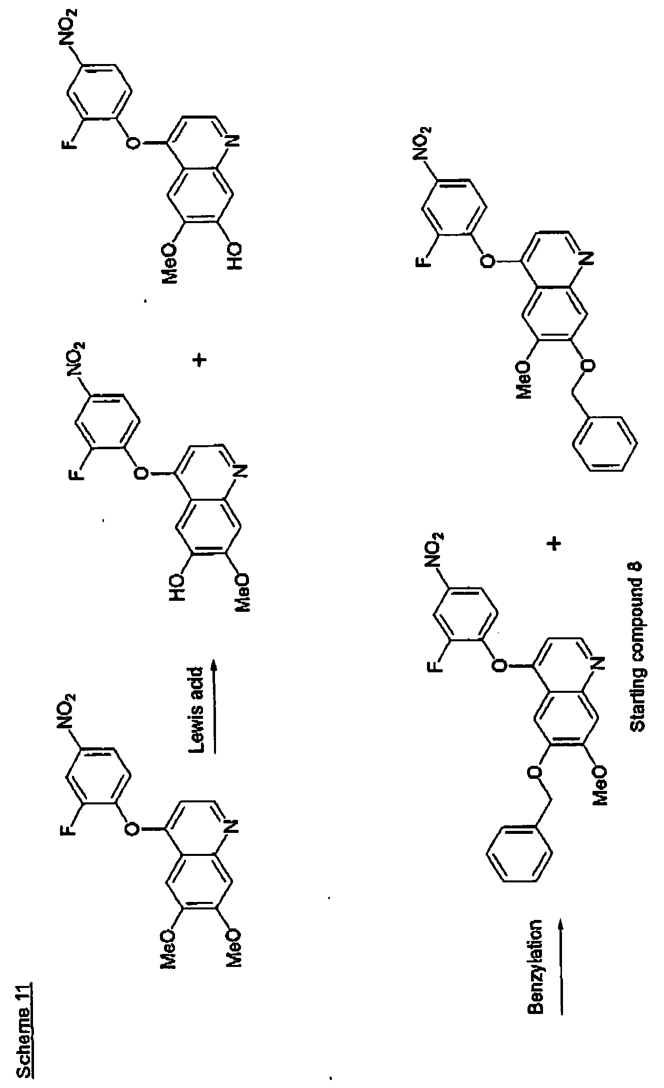

Starting compounds 5, 6, and 7 were synthesized according to scheme 6.

### Production Example 1 (starting compound 1)

4-Aminophenol (12.21 g) and sodium methoxide (28% methanol solution, 21.07 g) were dissolved in N,N-dimethylacetamide (140 ml), and the solution was stirred at room temperature for one hr. The solvent was removed by distillation under the reduced pressure. 7-(Benzyloxy)-4-chloro-6-methoxyquinoline (21.00 g) and N,N-dimethylacetamide (210 ml) were added to the residue, and the mixture was stirred at 120°C for 22 hr. The solvent was removed by distillation under the reduced pressure. Water (300 ml) was added to the residue, and the mixture was stirred at room temperature for 4 hr. The resultant precipitate was collected by filtration and was dried to give a contemplated compound (24.90 g, yield 96%).

### Production Example 2 (starting compound 2)

7-(Benzyloxy)-4-chloro-6-methoxyquinoline (9.00 g) and 3-fluoro-4-nitrophenol (5.66 g) were added to chlorobenzene (60 ml), and the mixture was stirred at 120°C for 21 hr. Chloroform (100 ml) and an aqueous sodium hydroxide solution (2.4 g of sodium hydroxide dissolved in 100 ml of water) were added to the reaction solution, and the mixture was stirred at room temperature overnight. The organic layer was extracted with chloroform, and the chloroform layer was washed with a saturated aqueous sodium hydrogencarbonate solution and saturated brine. The chloroform layer was dried over sodium sulfate, and the solvent was removed by distillation under the reduced pressure. The crude was washed with hexane/ethyl acetate (1/1), was filtered, and was dried to give a contemplated compound (10.39 g, yield 82%).

### Production Example 3 (starting compound 3)

7-(Benzyloxy)-4-(3-fluoro-4-nitrophenoxy)-6-methoxyquinoline (4.11 g), ammonium chloride (2.62 g) and zinc (12.80 g) were added to methanol (80 ml), and the mixture was stirred at 100°C for 3 hr. The reaction solution was filtered, and the filtrate was concentrated. A saturated aqueous sodium hydrogencarbonate solution was added to the crude, and the mixture was stirred at room temperature overnight. Chloroform was added to the solution, followed by extraction. The chloroform layer was dried over sodium sulfate. The solvent was removed by distillation under the reduced pressure to give a contemplated compound (1.80 g, yield 47%).

### Production Example 4 (starting compound 4)

7-(Benzyloxy)-4-chloro-6-methoxyquinazoline (500 mg) and tetra-n-butylammonium chloride (230 mg) were added to ethyl methyl ketone (20 ml) (solution A). 4-Aminophenol (270 mg) and sodium hydroxide (99 mg) were added to water (10 ml) (solution B). Solution A and solution B were mixed together, and the mixture was heated under reflux for 2 hr. Ethyl methyl ketone was removed by distillation under the reduced pressure, and the crude was extracted with chloroform. The chloroform layer was washed with a saturated aqueous sodium carbonate solution and saturated brine and was dried over anhydrous sodium sulfate. The solvent was removed by distillation under the reduced pressure, and the crude was purified by chromatography on silica gel, developing with chloroform/acetone to quantitatively give a contemplated compound.

### Production Example 5: Production of 2-amino-5-benzyloxy-4-methoxyacetophenone (starting compound 5)

3',4'-Dihydroxyacetophenone (20.1 g) was dissolved in N,N-dimethylformamide (320 ml), lithium carbonate (24.4 g) and methyl iodide (20.5 ml) were added thereto, and the mixture was stirred at 55°C overnight. The reaction solution was ice-cooled and was acidified by the addition of a 10% aqueous hydrochloric acid solution. Chloroform was added, and the mixture was extracted twice. The extract was washed with saturated brine, was dried over sodium sulfate and was then concentrated and dried. The solid was dissolved in N,N-dimethylformamide (200 ml). Potassium carbonate (21.8 g), tetrabutylammonium iodide (4.8 g) and benzyl bromide (18.9 ml) were added thereto, and the mixture was stirred at 100°C for one hr. Water was added thereto, and the mixture was extracted twice with chloroform. The extract was washed with saturated brine, was dried over sodium sulfate, and was then concentrated and dried. The solid was dissolved in acetic acid (95 ml), fuming nitric acid (13.6 ml) was added by portions under ice cooling, and the mixture was stirred at room temperature for 3 hr. Under ice cooling, the reaction solution was neutralized by the addition of a 10% aqueous sodium hydroxide solution. Chloroform was added to dissolve the resultant solid. The reaction solution was extracted twice with chloroform, and the extract was washed with saturated brine, was dried over sodium sulfate, and was then concentrated and dried. Ethanol was added to the solid, and the mixture was heated to 100°C for dissolution. Water (20 ml), ammonium chloride (21.1 g), and zinc powder (112 g) were added thereto, and the mixture was stirred at 100°C for one hr. The reaction solution was filtered while hot and was washed with a chloroform-methanol mixed solution. The mother liquor was concentrated, ethyl acetate and 10% sodium hydroxide were added to the residue, and the mixture was vigorously stirred. The insolubles were then removed by filtration. The mother liquor was extracted with ethyl acetate, was washed with saturated brine, was dried over sodium sulfate, and was then concentrated and dried. The residue was purified by chromatography on silica gel, developing with hexane/ethyl acetate/dichloromethane to give the title compound (13.1 g, yield 37%) (four steps).
¹H-NMR (CDCl₃, 400 MHz): 2.39 (s, 3H), 3.89 (s, 3H), 5.05 (s, 2H), 6.25 (s, 1 H), 7.15 (s, 1 H), 7.29 - 7.45 (m, 5H)

### Production Example 6: Production of 6-benzyloxy-7-methoxy-4-quinolone (starting compound 6)

2-Amino-5-benzyloxy-4-methoxyacetophenone (13.1 g), tetrahydrofuran (anhydrous) (200 ml), and sodium methoxide (13.1 g) were added, and the mixture was stirred at room temperature for 30 min. Ethyl formate (19.4 ml) was added thereto, and the mixture was further stirred at room temperature for one hr. Water was added, and the mixture was stirred at room temperature for one hr, followed by concentration under the reduced pressure. The concentrate was weakly acidified by the addition of a 10% aqueous hydrochloric acid solution, chloroform was added thereto, followed by extraction. The extract was washed with saturated brine and was dried over sodium sulfate, and the solvent was then removed by distillation under the reduced pressure. The crude was purified by chromatography on silica gel, developing with chloroform/methanol to give the title compound (11.5 g, yield 85%).
¹H-NMR (CDCl₃, 400 MHz): δ 3.97 (s, 3H), 5.19 (s, 2H), 6.28 (d, J = 7.3 Hz, 1H), 7.02 (s, 1 H), 7.29 - 7.41 (m, 3H), 7.47 - 7.51 (m, 2H), 7.71 (s, 1H), 7.86 (d, J = 7.3 Hz, 1H)

### Production Example 7: Production of 6-benzyloxy-4-chloro-7-methoxyquinoline (starting compound 7)

6-Benzyloxy-7-methoxy-4-quinolone (2.4 g), diisopropylamine (7.4 ml), and phosphorus oxychloride (2.0 ml) were added, and the mixture was stirred at 110°C for one hr. The reaction solution was concentrated under the reduced pressure, and chloroform and ice water were then added to the concentrate. The mixture was weakly acidified by the addition of 28% aqueous ammonia and was extracted with chloroform. The extract was washed with saturated brine and was dried over sodium sulfate, and the solvent was then removed by distillation under the reduced pressure. The crude was purified by chromatography on silica gel, developing with chloroform/methanol to give the title compound (1.6 g, yield 63%).
¹H-NMR (CDCl₃, 400 MHz): δ 4.04 (s, 3H), 5.32 (s, 2H), 7.32 - 7.44 (m, 4H), 7.45 (s, 1 H), 7.49 (s, 1H), 7.51 - 7.55 (m, 2H), 8.57 (d, J = 4.9 Hz, 1 H)
Mass spectrometric value (ESI-MS, m/z): 300 (M+1)

### Production Example 8: Production of 4-[(6-benzyloxy-7-methoxy-4-quinolyl)oxy]-3-fluoro-nitrobenzene (starting compound 8)

4-[(6,7-Dimethoxy-4-quinolyl)oxy]-3-fluoro-nitrobenzene (4.3 g) was dissolved in chloroform (200 ml), aluminum chloride (10 g) was added thereto, and the mixture was heated under reflux for 2 hr. The solvent was removed by distillation, and water (200 ml) was then carefully added to the residue. The precipitated crude crystal (6.5 g) was collected by filtration. The crude crystal was dissolved in dimethylformamide (150 ml), potassium carbonate (9.0 g) and benzyl chloride (4.5 g) were added thereto, and the mixture was stirred at room temperature for 5 hr. The reaction solution was extracted with ethyl acetate, the extract was then washed with saturated brine and was dried over anhydrous sodium sulfate, and the solvent was removed by distillation under the reduced pressure. The residue was purified by column chromatography on silica gel, and the title compound was obtained from n-hexane : ethyl acetate (1 : 4) fraction (1.4 g, yield 27%).
¹H-NMR (CDCl₃, 400 MHz): 4.04 (s, 3H), 5.26 (s, 2H), 6.57 (d, J = 5.1 Hz, 1 H), 7.15 - 7.47 (m, 6H), 7.33 (s, 1H), 7.47 (s, 1 H), 8.02 - 8.05 (m, 1 H), 8.13 - 8.16 (m, 1 H), 8.57 (d, J = 5.1 Hz, 1H)

### Production Example 9: Production of N-(2-hydroxyethyl)-2,2,6,6-tetramethylpiperidine

2,2,6,6-Tetramethylpiperidine (2.78 g) was dissolved in anhydrous chloroform (80 ml), and triethylamine (10.1 g) was added thereto. Subsequently, chloroglyoxylic acid ethyl ester (5.40 g) dissolved in anhydrous chloroform (5 ml) was added at 0°C, and the mixture was stirred at room temperature for 20 hr. A saturated aqueous sodium hydrogencarbonate solution was added thereto, and the organic layer was separated. The organic layer was washed with saturated brine and was dried over anhydrous sodium sulfate, and the solvent was removed by distillation under the reduced pressure. The residue was purified by column chromatography on silica gel, eluting with n-hexane : ethyl acetate (4 : 1) mixed solvent to give N-(glyoxylic ethyl ester)-2,2,6,6-tetramethylpiperidine (4.50 g, yield 94%).
¹H-NMR (CDCl₃, 400 MHz): 0.85 (t, J = 6.8 Hz, 3H), 1.46 (s, 12H), 1.67 (s, 3H), 4.25 (q, J = 6.8 Hz, 2H)

N-(Glyoxylic ethyl ester)-2,2,6,6-tetramethylpiperidine (4.50 g) was dissolved in tetrahydrofuran (100 ml), lithium aluminium hydride (2.14 g) was added thereto at 0°C, and the mixture was then heated under reflux for one hr. The excess reagent was decomposed with sodium sulfate decahydrate, followed by filtration through Celite. The filtrate was concentrated under the reduced pressure to give the title compound (3.40 g, yield 100%).
¹H-NMR (CDCl₃, 400 MHz): 1.02 (s, 12H), 1.41 - 1.65 (m, 6H), 2.68 - 2.72 (m, 2H), 2.95 (br s, 1 H), 3.41 - 3.45 (m, 2H)

### Compound 1: 1-[4-(6,7-Dimethoxy-quinolin-4-yloxy)-phenyl]-3-(3,3-dimethyl-butyl)-urea hydrochloride

4-[(6,7-Dimethoxy-quinolyl)oxy]aniline (starting compound A) (2 g) was dissolved in chloroform (100 ml), and triethylamine (2 ml) was added thereto. A solution of triphosgene (1 g) in chloroform (4 ml) was added dropwise, and the mixture was stirred at room temperature for 30 min. 3,3-Dimethylbutylamine (starting, compound B) (750 mg) was added thereto, and the mixture was stirred at room temperature for 5 hr. Water and chloroform were added to the reaction solution, and the mixture was extracted with chloroform. The extract was washed with saturated brine and was dried over sodium sulfate, and the solvent was then removed by distillation under the reduced pressure. The crude was purified by chromatography on silica gel, developing with chloroform/acetone to give 1-[4-(6,7-dimethoxy-quinolin-4-yloxy)-phenyl]-3-(3,3-dimethyl-butyl)-urea (1.70 g, yield 59%).
¹H-NMR (CDCl₃, 400 MHz): 0.93 (s, 9H), 1.42 - 1.46 (m, 2H), 3.27 - 3.32 (m, 2H), 4.03 (s, 3H), 4.03 (s, 3H), 5.03 (br, 1H), 6.44 (d, J = 5.3 Hz, 1H), 7.11 (d, J = 9.0 Hz, 2H), 7.41 (s, 1H), 7.43 (d, J = 8.8 Hz, 2H), 7.55 (s, 1 H), 8.46 (d, J = 5.1 Hz, 1 H), 8.84 (br, 1H)
Mass spectrometric value (ESI-MS, m/z): 424 (M⁺+1)

Methanol (20 ml) and chloroform (2 ml) were added to and dissolved in 1-[4-(6,7-dimethoxy-quinolin-4-yloxy)-phenyl]-3-(3,3-dimethyl-butyl)-urea, and the solution was acidified by the addition of hydrogen chloride methanol and was concentrated. Diethyl ether was added to the residue, and the mixture was filtered to give the title compound (1.75 g, yield 91%).
¹H-NMR (CDCl₃, 400 MHz): 0.92 (s, 9H), 1.45 - 1.49 (m, 2H), 3.24 - 3.30 (m, 2H), 4.10 (s, 3H), 4.14 (s, 3H), 5.98 (br, 1H), 6.48 (d, J = 6.6 Hz, 1H), 7.02 (d, J = 9.0 Hz, 2H), 7.65 (s, 1 H), 7.72 (d, J = 9.0 Hz, 2H), 7.88 (s, 1H), 8.18 (d, J = 6.6 Hz, 1H), 8.84 (br, 1 H)
Mass spectrometric value (ESI-MS, m/z): 424 (M⁺+1)

### Compound 41: 1-(3,3-Dimethyl-butyl)-3-{2-fluoro-4-[6-methoxy-7-(2-morpholin-4-yl-ethoxy)-quinolin-4-yloxy]-phenyl}-urea hydrochloride

4-[(7-Benzyloxy-6-methoxy-4-quinolyl)oxy]-2-fluoro-aniline (starting compound A) (3.0 g) was dissolved in anhydrous chloroform (100 ml), and triethylamine (3.9 g) was added thereto. Subsequently, triphosgene (2.3 g) dissolved in anhydrous chloroform (5 ml) was added, and the mixture was stirred at room temperature for 30 min. 3,3-Dimethylbutylamine (starting compound B) (1.6 g) dissolved in anhydrous chloroform (5 ml) was then added thereto, and the mixture was further stirred at room temperature for one hr. Saturated sodium hydrogencarbonate solution was added thereto, the mixture was stirred, and the organic layer was then separated. The organic layer was washed with saturated brine and was dried over anhydrous sodium sulfate, and the solvent was removed by distillation under the reduced pressure. The residue was purified by column chromatography on silica gel, and 1-[4-([7-benzyloxy-6-methoxy-quinolin-4-yloxy]-2-fluorophenyl)-3-(3,3-dimethyl-butyl)urea was obtained from chloroform : methanol (98 : 2) fraction (3.9 g, yield 97%),
¹H-NMR (CDCl₃, 400 MHz): 0.93 (s, 9H), 1.43 - 1.47 (m, 2H)3.26 - 3.31 (m, 2H), 4.01 (s, 3H), 4.78 (br s, 1H), 5.30 (s, 2H), 6.45 (d, J = 5.4 Hz, 1H), 6.57 (br s, 1 H), 6.88 - 6.95 (m, 2H), 7.28 - 7.49 (m, 5H), 7.44 (s, 1H), 7.50 (s, 1H), 8.14 (t, J = 8.8 Hz, 1H), 8.45 (d, J = 5.4 Hz, 1 H)

1-[4-([7-Benzyloxy-6-methoxy-quinolin-4-yloxy]-2-fluorophenyl)-3-(3,3-dimethyl-butyl)urea (11 g) prepared above was suspended in trifluoroacetic acid (20 ml) and methanesulfonic acid (1 ml), and the suspension was heated under refluxed for one hr. The solvent was removed by distillation under the reduced pressure, and water was added to the residue. The solution was adjusted to substantially pH 7 by the addition of a 10% sodium hydroxide solution. The resultant precipitate was collected by filtration to give 1-(3,3-dimethyl-butyl)-3-[2-fluoro-4-(7-hydroxy-6-methoxy-quinolin-4-yloxy)-phenyl]-urea. N, N-Dimethylformamide (2 ml) was added to 1-(3,3-dimethyl-butyl)-3-[2-fluoro-4-(7-hydroxy-6-methoxy-quinolin-4-yloxy)-phenyl]-urea (103 mg), potassium carbonate (166 mg), and 4-(2-chloroethyl)morpholine hydrochloride (starting compound C) (69 mg), and the mixture was stirred at 75 to 80°C for 16 hr. Water and ethyl acetate were added to the reaction solution, the mixture was extracted with ethyl acetate. The extract was washed with saturated brine and was dried over sodium sulfate, and the solvent was then removed by distillation under the reduced pressure. The crude was purified by thin layer chromatography on silica gel, developing with chloroform/methanol to give 1-(3,3-dimethyl-butyl)-3-{2-fluoro-4-[6-methoxy-7-(2-morpholin-4-yl-ethoxy)-quinolin-4-yloxy]-phenyl}-urea (47.7 mg, yield 37%).
¹H-NMR (CDCl₃+CD₃OD, 400 MHz): 0.96 (s, 9H), 1.45 - 1.51 (m, 2H), 2.72 (br, 4H), 3.02 (t, J = 5.6 Hz, 2H), 3.28 - 3.34 (m, 2H), 3.78 - 3.81 (m, 4H), 4.02 (s, 3H), 4.40 (t, J = 5.6 Hz, 2H), 5.16 (br, 1H), 6.51 (d, J = 5.6 Hz, 1H), 6.89 (dd, J = 2.7, 11.2 Hz, 1H), 6.91 (br, 1H), 6.95 - 6.97 (m, 1H), 7.52 (s, 1H), 7.55 (s, 1H), 8.24 (dd, J = 9.0, 9.0 Hz, 1H), 8.46 (d, J = 5.6 Hz, 1H)
Mass spectrometric value (ESI-MS, m/z): 541 (M⁺ +1)

1-(3,3-Dimethyl-butyl)-3-{2-fluoro-4-[6-methoxy-7-(2-morpholin-4-yl-ethoxy)-quinolin-4-yloxy]-phenyl}-urea (42.7 mg) was dissolved in chloroform(1 ml)/methanol (1 ml). Hydrogen chloride methanol was added thereto, the mixture was concentrated using an evaporator, and the concentrate was dried under the reduced pressure through a vacuum pump to give the title compound (48.9 mg).
¹H-NMR (CDCl₃+CD₃OD, 400 MHz): 0.96 (s, 9H), 1.45 - 1.51 (m, 2H), 3.22 - 3.32 (m, 4H), 3.71 - 3.80 (m, 4H), 4.00 - 4.10 (m, 5H), 4.18 - 4.28 (m, 2H), 4.94 (br, 2H), 6.84 (d, J = 5.1 Hz, 1H), 6.97 (d, J = 9.0 Hz, 2H), 7.64 (s, 1 H), 8.01 (s, 1H), 8.38 (t, J = 9.0 Hz, 1H), 8.57 (d, J = 4.6 Hz, 1 H)
Mass spectrometric value (ESI-MS, m/z): 563 (M+Na)⁺

### Compound 43: 1-(3,3-Dimethyl-butyl)-3-{2-fluoro-4-[6-methoxy-7-(2-piperidin-1-yl-ethoxy)-quinolin-4-yloxy]-phenyl}-urea hydrochloride

In the same manner as in compound 41, 1-[4-([7-benzyloxy-6-methoxy-quinolin-4-yloxy]-2-fluorophenyl)-3-(3,3-dimethyl-butyl)urea was synthesized from 4-[(7-benzyloxy-6-methoxy-4-quinolyl)oxy]-2-fluoroaniline (starting compound A) and 3,3-dimethylbutylamine (starting compound B) and was debenzylated to give a crude product of a 7-hydroxyurea compound. The crude product was dissolved in dimethylformamide (100 ml), potassium carbonate (18 g) and 1-bromo-2-chloroethane (starting compound C) (11 g) were added thereto, and the mixture was stirred at room temperature for 20 hr. The reaction solution was extracted with ethyl acetate, and the extract was then washed with saturated brine and was dried over anhydrous sodium sulfate. The solvent was removed by distillation under the reduced pressure. The residue was washed with n-hexane : ethyl acetate (2 : 1) mixed solvent and was then collected by filtration to give 1-{4-[7-(2-chloroethoxy)-6-methoxy-quinolin-4-yloxy]-2-fluorophenyl}-3-(3,3-dimethyl-butyl)urea (7.7 g, yield 74%).
¹H-NMR (CDCl₃, 400 MHz): 0.94 (s, 3H), 1.44 - 1.48 (m, 2H), 3.26 - 3.32 (m, 2H), 3.91 - 3.95 (m, 2H), 4.01 (s, 3H), 4.41 - 4.45 (m, 2H), 4.79 - 4.81 (m, 1 H), 6.47 (d, J = 5.4 Hz, 1H), 6.55 - 6.57 (m, 1H), 6.89 - 6.96 (m, 2H), 7.40 (s, 1 H), 7.51 (s, 1 H), 8.10 (t, J = 8.8 Hz, 1H), 8.47 (d, J = 5.4 Hz, 1H)

N,N-Dimethylformamide (80 ml) was added to 1-{4-[7-(2-chloroethoxy)-6-methoxy-quinolin-4-yloxy]-2-fluorophenyl}-3-(3,3-dimethyl-butyl)urea (1.98 g) prepared above, potassium carbonate (2.82 g), and piperidine (starting compound D) (2.02 ml), and the mixture was stirred at 70 to 75°C for 17 hr. Piperidine (starting compound D) (0.8 ml) was added, and the mixture was further stirred at 70 to 75°C for 23 hr. Water and ethyl acetate were added to the reaction solution, and the mixture was extracted with ethyl acetate. The extract was washed with saturated brine and was dried over sodium sulfate, and the solvent was then removed by distillation under the reduced pressure. The crude was purified by alumina (grade III) chromatography, developing with chloroform/methanol to give 1-(3,3-dimethyl-butyl)-3-{2-fluoro-4-[6-methoxy-7-(2-piperidin-1-yl-ethoxy)-quinolin-4-yloxy]-phenyl}-urea (1.69 g, yield 78%).
¹H-NMR (CDCl₃, 400 MHz): δ 0.95 (s, 9H), 1.43 - 1.52 (m, 4H), 1.62 - 1.70 (m, 4H), 2.53 - 2.62 (m, 4H), 2.92 (t, J = 5.9 Hz, 2H), 3.24 - 3.31 (m, 2H), 4.02 (s, 3H), 4.32 (t, J = 5.9 Hz, 2H), 6.48 (d, J = 5.4 Hz, 1H), 6.87 - 6.97 (m, 2H), 7.38 (s, 1H), 7.52 (s, 1 H), 8.19 - 8.26 (m, 1 H), 8.43 (d, J = 5.4 Hz, 1 H)
Mass spectrometric value (ESI-MS, m/z): 539 (M+1)

Methanol (20 ml) and chloroform (2 ml) were added to and dissolved in 1-(3,3-dimethyl-butyl)-3-{2-fluoro-4-[6-methoxy-7-(2-piperidin-1-yl-ethoxy)-quinolin-4-yloxy]-phenyl}-urea, and the solution was acidified by the addition of hydrogen chloride methanol. The acidified solution was concentrated, and diethyl ether was added to the residue, followed by filtration to give the title compound (1.75 g, yield 91%).
¹H-NMR (CDCl₃, 400 MHz): δ 0.93 (s, 9H), 1.46 - 1.52 (m, 2H), 1.78-1.96 (m, 4H), 2.13 - 2.27 (m, 2H), 3.03 - 3.12 (m, 2H), 3.21 - 3.27 (m, 2H), 3.68 - 3.83 (m, 4H), 4.05 (s, 3H), 4.87 - 4.94 (m, 2H), 6.82 (d, J = 6.6 Hz, 1H), 6.87 - 6.96 (m, 2H), 7.58 (s, 1H), 7.97 (s, 1 H), 8.30 - 8.33 (m, 1H), 8.56 (d, J = 6.8 Hz, 1H)
Mass spectrometric value (ESI-MS, m/z): 539 (M+1)

### Compound 50: 1-{2-Chloro-4-[6-methoxy-7-(2-piperidin-2-yl-ethoxy)-quinolin-4-yloxy]-phenyl}-3-(3,3-dimethyl-butyl)-urea

2-Piperidine ethanol (starting compound A) (1.93 g, 15 mmol) and triethylamine (5 ml) were dissolved in chloroform (25 ml). Di-tert-butyl dicarbonate (3.3 g, 15 mmol) was dissolved in chloroform (5 ml), the solution was added to the mixed solution, and the mixture was stirred at room temperature for 2 hr. The solvent was removed by distillation, water was added to the residue, and the mixture was extracted with ethyl acetate. The organic layer was washed with saturated brine and was dried over sodium sulfate. The reaction solution was concentrated. The compound (1.5 g) thus obtained, triphenylphosphine (1.5 g, 5.7 mmol), and 1-{2-chloro-4-[7-hydroxy-6-methoxy-quinolin-4-yloxy]-phenyl}-3-(3,3-dimethyl-butyl)-urea (starting compound B) (1.12 g, 2.5 mmol) were dissolved in tetrahydrofuran (30 ml), and the solution was cooled to 0°C. 40% diethylazodicarboxylate (8 ml) was added thereto, and the temperature was returned to room temperature before the mixture was stirred for 3 days. Water was added thereto, and the mixture was extracted with ethyl acetate. The organic layer was washed with water and saturated brine and was dried over sodium sulfate. The reaction solution was concentrated, and the concentrate was loaded on a chromatographic silica gel column and developed with chloroform/methanol (15/1) to give tert-butyl 2-[({4-[3-chloro-4-({[(3,3-dimethylbutyl)amino]carbonyl}amino)phenoxy]-6-methoxy-7-quinolyl}oxy)methyl]-1-piperidine carboxylate. Next, 25% trifluoromethylacetic acid was added to tart-butyl 2-[({4-[3-chloro-4-({[(3,3-dimethylbutyl)amino]carbonyl}amino)phenoxy]-6-methoxy-7-quinolyl}oxy)methyl]-1-piperidine carboxylate, and the mixture was stirred at room temperature for one hr. The solvent was removed by distillation, and the residue was loaded on a chromatographic silica gel column and developed with chloroform/methanol to give the title compound (yield 50%, 694 mg).
Mass spectrometric value (ESI-MS, m/z): 556 (M⁺+1)

### Compound 61: 1-(3,3-Dimethyl-butyl)-3-(2-fluoro-4-[6-methoxy-7-[2-(2,2,6,6-tetramethyl-piperidin-1-yl)-ethoxy]-quinolin-4-yloxy}-phenyl)-urea

4-(3-Fluoro-4-nitrophenoxy)-6-methoxy-7-quinolinol (450 mg) (starting compound A) and N-(2-hydroxyethyl)-2,2,6,6-tetrahydropiperidine (500 mg) (starting compound B) produced in Production Example 9 were dissolved in tetrahydrofuran (40 ml), triphenylphosphine (1.80 g) was added thereto, and the mixture was stirred at room temperature for 10 min. Diethylazocarboxylate (40% toluene solution, 3.2 ml) was added thereto, and the mixture was stirred at room temperature for 20 hr. The reaction solution was extracted with ethyl acetate, the organic layer was washed with saturated brine and was dried over anhydrous sodium sulfate, and the solvent was then removed by distillation under the reduced pressure. The residue was purified by column chromatography on silica gel, eluting with methanol : ethyl acetate (1 : 99) mixed solvent to give 4-(3-fluoro-4-nitrophenoxy)-6-methoxy-7-[2-(2,2,6,6-tetramethylpiperidino)ethoxy]quinoline (315 mg, yield 49%).

4-(3-Fluoro-4-nitrophenoxy)-6-methoxy-7-[2-(2,2,6,6-tetramethylpiperidino)ethoxy]quinoline (315 mg) was dissolved in methanol (30 ml), ammonium chloride (170 mg) and zinc (820 mg) were added thereto, and the mixture was then heated under reflux for 5 hr. The reaction solution was filtered through Celite, and the filtrate was then treated with an aqueous sodium hydrogencarbonate solution and was extracted with chloroform. The organic layer was dried over anhydrous sodium sulfate, and the solvent was then removed by distillation under the reduced pressure. The residue was purified by column chromatography on silica gel, eluting with n-hexane : ethyl acetate (3 : 1) mixed solvent to give 2-fluoro-4-({6-methoxy-7-[2-(2,2,6,6-tetramethylpiperidino)ethoxy]-4-quinolyl}oxy)aniline (63 mg, yield 21%).

2-Fluoro-4-({6-methoxy-7-[2-(2,2,6,6-tetramethylpiperidino)-ethoxy]-4-quinolyl}oxy)aniline (63 mg) was dissolved in anhydrous chloroform (15 ml), triethylamine (68 mg) and triphosgene (40 mg) were added thereto, and the mixture was then stirred at room temperature for 30 min. 3,3-Dimethylbutylamine (41 mg) (starting compound C) was added thereto, and the mixture was further stirred at room temperature for one hr. The reaction solution was treated with an aqueous sodium hydrogencarbonate solution and was then extracted with chloroform. The organic layer was dried over anhydrous sodium sulfate, and the solvent was then removed by distillation under the reduced pressure. The residue was purified by neutral alumina (grade III) chromatography, eluting with methanol : chloroform (0.5 : 99.5) mixed solvent to give the title compound (70 mg, yield 90%).
¹H-NMR (CDCl₃, 400 MHz): 0.88 (s, 9H), 1.12 (s, 12H), 1.32 - 1.52 (m, 8H), 2.95 - 3.00 (m, 2H), 3.21 - 3.27 (m, 2H), 3.96 (s, 3H), 4.00 - 4.17 (m, 2H), 5.03 - 5.06 (m, 1H), 6.39 (d, J = 5.4 Hz, 1H), 6.77 - 6.93 (m, 3H), 7.36 (s, 1H), 7.42 (s, 1H), 8.11 (t, J = 9.0 Hz, 1H), 8.41 (d, J = 5.4 Hz, 1H)
Mass spectrometric value (ESI-MS, m/z): 595 (M⁺+1)

Compound 1, compound 41, compound 43, compound 50, and compound 61 had the following chemical structures.

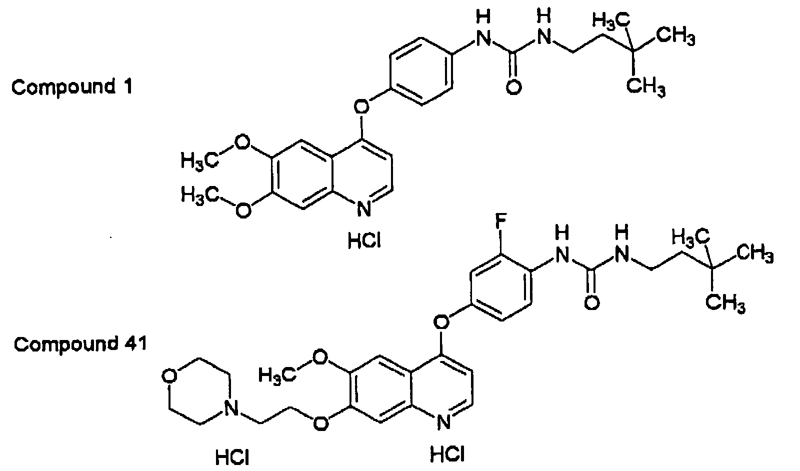

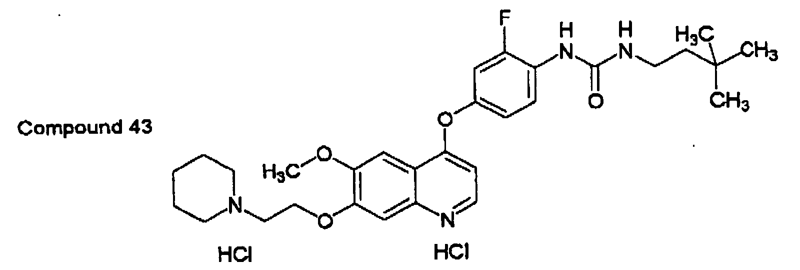

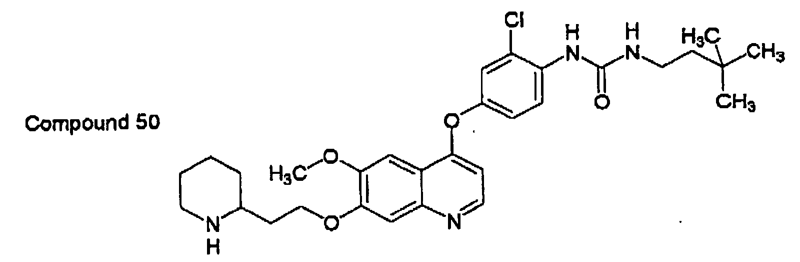

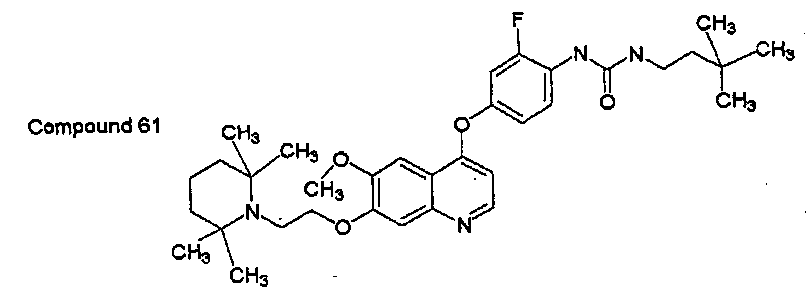

The following compounds were synthesized according to the Synthesis Examples of the above compounds.

| Compound No. | Name of compound |
|---|---|
| 2 | 1-[4-(6,7-Dimethoxy-quinolin-4-yloxy)-2-methyl-phenyl]-3-(3,3-dimethyl-butyl)-urea |
| 3 | 1-[4-(6,7-Dimethoxy-quinolin-4-yloxy)-3-methyl-phenyl-3-(3,3-dimethyl-butyl)-urea |
| 4 | 1-[4-(6,7-Dimethoxy-quinolin-4-yloxy)-2-methoxy-phenyl]-3-(3,3-dimethyl-butyl)-urea |
| 5 | 1-[4-(6,7-Dimethoxy-quinolin-4-yloxy)-3-methoxy-phenyl]-3-(3,3-dimethyl-butyl)-urea |
| 6 | 1-[3,5-Dichloro-4-(6,7-dimethoxy-quinolin-4-yloxy)-phenyl]-3-(3,3-dimethyl-butyl)-urea |
| 7 | 1-[4-(6,7-Dimethoxy-quinolin-4-yloxy)-2,3-dimethyl-phenyl]-3-(3,3-dimethyl-butyl)-urea |
| 8 | 1-[3-Chloro-4-(6,7-dimethoxy-quinolin-4-yloxy)-phenyl]-3-(3,3-dimethyl-butyl)-urea |
| 9 | 1-[4-(6,7-Dimethoxy-quinolin-4-yloxy)-2-fluoro-phenyl]-3-(3,3-dimethyl-butyl)-urea hydrochloride |
| 10 | 1-[4-(6,7-Dimethoxy-quinolin-4-yloxy)-3-fluoro-phenyl]-3-(3,3-dimethyl-butyl)-urea hydrochloride |
| 11 | 1-[2-Chloro-4-(6,7-dimethoxy-quinolin-4-yloxy)-phenyl]-3-(3,3-dimethyl-butyl)-urea |
| 12 | 1-[3-Chloro-4-(6,7-dimethoxy-quinolin-4-yloxy)-phenyl]-3-(3,3-dimethyl-cyclohexyl)-urea hydrochloride |
| 13 | 1-[3-Chloro-4-(6,7-dimethoxy-quinolin-4-yloxy)-phenyl]-3-(3,3,5-trimethyl-cyclohexyl)-urea |
| 14 | 1-[4-(6,7-Dimethoxy-quinolin-4-yloxy)-3-fluoro-phenyl]-3-(3,3-dimethyl-cyclohexyl)-urea |
| 15 | 1-[4-(6,7-Dimethoxy-quinolin-4-yloxy)-3-fluoro-phenyl]-3-(3,3,5-trimethyl-cyclohexyl)-urea |
| 16 | 1-(2-Cyclohexyl-ethyl)-3-[4-(6,7-dimethoxy-quinolin-4-yloxy)-3-fluoro-phenyl]-urea |
| 17 | 1-[3-Chloro-4-(6,7-dimethoxy-quinolin-4-yloxy)-phenyl]-3-(2-cyclohexyl-ethyl)-urea |
| 18 | 1-(2-Cyclopentyl-ethyl)-3-[4-(6,7-dimethoxy-quinolin-4-yloxy)-3-fluoro-phenyl]-urea |
| 19 | 1-Butyl 3-[4-(6,7-dimethoxy-quinolin-4-yloxy)-phenyl]-urea |
| 20 | 1-[4-(6,7-Dimethoxy-quinolin-4-yloxy)-phenyl]-3-isopropyl-urea |
| 21 | 1-[4-(6,7-Dimethoxy-quinolin-4-yloxy)-phenyl]-3-propyl-urea |
| 22 | 1-[4-(6,7-Dimethoxy-quinolin-4-yloxy)-phenyl]-3-hexyl-urea |
| 23 | 1-[4-(6,7-Dimethoxy-quinolin-4-yloxy)-phenyl]-3-pentyl-urea |
| 24 | 1-[4-(6,7-Dimethoxy-quinolin-4-yloxy)-phenyl]-3-(3-methoxy-propyl)-urea |
| 25 | 1-[4-(6,7-Dimethoxy-quinolin-4-yloxy)-phenyl]-3-(2-dimethylamino-ethyl)-urea |
| 26 | 1-Cyclohexyl-3-[4-(6,7-dimethoxy-quinolin-4-yloxy)-phenyl]-urea |
| 27 | 1-[4-(6,7-Dimethoxy-quinolin-4-yloxy)-phenyl]-3-methyl-urea |
| 28 | 1-[4-(6,7-Dimethoxy-quinolin-4-yloxy)-phenyl]-3-ethyl-urea |
| 29 | 1-[4-(6,7-Dimethoxy-quinolin-4-yloxy)-phenyl]-3-(3-methyl-butyl)-urea |
| 30 | 1-Cyclohexylmethyl-3-[4-(6,7-dimethoxy-quinolin-4-yloxy)-phenyl]-urea |
| 31 | 1-(2-Cyclohexyl-ethyl)-3-[4-(6,7-dimethoxy-quinolin-4-yloxy)-phenyl]-urea |
| 32 | 1-[4-(6,7-Dimethoxy-quinolin-4-yloxy)-phenyl]-3-(2,2-dimethyl-propyl)-urea |
| 33 | 1-(3-Cyclohexyl-propyl)-3-[4-(6,7-dimethoxy-quinolin-4-yloxy)-phenyl]-urea |
| 34 | 1-[4-(6,7-Dimethoxy-quinolin-4-yloxy)-phenyl]-3-(4-methyl-pentyl)-urea |
| 35 | 1-[4-(6,7-Dimethoxy-quinolin-4-yloxy)-phenyl]-3-(4,4-dimethyl-pentyl)-urea |
| 36 | 1-[4-(6,7-Dimethoxy-quinazolin-4-yloxy)-phenyl]-3-(3,3-dimethyl-butyl)-urea |
| 37 | 1-[2-Nitro-4-(6,7-dimethoxy-quinolin-4-yloxy)-phenyl]-3-(3, 3-dimethyl-butyl)-urea |
| 39 | 1-[4-(6,7-Dimethoxy-quinolin-4-yloxy)-phenyl]-3-(4-hydroxy-3,3-dimethyl-butyl)-urea |
| 40 | 1-{4-[6-Methoxy-7-(2-piperidin-1-yl-ethoxy)-quinolin-4-yloxy]-phenyl}-3-(3,3-dimethyl-butyl)-urea hydrochloride |
| 42 | 1-{2-Chloro-4-[6-methoxy-7-(2-piperidin-1-yl-ethoxy)-quinolin-4-yloxy]-phenyl}-3-(3,3-dimethyl-butyl)-urea |
| 44 | 1-(3,3-Dimethyl-butyl)-3-{3-fluoro-4-[6-methoxy-7-(2-piperidin-1-yl-ethoxy)-quinolin-4-yloxy]-phenyl}-urea |
| 45 | 1-{2-Chloro-4-[6-methoxy-7-(3-pyrrolidin-1-yl-propoxy)-quinolin-4-yloxy]-phenyl}-3-(3,3-dimethyl-butyl)-urea |
| 46 | 1-{2-Chloro-4-[6-methoxy-7-(3-piperidin-1-yl-propoxy)-quinolin-4-yloxy]-phenyl}-3-(3,3-dimethyl-butyl)-urea |
| 47 | 1-{2-Chloro-4-[7-(3-diethylamino-propoxy)-6-methoxy-quinolin-4-yloxy]-phenyl}-3-(3,3-dimethyl-butyl)-urea |
| 48 | 1-{2-Chloro-4-[6-methoxy-7-(2-pyrrolidin-1-yl-ethoxy)-quinolin-4-yloxy]-phenyl}-3-(3,3-dimethyl-butyl)-urea |
| 49 | 1-{2-Chloro-4-[6-methoxy-7-(2-piperidin-4-yl-ethoxy)-quinolin-4-yloxy]-phenyl}-3-(3,3-dimethyl-butyl)-urea |
| 51 | 1-(2-Chloro-4-{6-methoxy-7-[2-(4-methyl-piperidin-1-yl)-ethoxy]-quinolin-4-yloxy}-phenyl)-3-(3,3-dimethyl-butyl)-urea |
| 52 | 1-(2-Chloro-4-{6-methoxy-7-[3-(4-methyl-piperidin-1-yl)-propoxy]-quinolin-4-yloxy}-phenyl)-3-(3,3-dimethyl-butyl)-urea |
| 53 | 1-(3,3-Dimethyl-butyl)-3-(4-{7-[2-(3,5-dimethyl-piperidin-1-yl)-ethoxy]-6-methoxy-quinolin-4-yloxy}-phenyl)-urea |
| 54 | 1-(3,3-Dimethyl-butyl)-3-(4-{7-[3-(3,5-dimethyl-piperidin-1-yl)-propoxy]-6-methoxy-quinolin-4-yloxy}-phenyl)-urea |
| 55 | 1-(3,3-Dimethyl-cyclohexyl)-3-{3-fluoro-4-[6-methoxy-7-(2-piperidin-1-yl-ethoxy)-quinolin-4-yloxy]-phenyl}-urea |
| 56 | 1-{3-Fluoro-4-[6-methoxy-7-(2-piperidin-1-yl-ethoxy)-quinolin-4-yloxy]-phenyl}-3-(3,3,5-trimethyl-cyclohexyl)-urea |
| 57 | 1-{2-Fluoro-4-[6-methoxy-7-(2-piperidin-1-yl-ethoxy)-quinolin-4-yloxy]-phenyl}-3-(3,3,5-trimethyl-cyclohexyl)-urea |
| 58 | 1-(2-Cyclohexyl-ethyl)-3-{3-fluoro-4-[6-methoxy-7-(2-piperidin-1-yl-ethoxy)-quinolin-4-yloxy]-phenyl}-urea |
| 59 | 1-(2-Cyclopentyl-ethyl)-3-{3-fluoro-4-[6-methoxy-7-(2-piperidin-1-yl-ethoxy)-quinolin-4-yloxy]-phenyl}-urea |
| 60 | 1-(3,3-Dimethyl-butyl)-3-(4-{7-[2-(2,6-dimethyl-piperidin-1-yl)-ethoxy]-6-methoxy-quinolin-4-yloxy}-2-fluoro-phenyl)-urea |
| 62 | 1-(3,3-Dimethyl-butyl)-3-(4-{7-[2-(2,6-dimethyl-piperidin-1-yl)-ethoxy]-6-methoxy-quinolin-4-yloxy}-3-fluoro-phenyl)-urea |
| 63 | 1-{2-Fluoro-4-[6-methoxy-7-(2-piperidin-1-yl-ethoxy)-quinolin-4-yloxy]-phenyl}-3-(4-hydroxy-3,3-dimethyl-butyl)-urea |
| 64 | 1-(2-Cyclohexyl-ethyl)-3-{2-fluoro-4-[6-methoxy-7-(2-piperidin-1-yl-ethoxy)-quinolin-4-yloxy]-phenyl}-urea |
| 65 | 1-(2-Cyclopentyl-ethyl)-3-{2-fluoro-4-[6-methoxy-7-(2-piperidin-1-yl-ethoxy)-quinolin-4-yloxy]-phenyl}-urea |
| 66 | 1-(3,3-Dimethyl-butyl)-3-{4-[6-methoxy-7-(2-morpholin-4-yl-ethoxy)-quinolin-4-yloxy]-phenyl}-urea |
| 67 | 1-(3,3-Dimethyl-butyl)-3-{2-fluoro-4-[6-methoxy-7-(2-morpholin-4-yl-ethoxy)-quinolin-4-yloxy]-phenyl}-urea |
| 68 | 1-(3,3-Dimethyl-butyl)-3-{4-[7-methoxy-6-(2-morpholin-4-yl-ethoxy)-quinolin-4-yloxy]-phenyl}-urea |
| 69 | 1-(3,3-Dimethyl-cyclohexyl)-3-{2-fluoro-4-[6-methoxy-7-(2-morpholin-4-yl-ethoxy)-quinolin-4-yloxy]-phenyl}-urea |
| 70 | 1-(3-Chloro-4-{7-[2-(2,6-dimethyl-morpholin-4-yl)-ethoxy]-6-methoxy-quinolin-4-yloxy}-phenyl)-3-(3,3-dimethyl-butyl)-urea |
| 71 | 1-(2-Cycloheptyl-ethyl)-3-(4-{7-[2-(2,6-dimethyl-morpholin-4-yl)-ethoxy]-6-methoxy-quinolin-4-yloxy}-2-fluoro-phenyl)-urea |
| 72 | 1-(2-Cyclohexyl-ethyl)-3-{2-fluoro-4-[6-methoxy-7-(2-morpholin-4-yl-ethoxy)-quinolin-4-yloxy]-phenyl}-urea |
| 73 | 1-{2-Fluoro-4-[6-methoxy-7-(2-morpholin-4-yl-ethoxy)-quinolin-4-yloxy]-phenyl}-3-(3-methyl-butyl)-urea |
| 74 | 1-{4-[7-(2-Azepan-1-yl-ethoxy)-6-methoxy-quinolin-4-yloxy]-2-chloro-phenyl}-3-(3,3-dimethyl-butyl)-urea |
| 75 | 1-(3,3-Dimethyl-butyl)-3-(4-{6-methoxy-7-[3-(4-methyl-piperazin-1-yl)-propoxy]-quinolin-4-yloxy}-phenyl)-urea hydrochloride |
| 76 | 1-(3,3-Dimethyl-butyl)-3-(2-fluoro-4-{6-methoxy-7-[3-(4-methyl-piperazin-1-yl)-propoxy]-quinolin-4-yloxy}-phenyl)-urea |
| 77 | 1-(3,3-Dimethyl-butyl)-3-(3-chloro-4-{6-methoxy-7-[3-(4-methyl-piperazin-1-yl)-propoxy]-quinolin-4-yloxy}-phenyl)-urea |

For these compounds, chemical structures, starting compounds, synthesis methods, and data for specifying the compounds are shown below. Numerals described in the column of "Synthesis method" indicate that the compound was synthesized as described in the Synthesis Example for the compound number.

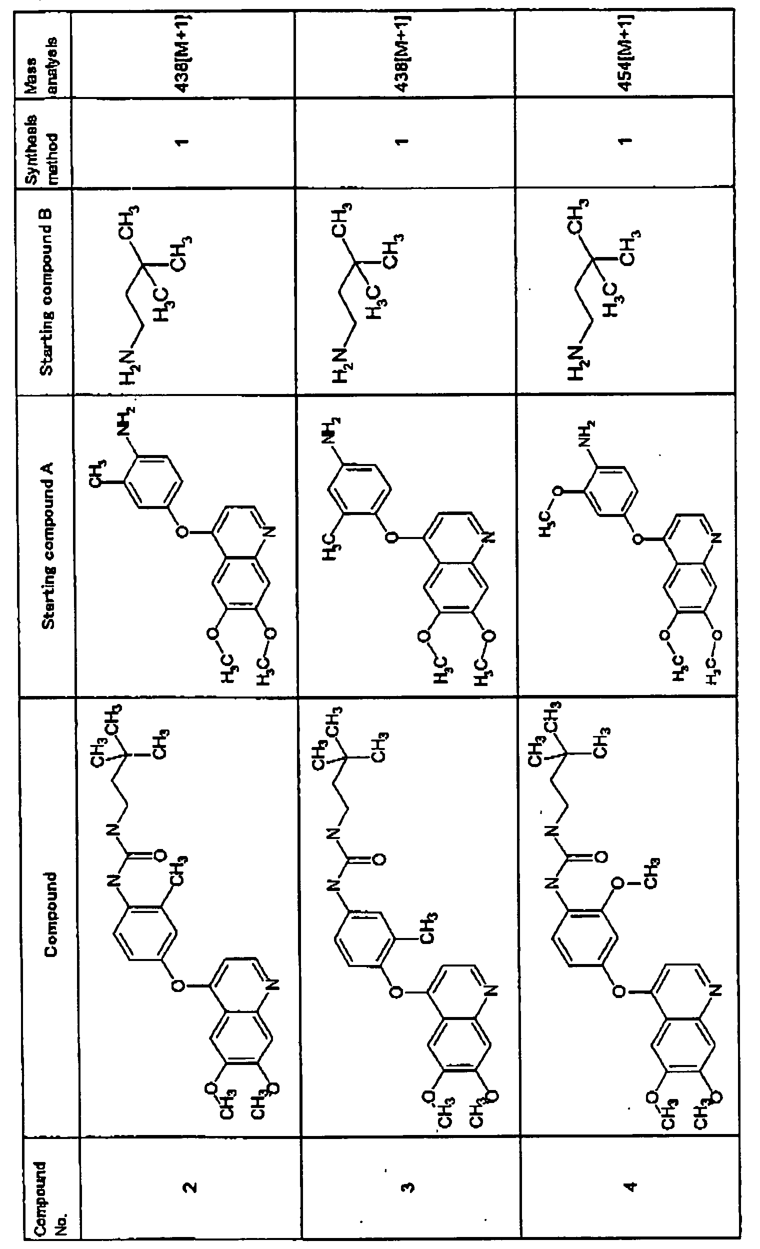

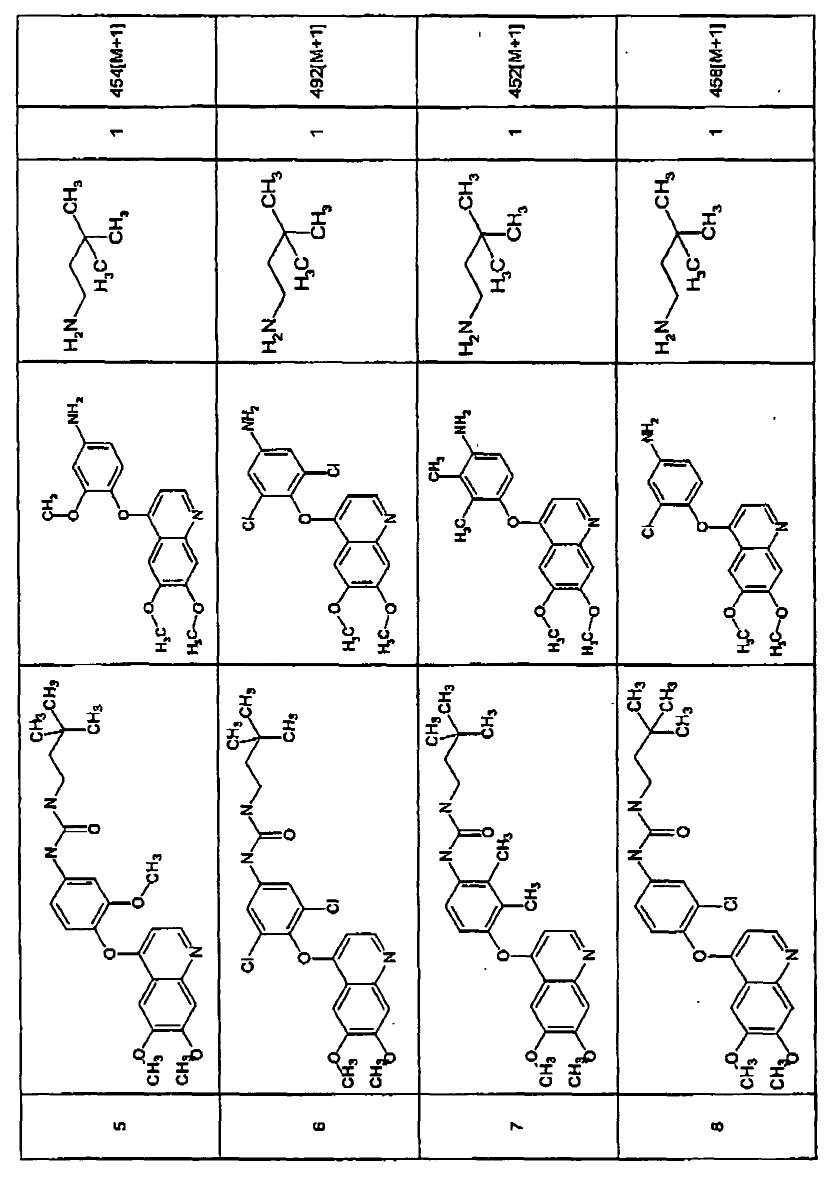

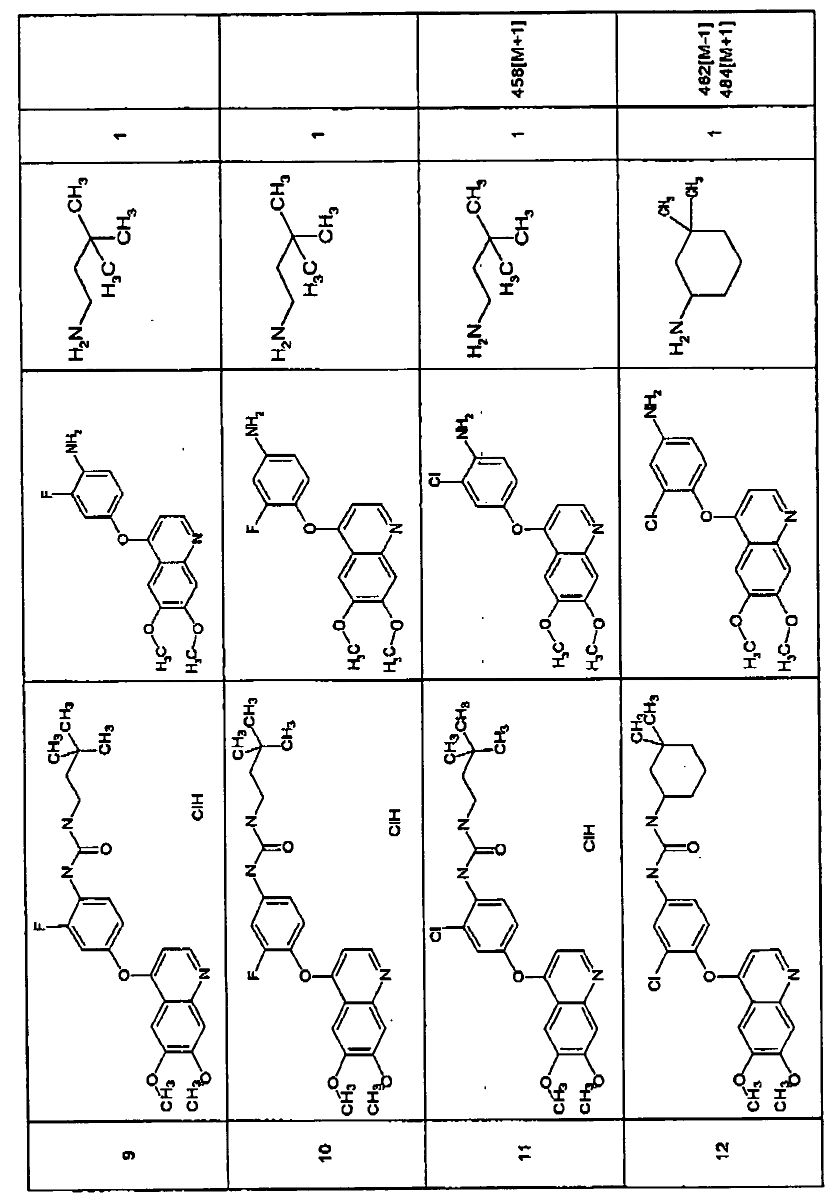

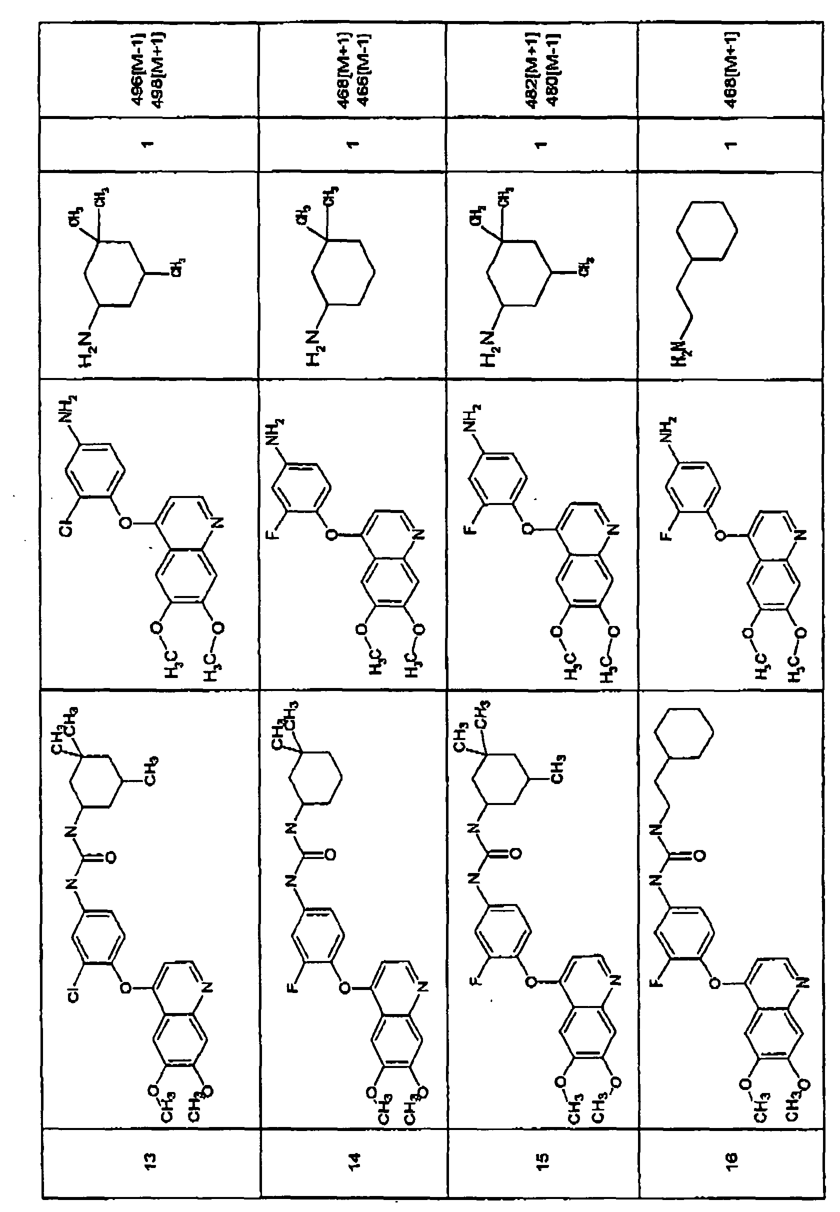

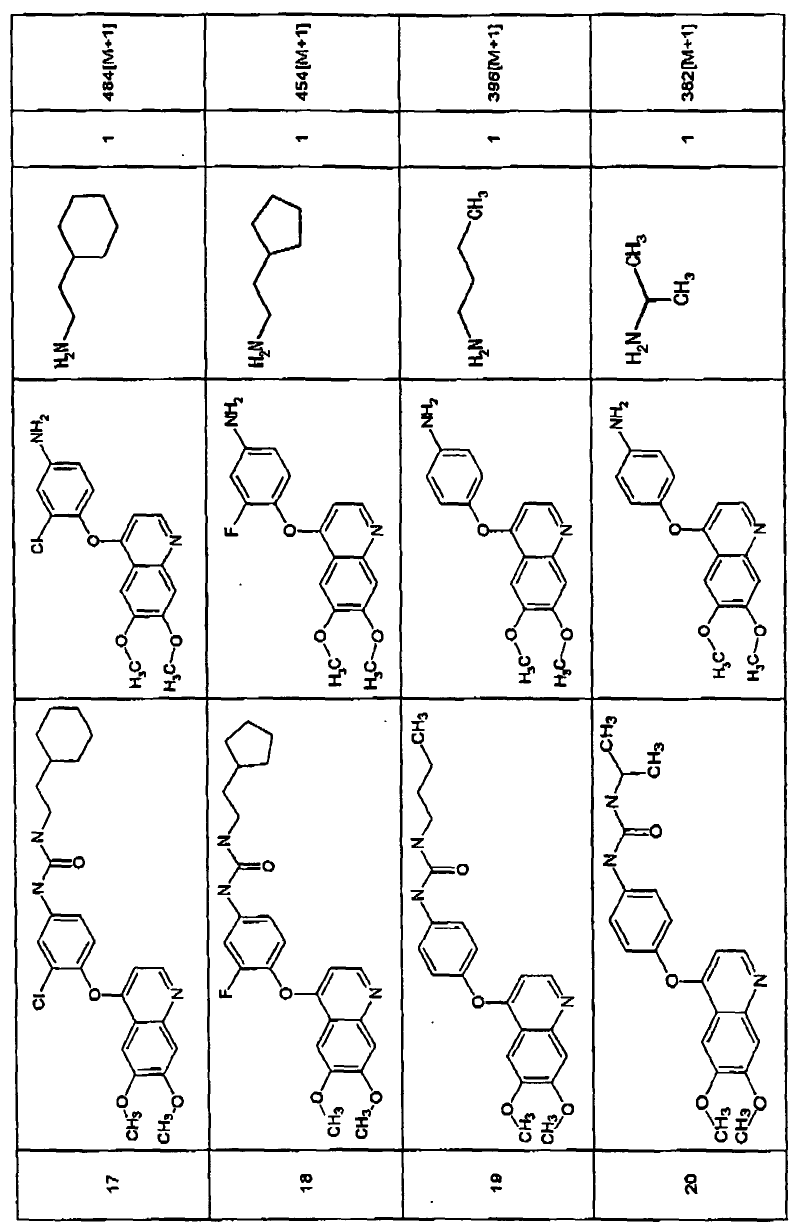

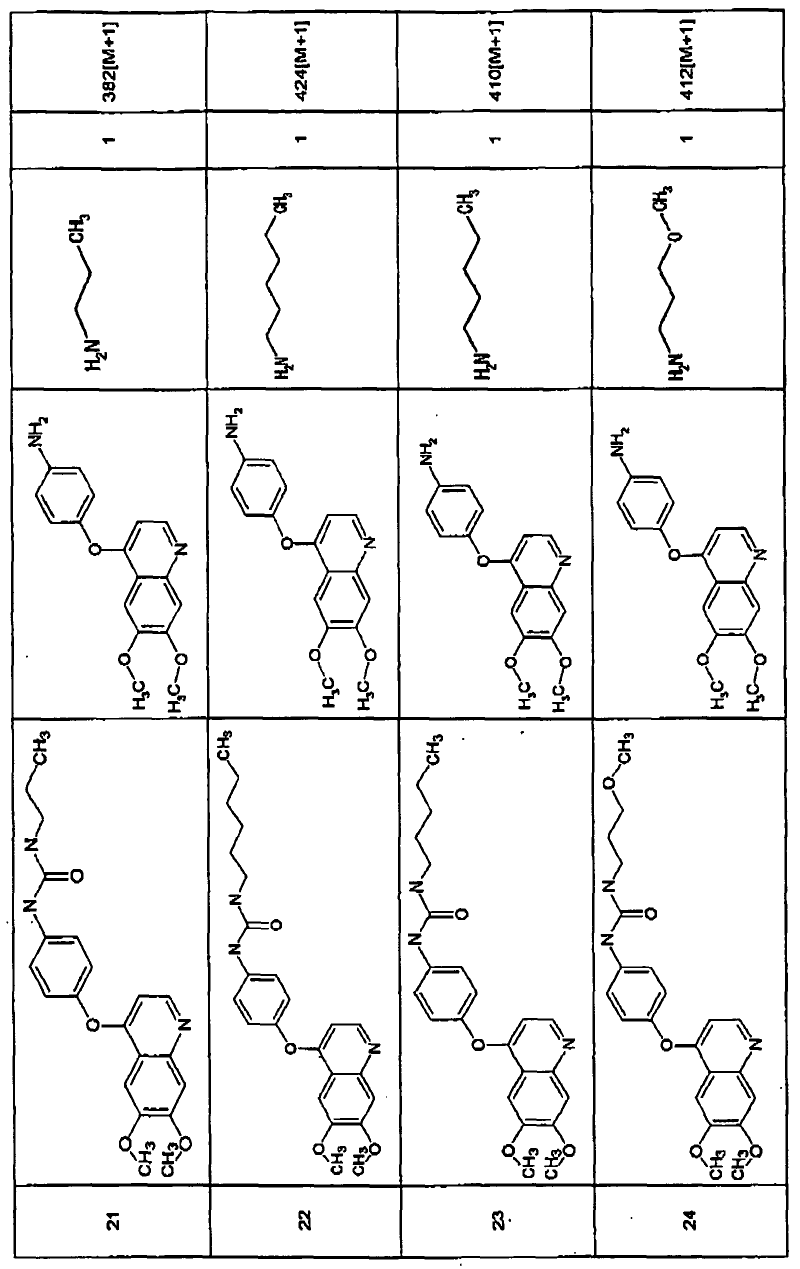

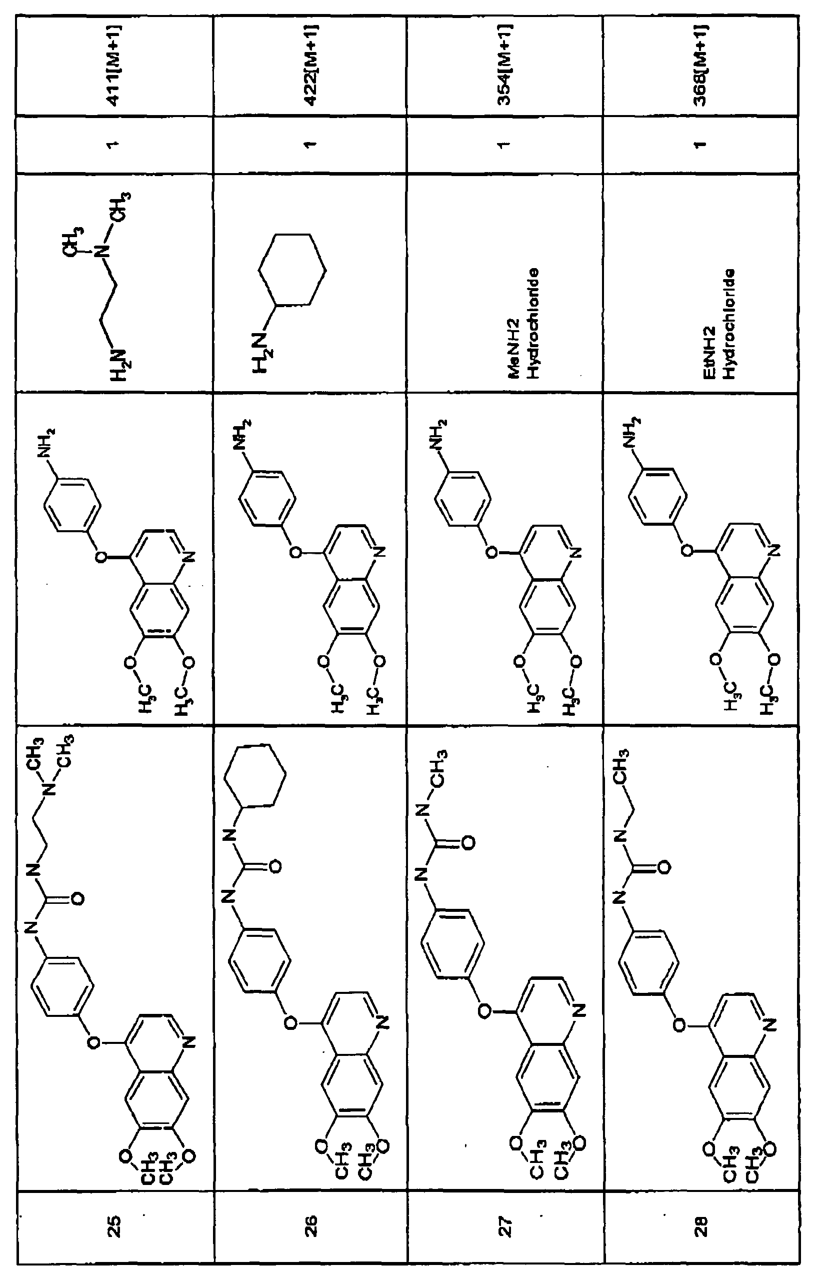

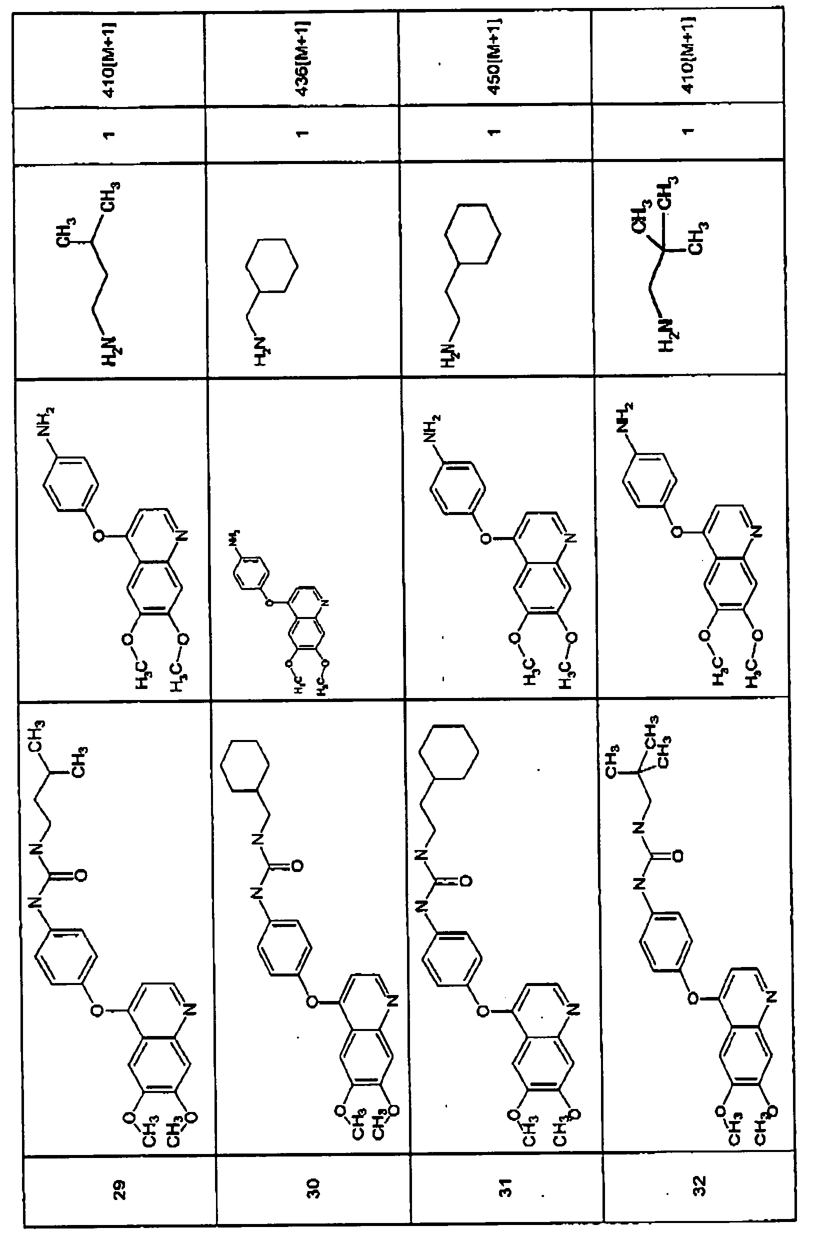

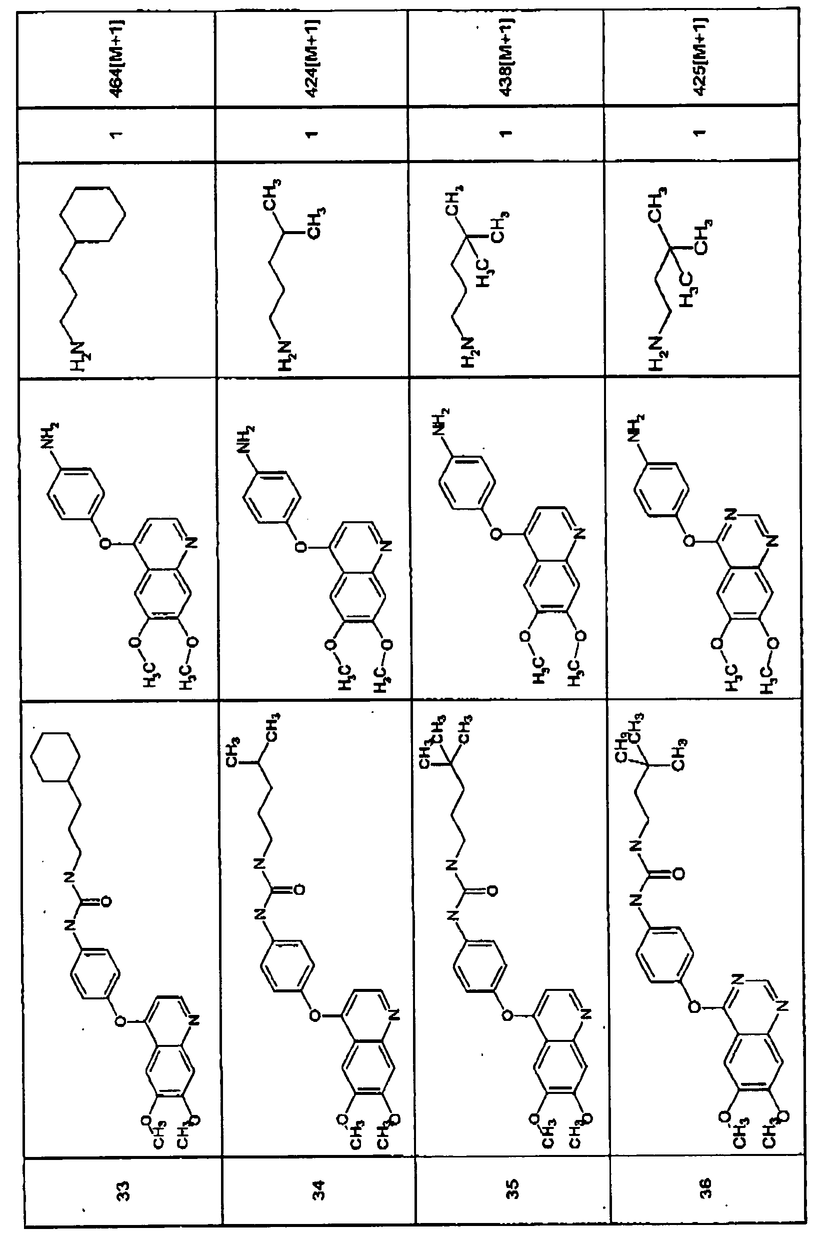

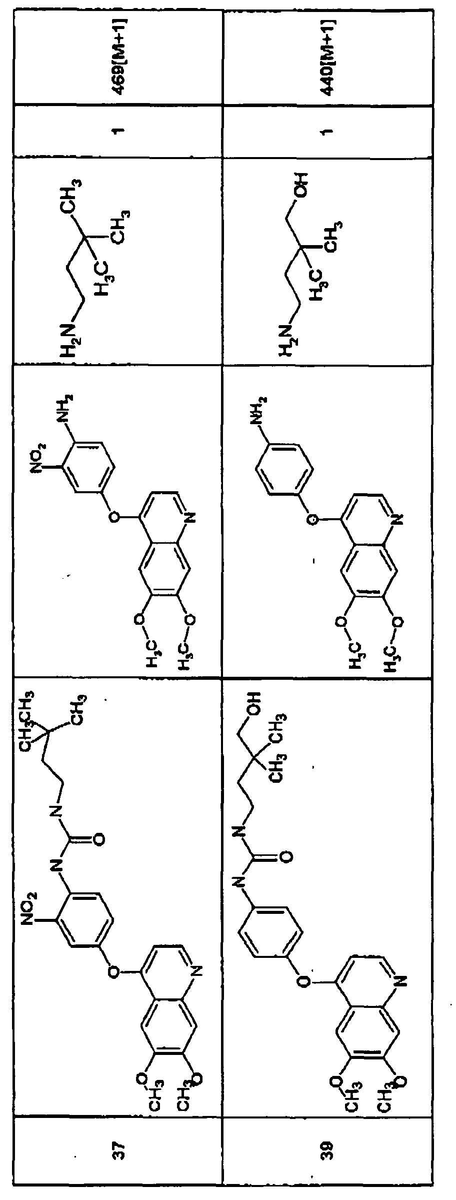

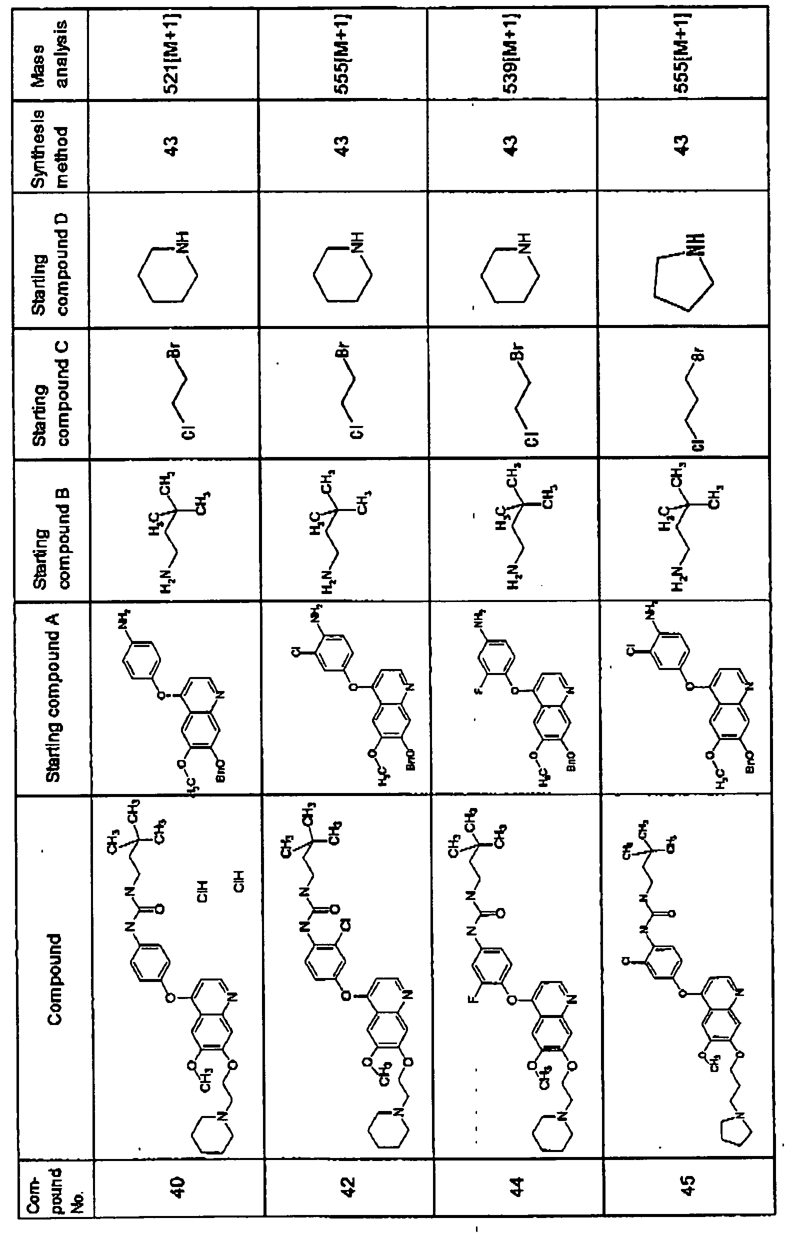

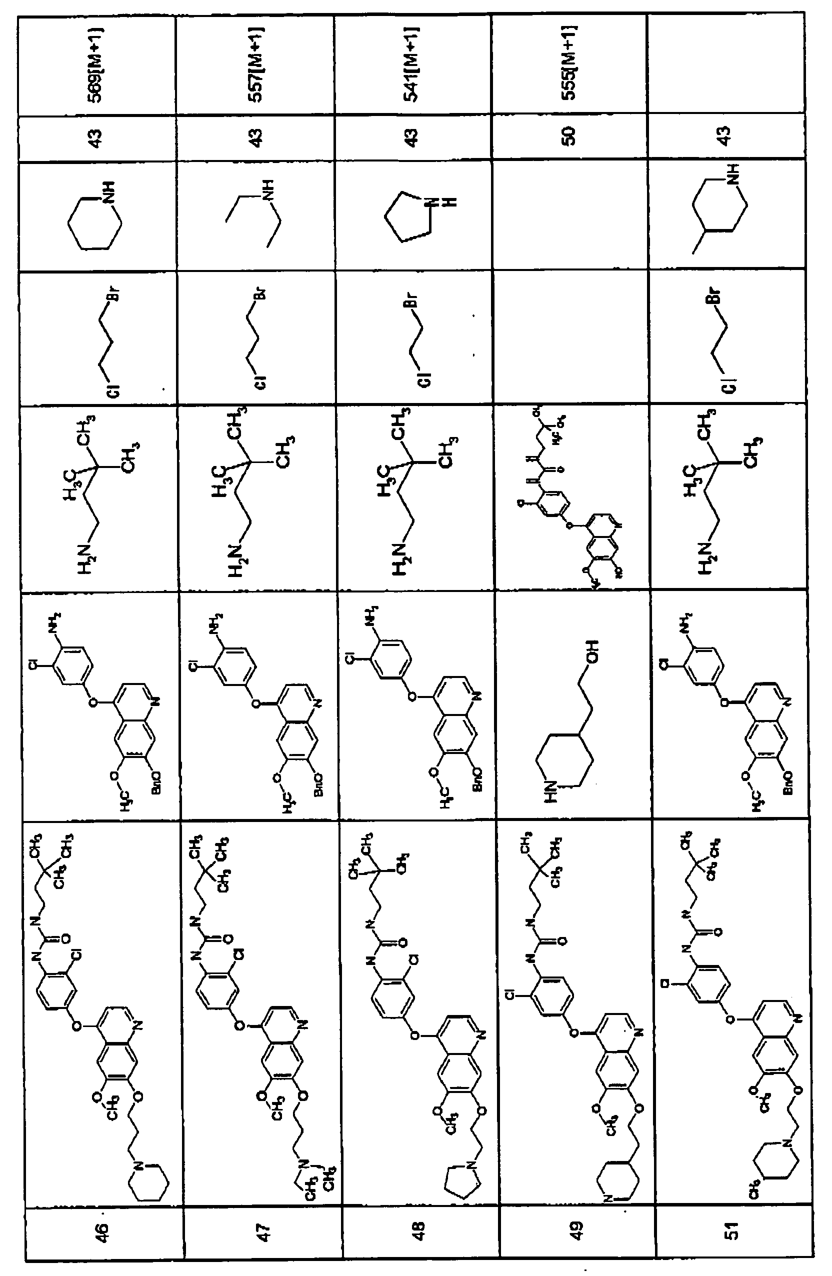

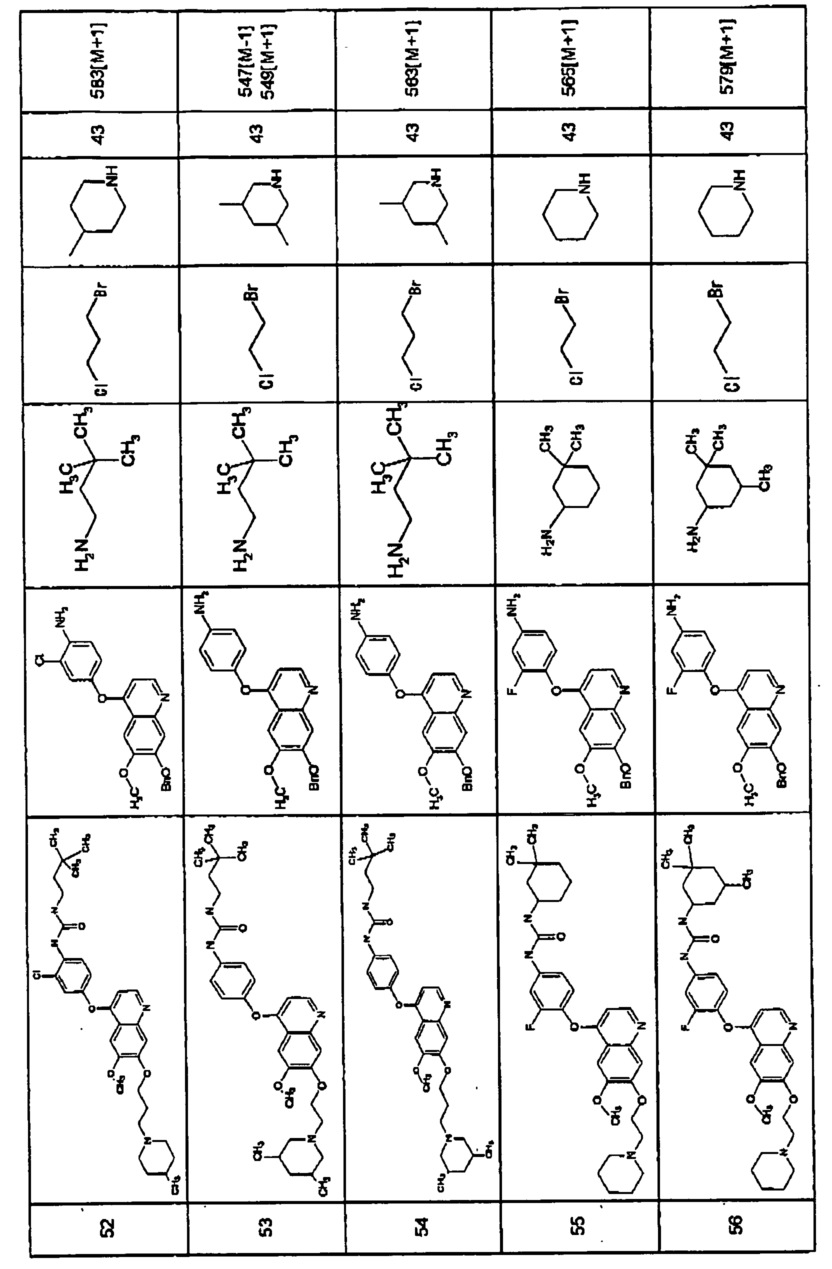

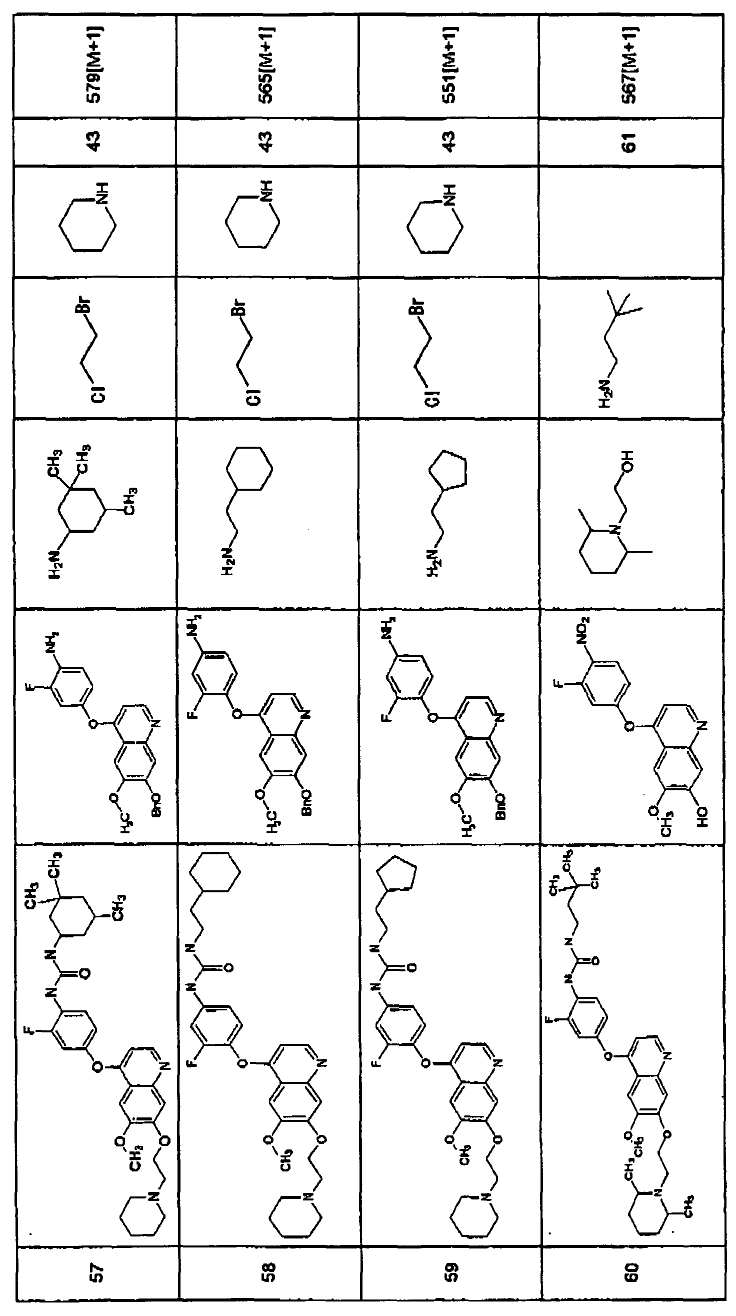

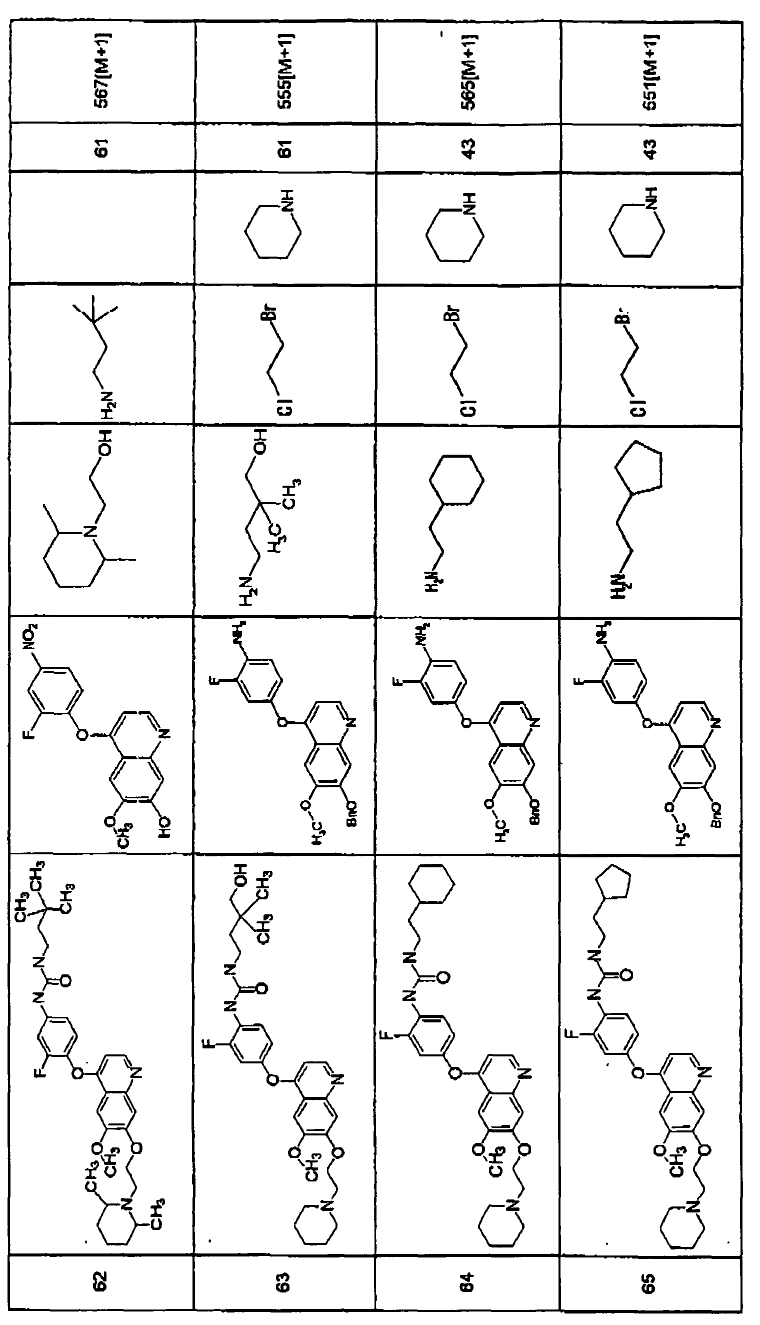

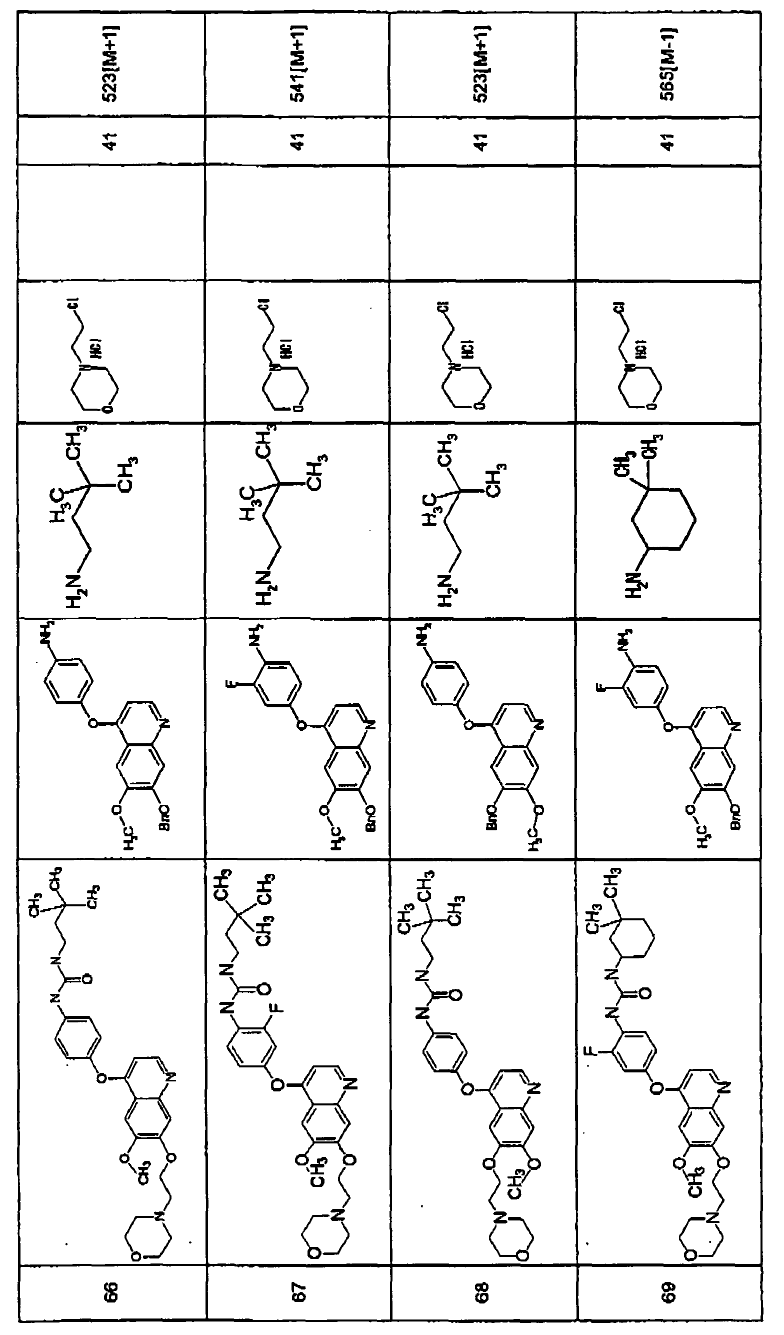

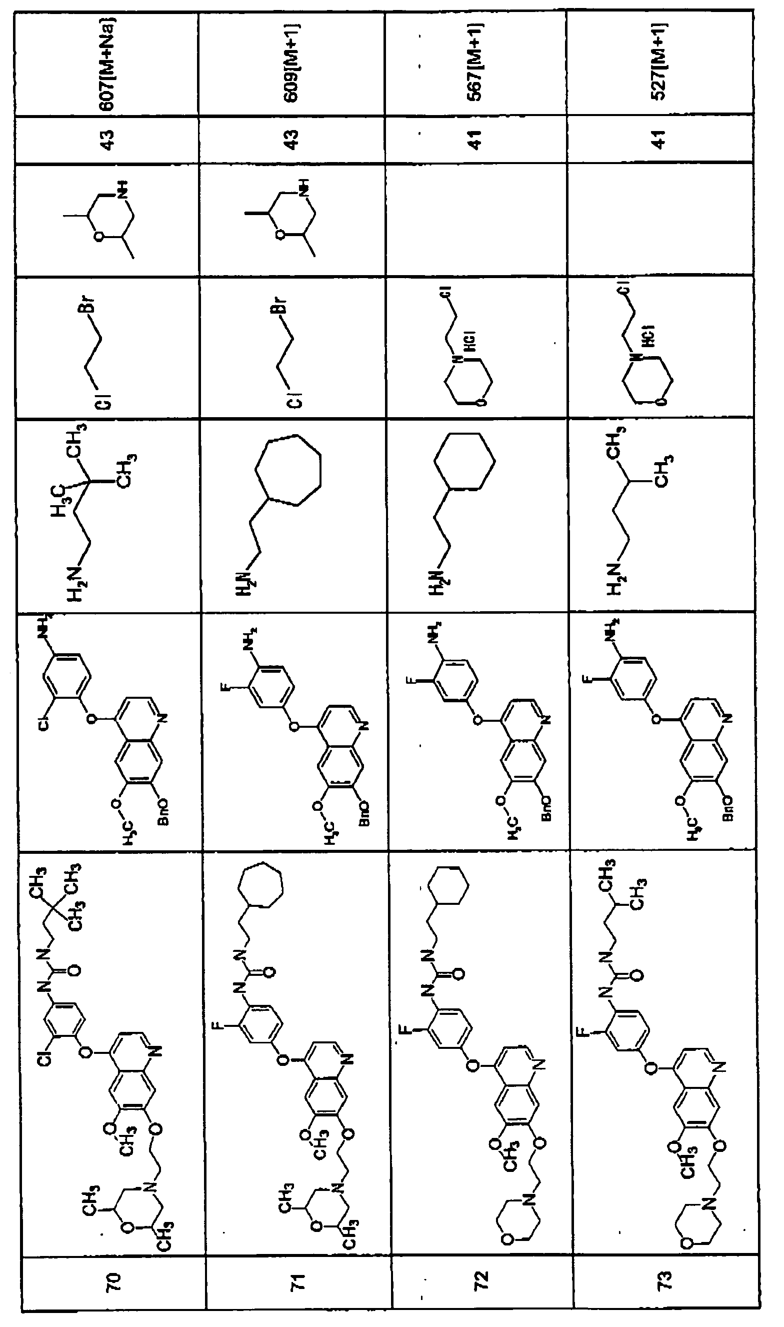

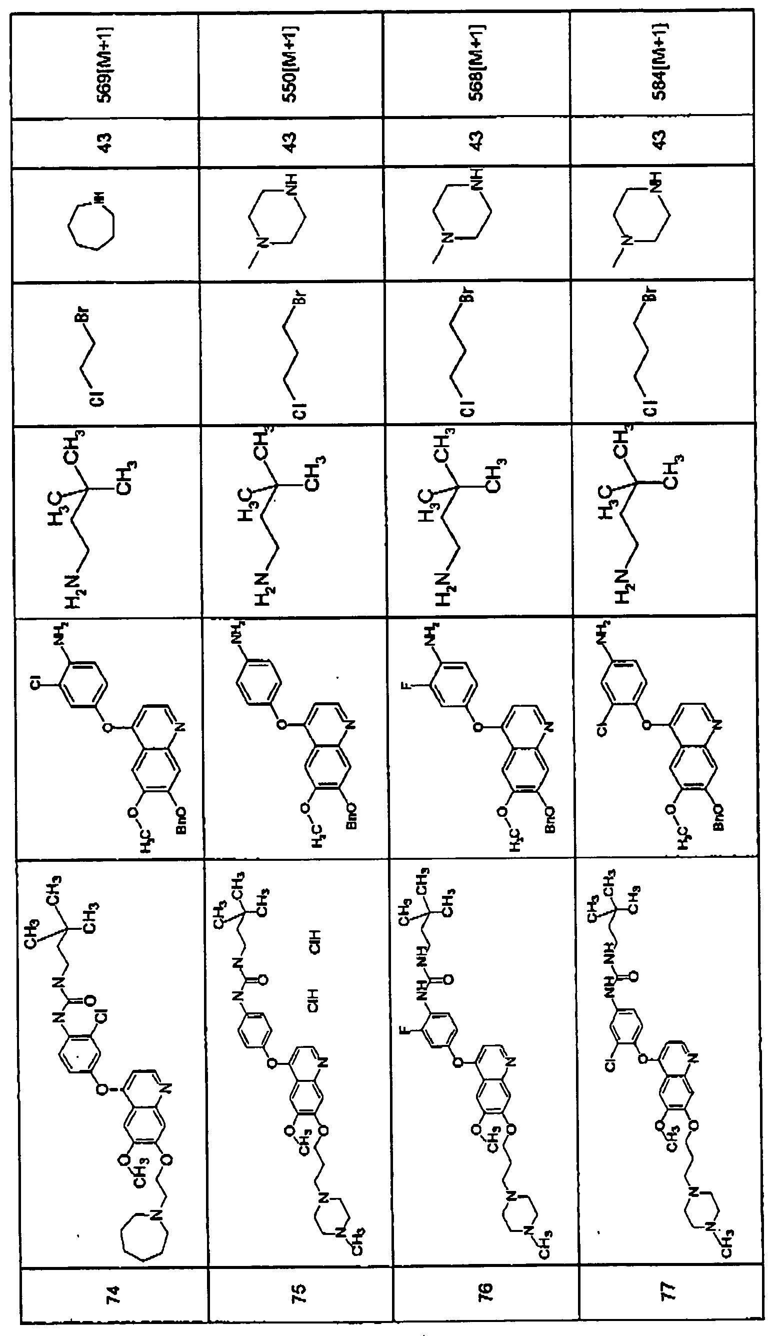

### Compound 9: 1-[4-(6,7-Dimethoxy-quinolin-4-yloxy)-2-fluoro-phenyl]-3-(3,3-dimethyl-butyl)-urea hydrochloride

¹H-NMR (CDCl₃, 400 MHz): 0.96 (s, 9H), 1.45 - 1.51 (m, 2H), 3.28 - 3.35 (m, 2H), 4.04 (s, 3H), 4.05 (s, 3H), 4.74 (t, J = 5.4 Hz, 1 H), 6.48 - 6.53 (m, 2H), 6.92 - 7.00 (m, 2H), 7.42 (s, 1 H), 7.51 (s, 1 H), 8.17 (t, J = 9.0 Hz, 1H), 8.50 (d, J = 5.4 Hz, 1 H)

### Compound 10: 1-[4-(6,7-Dimethoxy-quinolin-4-yloxy)-3-fluoro-phenyl]-3-(3,3-dimethyl-butyl)-urea hydrochloride

¹H-NMR (CDCl₃, 400 MHz): 0.92 (s, 9H), 1.41 - 1.45 (m, 2H), 3.26 - 3.32 (m, 2H), 4.02 (s, 3H), 4.04 (s, 3H), 5.36 (br, 1H), 6.39 (d, J = 5.4 Hz, 1 H), 7.07 - 7.13 (m, 2H), 7.40 (s, 1 H), 7.49 - 7.52 (m, 1 H), 7.58 (s, 1H), 7.86 (br, 1 H), 8.44 (d, J = 5.4 Hz, 1 H)

### Compound 11: 1-[2-Chloro-4-(6,7-dimethoxy-quinolin-4-yloxy)-phenyl]-3-(3,3-dimethyl-butyl)-urea hydrochloride

¹H-NMR (CDCl₃, 400 MHz): 0.95 (s, 9H), 1.45 - 1.50 (m, 2H), 3.27 - 3.35 (m, 2H), 4.04 (s, 3H), 4.04 (s, 3H), 5.61 (br, 1 H), 6.48 (d, J = 5.4 Hz, 1H), 7.10 (dd, J = 2.7, 9.0 Hz, 1 H), 7.17 (br, 1H), 7.18 (d, J = 2.7 Hz, 1 H), 7.43 (s, 1H), 7.51 (s, 1H), 8.29 (d, J = 9.0 Hz, 1H), 8.49 (d, J = 5.4 Hz, 1H)
Mass spectrometric value (ESI-MS, m/z): 458 (M⁺+1)

### Compound 12: 1-[3-Chloro-4-(6,7-dimethoxy-quinolin-4-yloxy)-phenyl]-3-(3,3-dimethyl-cyclohexyl)-urea

¹H NMR (CDCl₃, 400 MHz): δ 0.85 (s, 3H), 0.89 (s, 3H), 0.80 - 2.00 (m, 8H), 3.68 - 3.82 (m, 1H), 3.96 (s, 3H), 3.98 (s, 3H), 5.00 (d, J = 7.6 Hz, 1 H), 6.22 (d, J = 5.4 Hz, 1H), 7.05 (d, J = 8.8 Hz, 1 H), 7.23 (dd, J = 2.4, 8.8 Hz, 1H), 7.34 (s, 1H), 7.38 (s. 1H), 7.53 (s, 1H), 7.57 (d, J = 2.7 Hz, 1H), 8.37 (d, J = 5.4 Hz, 1H)
ESI-MS: m/z = 484 (M+1), 482 (M - 1)

### Compound 13: 1-[3-Chloro-4-(6,7-dimethoxy-quinolin-4-yloxy)-phenyl]-3-(3,3,5-trimethyl-cyclohexyl)-urea

¹H NMR (CDCl₃, 400 MHz): δ 0.89 (d, J = 6.6 Hz, 3H), 0.93 (s, 3H), 0.97 (s, 3H), 0.53 - 2.20 (m, 7H), 3.80 - 3.92 (m, 1 H), 4.04 (s, 3H), 4.05 (s, 3H), 4.86 (d, J = 8.1 Hz, 1H), 6.30 (d, J = 5.4 Hz, 1 H), 7.11 (s, 1 H), 7.13 (d, J = 8.8 Hz, 1H), 7.30 (dd, J = 2.4, 8.8 Hz, 1H), 7.41 (s, 1H), 7.60 (s, 1 H), 7.64 (d, J = 2.7 Hz, 1H), 8.45 (d, J = 5.4 Hz, 1H)
ESI-MS: m/z = 498 (M+1), 496 (M - 1)

### Compound 14: 1-[4-(6,7-Dimethoxy-quinolin-4-yloxy)-3-fluoro-phenyl]-3-(3,3-dimethyl-cyclohexyl)-urea

¹H NMR (CDCl₃, 400 MHz): δ 0.84 (s, 3H), 0.88 (s, 3H), 0.78 - 2.20 (m, 8H), 3.64 - 3.82 (m, 1 H), 3.96 (s, 3H), 3.97 (s, 3H), 5.10 (d, J = 7.8 Hz, 1H), 6.32 (d, J = 5.4 Hz, 1H), 6.98 - 7.08 (m, 2H), 7.33 (s, 1 H), 7.43 (dd, J = 2.1, 12.6 Hz, 1 H), 7.51 (s, 1 H), 7.61 (s, 1 H), 8.38 (d, J = 5.4 Hz, 1 H)
ESI-MS: m/z = 468 (M+1), 466 (M-1)

### Compound 15: 1-[4-(6,7-Dimethoxy-quinolin-4-yloxy)-3-fluoro-phenyl]-3-(3,3,5-trimethyl-cyclohexyl)-urea

¹H NMR (CDCl₃, 400 MHz): δ 0.85 (d, J = 6.4 Hz, 3H), 0.89 (s, 3H), 0.93 (s, 3H), 0.52 - 2.20 (m, 7H), 3.84 - 3.92 (m, 1H), 4.01 (s, 3H), 4.02 (s, 3H), 5.06 (d, J = 8.1 Hz, 1H), 6.37 (d, J = 5.4 Hz, 1 H), 7.03 - 7.10 (m, 2H), 7.38 (s, 1H), 7.48 (dd, J = 2.4, 12.7 Hz, 1H), 7.51 (s, 1H), 7.56 (s, 1 H), 8.42 (d, J = 5.4 Hz, 1 H)
ESI-MS: m/z = 482 (M+1), 480 (M-1)

### Compound 40: 1-{4-[6-Methoxy-7-(2-piperidin-1-yl-ethoxy)-quinolin-4-yloxy]-phenyl}-3-(3,3-dimethyl-butyl)-urea hydrochloride

¹H-NMR (CD₃OD, 400 MHz): 0.97 (s, 9H), 1.48 - 2.02 (m, 8H), 3.19 (m, 2H), 3.25 (m, 2H), 3.72 - 3.80 (m, 4H), 4.12 (s, 3H), 4.76 (m, 2H), 6.94 (d, J = 6.8 Hz, 1H), 7.24 (d, J = 9.0 Hz, 2H), 7.61 (d, J = 8.8 Hz, 2H), 7.64 (s, 1 H), 7.88 (s, 1 H), 8.70 (d, J = 6.6 Hz, 1 H)
Mass spectrometric value (ESI-MS, m/z): 521 (M⁺+1)

### Compound 42: 1-{2-Chloro-4-[6-methoxy-7-(2-piperidin-1-yl-ethoxy)-quinolin-4-yloxy]-phenyl}-3-(3,3-dimethyl-butyl)-urea

¹H-NMR (CDCl₃, 400 MHz): 0.97 (s, 9H), 1.42 - 1.54 (m, 4H), 1.58 - 1.68 (m, 4H), 2.57 (br, 4H), 2.93 (t, J = 6.3 Hz, 2H), 3.28 - 3.36 (m, 2H), 4.01 (s, 3H), 4.34 (t, J = 6.3 Hz, 2H), 4.74 (s, 1H), 6.47 (d, J = 5.4 Hz, 1 H), 6.70 (s, 1H), 7.10 (dd, J = 2.7, 9.0 Hz, 1 H), 7.21 (d, J = 2.7 Hz, 1H), 7.41 (s, 1 H), 7.49 (s, 1 H), 8.25 (d, J = 9.0 Hz, 1H), 8.49 (d, J = 5.1 Hz, 1 H)
Mass spectrometric value (ESI-MS, m/z): 555 (M⁺+1)

### Compound 44: 1-(3,3-Dimethyl-butyl)-3-{3-fluoro-4-[6-methoxy-7-(2-piperidin-1-yl-ethoxy)-quinolin-4-yloxy]-phenyl}-urea

¹H-NMR (CDCl₃, 400 MHz): δ 0.93 (s, 9H), 1.41 - 1.51 (m, 4H), 1.63 - 1.70 (m, 4H), 2.57 - 2.64 (m, 4H), 2.96 (t, J = 6.0 Hz, 2H), 3.25 - 3.32 (m, 2H), 4.00 (s, 3H), 4.34 (t, J = 6.0 Hz, 2H), 5.21 - 5.26 (m, 1 H), 6.36 (d, J = 5.4 Hz, 1 H), 7.04 - 7.07 (m, 2H), 7.40 (s, 1 H), 7.49 - 7.55 (m, 2H), 7.57 (s, 1 H), 8.43 (d, J = 5.4 Hz, 1 H)
Mass spectrometric value (ESI-MS, m/z): 539 (M+1)

### Compound 51: 1-(2-Chloro-4-{6-methoxy-7-[2-(4-methyl-piperidin-1-yl)-ethoxy]-quinolin-4-yloxy}-phenyl)-3-(3,3-dimethyl-butyl)-urea

¹H-NMR (CDCl₃, 400 MHz): 0.94 (d, J = 6.1 Hz, 3H), 0.96 (s, 9H), 1.23 - 1.26 (m, 2H), 1.47 - 1.51 (m, 2H), 1.64 - 1.67 (m, 2H), 2.12 - 2.18 (m, 2H), 2.95 (t, J = 6.1 Hz, 2H), 3.01 - 3.04 (m, 2H), 3.29 - 3.33 (m, 2H), 4.01 (s, 3H), 4.32 (t, J = 6.1 Hz, 2H), 5.09 (t, J = 5.4 Hz, 1H), 6.47 (d, J = 5.4 Hz, 1 H), 6.89 (s, 1 H), 7.10 (dd, J = 2.7, 9.0 Hz, 1 H), 7.19 (d, J = 2.7 Hz, 1 H), 7.41 (s, 1H), 7.50 (s, 1 H), 8.27 (d, J = 9.0 Hz, 1H), 8.49 (d, J = 5.4 Hz, 1H)

### Compound 53: 1-(3,3-Dimethyl-butyl)-3-(4-{7-[2-(3,5-dimethyl-piperidin-1-yl)-ethoxy]-6-methoxy-quinolin-4-yloxy}-phenyl)-urea

¹H-NMR (CDCl₃, 400 MHz): δ 0.90 (d, J = 6.1 Hz, 6H), 0.95 (s, 9H), 0.94 - 1.10 (m, 2H), 1.42 - 1.48 (m, 2H), 1.74 - 1.95 (m, 3H), 3.04 - 3.16 (m, 3H), 3.26 - 3.33 (m, 2H), 4.00 (s, 3H), 4.39 - 4.45 (m, 2H), 4.93 (br, 1 H), 6.39 (d, J = 5.1 Hz, 1 H), 6.91 (br, 1H), 7.05 (d, J = 9.0 Hz, 2H), 7.39 - 7.44 (m, 3H), 7.54 (s, 1 H), 8.43 (d, J = 5.4 Hz, 1 H)
Mass spectrometric value (ESI-MS, m/z): 549 (M+1), 547 (M-1)

### Compound 60: 1-(3,3-Dimethyl-butyl)-3-(4-{7-[2-(2,6-dimethyl-piperidin-1-yl)-ethoxy]-6-methoxy-quinolin-4-yloxy}-2-fluoro-phenyl)-urea

¹H-NMR (CDCl₃, 400 MHz): 0.92 (s, 9H), 1.18 (d, J = 6.3 Hz, 6H), 1.19 - 1.75 (m, 8H), 2.55 - 2.61 (m, 2H), 3.17 - 3.31 (m, 4H), 3.98 (s, 3H), 4.16 - 4.19 (m, 2H), 5.07 - 5.09 (m, 1 H), 6.44 (d, J = 5.3 Hz, 1H), 6.82 - 6.95 (m, 3H), 7.39 (s, 1H), 7.46 (s, 1 H), 8.18 (t, J = 9.0 Hz, 1H), 8.46 (d, J = 5.3 Hz, 1H)
Mass spectrometric value (ESI-MS, m/z): 567 (M⁺+1)

### Compound 62: 1-(3,3-Dimethyl-butyl)-3-(4-{7-[2-(2,6-dimethyl-piperidin-1-yl)-ethoxy]-6-methoxy-quinolin-4-yloxy}-3-fluoro-phenyl)-urea

¹H-NMR (CDCl₃, 400 MHz): 0.90 (s, 9H), 1.17 (d, J = 6.3 Hz, 6H), 1.27 - 1.67 (m, 8H), 2.54 - 2.61 (m, 2H), 3.16 - 3.23 (m, 2H), 3.24 - 3.29 (m, 2H), 3.99 (s, 3H), 4.02 - 4.18 (m, 2H), 5.15 - 5.18 (m, 1 H), 6.36 (d, J. = 5.4 Hz, 1H), 7.03 - 7.09 (m, 2H), 7.37 (s, 1H), 7.54 (s, 1 H), 7.46 - 7.50 (m, 1H), 7.64 (brs, 1H), 8.42 (d, J = 5.4 Hz, 1H)
Mass spectrometric value (ESI-MS, m/z): 567 (M⁺+1)

### Compound 66: 1-(3,3-Dimethyl-butyl)-3-{4-[6-methoxy-7-(2-morpholin-4-yl-ethoxy)-quinolin-4-yloxy]-phenyl}-urea

¹H-NMR (CD₃OD, 400 MHz): 0.85 (s, 9H), 1.32 - 1.38 (m, 2H), 2.54 - 2.57 (m, 4H), 2.85 - 2.88 (m, 2H), 3.17 - 3.23 (m, 2H), 3.64 - 3.69 (m, 4H), 3.93 (s, 3H), 4.23 - 4.26 (m, 2H), 5.36 - 5.38 (m, 1H), 6.34 (d, J = 5.2 Hz, 1H), 6.99 (d, J = 8.8 Hz, 2H), 7.39 (s, 1H), 7.40 (d, J = 5.2 Hz, 1H), 7.47 (s, 1H), 7.71 (brs, 1H), 8.36 (d, J = 5.2 Hz, 1H)
Mass spectrometric value (ESI-MS, m/z): 523 (M⁺+1)

### Compound 68: 1-(3,3-Dimethyl-buyl)-3-{4-[7-methoxy-6-(2-morpholin-4-yl-ethoxy)-quinolin-4-yloxy]-phenyl}-urea

¹H-NMR (CDCl₃, 400 MHz): δ 0.94 (s, 9H), 1.43 - 1.48 (m, 2H), 2.63 - 2.68 (m, 4H), 2.96 (t, J = 5.8 Hz, 2H), 3.26 - 3.33 (m, 2H), 3.73 - 3.77 (m, 4H), 4.02 (s, 3H), 4.33 (t, J = 6.0 Hz, 2H), 4.91 - 4.96 (m, 1H), 6.44 (d, J = 5.4 Hz, 1H), 6.96 (br, 1H), 7.08 (d, J = 9.0 Hz, 2H), 7.42 - 7.47 (m, 3H), 7.59 (s, 1H), 8.42 (d, J = 5.6 Hz, 1H)
Mass spectrometric value (ESI-MS, m/z): 523 (M+1)

### Compound 69: 1-(3,3-Dimethyl-cyclohexyl)-3-{2-fluoro-4-[6-methoxy-7-(2-morpholin-4-yl-ethoxy)-quinolin-4-yloxy]-phenyl}-urea

¹H-NMR (CDCl₃, 400 MHz): δ 0.94 (s, 3H), 0.98 (s, 3H), 0.95 - 1.12 (m, 2H), 1.33 - 1.40 (m, 1H), 1.50 - 1.65 (m, 2H), 1.71 - 1.77 (m, 1H), 2.03 - 2.10 (m, 1H), 2.61 - 2.66 (m, 4H), 2.95 (t, J = 5.9 Hz, 2H), 3.70 - 3.88 (m, 6H), 4.00 (s, 3H), 4.33 (t, J = 5.9 Hz, 2H), 4.94 (d, J = 7.8 Hz, 1H), 6.48 (d, J = 5.1 Hz, 1H), 6.79 (d, J = 2.6 Hz, 1H), 6.91 (dd, J = 2.6, 11.5 Hz, 1H), 6.96 (d, J = 9.0 Hz, 1H), 7.41 (s, 1H), 7.50 (s, 1H), 8.20 (t, J = 9.0 Hz, 1H), 8.48 (d, J = 5.1 Hz, 1H)
Mass spectrometric value (ESI-MS, m/z): 565 (M-1)

### Compound 70: 1-(3-Chloro-4-{7-[2-(2,6-dimethyl-morpholin-4-yl)-ethoxy]-6-methoxy-quinolin-4-yloxy}-phenyl)-3-(3,3-dimethyl-butyl)-urea

¹H-NMR (CDCl₃, 400 MHz): 0.95 (s, 9H), 1.17 (s, 3H), 1.18 (s, 3H), 1.42 - 1.50 (m, 2H), 1.90 - 1.98 (m, 2H), 2.85 - 2.95 (m, 4H), 3.26 - 3.35 (m, 2H), 3.67 - 3.77 (m, 2H), 4.03 (s, 3H), 4.33 (t, J = 5.9 Hz, 2H), 4.82 (br, 1H), 6.30 (d, J = 5.4 Hz, 1H), 6.88 (br, 1H), 7.14 (d, J = 8.8 Hz, 1H), 7.31 (dd, J = 2.7, 8.8 Hz, 1H), 7.42 (s, 1 H), 7.59 (s, 1H), 7.64 (d, J = 2.7 Hz, 1H), 8.45 (d, J = 5.1 Hz, 1H)
Mass spectrometric value (ESI-MS, m/z): 607 (M+Na)⁺

### Compound 76: 1-(3,3-Dimethyl-butyl)-3-(2-fluoro-4-{6-methoxy-7-[3-(4-methyl-piperazin-1-yl)-propoxy]-quinolin-4-yloxy}-phenyl)-urea

¹H-NMR (CDCl₃, 400 MHz): 0.96 (s, 9H), 1.45 - 1.52 (m, 2H), 1.84 (br, 4H), 2.01 - 2.18 (m, 2H), 2.35 (s, 3H), 2.48 - 2.70 (m, 6H), 3.27 - 3.36 (m, 2H), 4.01 (s, 3H), 4.25 (t, J = 6.6 Hz, 2H), 4.80 - 4.86 (m, 1H), 6.48 (d, J = 5.4 Hz, 1H), 6.56 - 6.60 (m, 1H), 6.90 - 7.00 (m, 2H), 7.42 (s, 1H), 7.49 (s, 1H), 8.17 (t, J = 9.0 Hz, 1H), 8.48 (d, J = 5.4 Hz, 1H)
Mass spectrometric value (ESI-MS, m/z): 568 (M+1)⁺

### Compound 77: 1-(3,3-Dimethyl-butyl)-3-(3-chloro-4-{6-methoxy-7-[3-(4-methyl-piperazin-1-yl)-propoxy]-quinolin-4-yloxy]-phenyl)-urea

¹H-NMR (CDCl₃, 400 MHz): 0.96 (s, 9H), 1.43 - 1.52 (m, 2H), 1.78 (br, 4H), 2.08 - 2.18 (m, 2H), 2.38 (s, 3H), 2.52 - 2.72 (m, 6H), 3.26 - 3.35 (m, 2H), 4.02 (s, 3H), 4.23 - 4.29 (m, 2H), 4.77 - 4.85 (m, 1H), 6.30 (d, J = 5.1 Hz, 1H), 6.81 (br, 1H), 7.15 (d, J = 8.8 Hz, 1H), 7.32 - 7.37 (m, 1H), 7.42 (s, 1H), 7.57 (s, 1H), 7.66 (d, J = 2.4 Hz, 1H), 8.44 (d, J = 5.4 Hz, 1H)
Mass spectrometric value (ESI-MS, m/z): 584 (M)⁺

### Pharmacological Test Example 1: Measurement of inhibitory activity against FLT3 autophosphorylation using ELISA method

Human leukemia cells MV4-11 (ATCC number: CRL-9591) were cultured in an IMDM medium containing 10% fetal calf serum (purchased from SIGMA) within a 5% carbon dioxide incubator until 50 to 90% confluent. The harvested cells were seeded into 96-wells, plates, containing an IMDM medium containing 0.1% fetal calf serum, in a 96-well multiscreen plate in an amount of 5.0 × 10⁵ per well, a solution of the test compound in dimethyl sulfoxide was added to each well, and the cultivation was continued at 37°C for one hr. The medium was removed, followed by washing with phosphate buffered saline. A lysis buffer (60 µl) (20 mM HEPES (pH 7.4), 150 mM NaCl, 0.2% Triton X-100, 10% glycerol, 5 mM sodium orthovanadylate, 5 mM disodium ethylenediaminetetraacetate, and 2 mM Na₄P₂O₇) was then added thereto. The mixture was shaken at 4°C for 2 hr to prepare a cell extracts.

Separately, phosphate buffered saline (50 µl, pH 7.4) containing 5 µg/ml of anti-phospho-tyrosine antibody (PY20; purchased from Transduction Laboratories) was added to a 96-well microplate for ELISA (Maxisorp; purchased from NUNC), followed by standing at 4°C overnight to form a solid phase on the wells. After washing of the plate, 250 µl of a blocking solution was added, followed by standing at room temperature for 3 hr to perform blocking. After washing, the whole quantity of the cell extract was transferred to the wells, and the plate was then allowed to stand at 4°C overnight. After washing, an anti-FLT3 antibody (Flt3/Flk2 (C-20), purchased from Santa Cruz Biotechnology) was allowed to react at room temperature for 2 hr, and, after washing, a peroxidase-labeled anti-rabbit Ig antibody (purchased from Amersham) was allowed to react at room temperature for one hr. After washing, a chromophoric substrate for peroxidase (purchased from Sumitomo Bakelite Co., Ltd.) was added thereto to initiate a reaction. After a suitable level of color development, a reaction termination solution was added to stop the reaction, and the absorbance at 450 nm was measured with a microplate reader. The FLT3-phosphorylation activity for each well was determined by presuming the absorbance without the addition of the medicament to be 100% FLT3-phosphorylation activity and the absorbance with the addition of a large excess of a positive control (Compound 1, 10 µM) to be 0% FLT3-phosphorylation activity. The concentration of the test compound was varied on several levels, the FLT3-phosphorylation inhibitory activity was determined for each case, and the concentration of the test compound necessary for inhibiting 50% of FLT3-phosphorylation (IC₅₀) was calculated. The results were as shown in Table 1.

**Table 1**

| Compound No. | IC50 (nM) |
|---|---|
| 1 | 2 |
| 3 | 7 |
| 6 | 109 |
| 8 | < 1 |
| 10 | < 1 |
| 40 | 93 |
| 41 | 308 |
| 43 | 216 |
| 44 | 200 |

### Pharmacological Test Example 2: Cell growth inhibitory test (MV4-11)

MV4-11 (ATCC number: CRL-9591) as human leukemia cells was seeded in an amount of 3 × 10³ using a DMEM medium containing 10% fetal calf serum (purchased from GIBCO) in a 96-well flat bottom plate. A solution of a test compound in dimethyl sulfoxide was added to each well and was cultured in a 5% carbon dioxide incubator for 3 days. Three days after the initiation of the culture, 10 µl of a WST-1 solution in Cell Counting Kit (purchased from Wako Pure Chemical Industries, Ltd.) was added to each well for a color reaction. After proper color development, the absorbance was measured with a microplate reader under conditions of measurement wavelength 450 nm and reference wavelength 650 nm. The growth ratio for each well was determined by presuming the absorbance without the addition of the medicament to be 100% growth ratio and the absorbance of the well without seeding of the cells to be 0% growth ratio. The concentration of the test compound was varied on several levels, the cell growth ratio for each case was determined, and the concentration of the test compound necessary for inhibiting 50% of cell growth ratio (IC₅₀) was calculated. The results were as shown in Table 2.

**Table 2**

| Compound No. | MV4-11 |
|---|---|
| 1 | < 1 |
| 2 | 395.0 |
| 3 | 2.0 |
| 4 | 889.0 |
| 5 | 23.0 |
| 6 | 8.0 |
| 7 | 558.0 |
| 8 | 1.0 |
| 9 | 8.0 |
| 10 | < 1 |
| 11 | 300.0 |
| 12 | 5.0 |
| 13 | 18.0 |
| 14 | 2.0 |
| 15 | 11.0 |
| 16 | 4.0 |
| 17 | 17.0 |
| 18 | 1.0 |
| 19 | 6.0 |
| 20 | 31.0 |
| 21 | 31.0 |
| 22 | 4.0 |
| 23 | 2.0 |
| 24 | 18.0 |
| 25 | 66.0 |
| 26 | 12.0 |
| 27 | 32.0 |
| 28 | 19.0 |
| 29 | 1.0 |
| 30 | 4.0 |
| 31 | 2.0 |
| 32 | 201.0 |
| 33 | 8.0 |
| 34 | < 1 |
| 35 | < 1 |
| 36 | < 1 |
| 37 | 47.0 |
| 39 | < 1 |
| 40 | 1.0 |
| 41 | 25.0 |
| 42 | 235.0 |
| 43 | 12.0 |
| 44 | 2.0 |
| 45 | 118.0 |
| 46 | 145.0 |
| 47 | 51.0 |
| 48 | 217.0 |
| 49 | 207.0 |
| 50 | 224.0 |
| 51 | 211.0 |
| 52 | 184.0 |
| 53 | 4.0 |
| 54 | 3.0 |
| 55 | 6.0 |
| 56 | 14.0 |
| 57 | 19.0 |
| 58 | 4.0 |
| 59 | 2.0 |
| 60 | 33.0 |
| 61 | 57.0 |
| 62 | 4.0 |
| 63 | 10.0 |
| 64 | 13.0 |
| 65 | 4.0 |
| 66 | < 1 |
| 67 | 2.0 |
| 68 | < 1 |
| 69 | 2.0 |
| 70 | 2.0 |
| 71 | 15.0 |
| 72 | 3.0 |
| 73 | 6.0 |
| 74 | 255.0 |
| 75 | < 1 |

### Pharmacological Test Example 3: Measurement of cell growth inhibitory activity using MTT method

Human leukemia cell line MOLM13 (subdivided from Hayashibara Biochemical Laboratories, Inc.) was cultured in an RPMI medium containing 10% fetal calf serum (purchased from Asahi Techno Glass Corporation) within a 5% carbon dioxide incubator until the cell density reached 50 to 90% of the maximum cell density. These cells were seeded into wells, containing an RPMI medium containing 10% fetal calf serum, in a 96-well flat bottom plate in an amount of 2 × 10⁴ per well. The amount of the solution of the medium in each well was brought to 100 µl, and a test compound was added thereto to 500 ng, 100 ng, 20 ng, and 4 ng per ml. After culture within an incubator for 72 hr, 10 µl of a 5 mg/ml MTT (3-(4,5-dimethylthiazol-2-yl)-2,5-diphenyl tetrazolium bromide) solution (purchased from SIGMA) was added to the medium, and a reaction was allowed to proceed for 4 hr. Thereafter, 100 µl of a 0.04 N hydrochloric acid-isopropanol solution was added to completely dissolve the resultant MTT formazan crystal. After the dissolution, absorbance at 550 nm was measured using 620 nm as a reference wavelength with a microplate reader (DIGISCAN) (It is regarded that, in the MTT method, the viable cell count is reflected, and linearity is observed in a cell count per well range of 5 × 10⁵ to 2 × 10² cells). After 72 hr, the absorbance of only the medium free from cells was subtracted from the absorbance of the well without the addition of the test compound to determine a value which was presumed to be 100%. On the other hand, the absorbance with the addition of the compound was expressed in terms of % growth. The concentration of the test compound necessary for inhibiting 50% of the cell growth ratio (IC₅₀) was calculated from the average of values obtained from three wells, The results are shown in Table 3.

**Table 3**

| Compound No. | IC₅₀(nM) |
|---|---|
| 1 | 24.4 |
| 5 | 93.1 |
| 6 | 12.4 |
| 8 | 13.3 |
| 10 | 20.8 |
| 12 | 5.37 |
| 13 | 3.87 |
| 14 | 5.13 |
| 15 | 9.5 |
| 16 | 18.8 |
| 17 | 20.5 |
| 18 | 40.3 |
| 26 | 31.3 |
| 41 | 34.8 |
| 43 | 32.5 |
| 44 | 19.3 |
| 45 | 255 |
| 46 | 76.8 |
| 47 | 135 |
| 53 | 16.7 |
| 54 | 19.7 |
| 55 | 36.8 |
| 56 | 11.3 |
| 57 | 120 |
| 58 | 51.6 |
| 60 | 108 |
| 62 | 26.5 |
| 63 | 47.8 |
| 64 | 195 |
| 65 | 24.6 |
| 67 | 16.1 |
| 68 | 13.3 |
| 69 | 17.7 |
| 70 | 29.6 |
| 72 | 54.5 |
| 73 | 21.5 |

### Pharmacological Test Example 4: Cell growth inhibitory activities on cultured human leukemia cells (MOLM-13)

Human leukemia cells MOLM13 (subdivided from Hayashibara Biochemical Laboratories, Inc.) were seeded in an amount of 3 × 10³ using a DMEM medium containing 10% fetal calf serum (purchased from GIBCO) in a 96-well flat bottom plate. A solution of a test compound in dimethyl sulfoxide was added to each well and was cultured in a 5% carbon dioxide incubator for 3 days. Three days after the initiation of the culture, 10 µl of a WST-1 solution in Cell Counting Kit (purchased from Wako Pure Chemical Industries, Ltd.) was added to each well for a color reaction. After proper color development, the absorbance was measured with a microplate reader under conditions of measurement wavelength 450 nm and reference wavelength 650 nm. The growth ratio for each well was determined by presuming the absorbance without the addition of the medicament to be 100% growth ratio and the absorbance of the well without seeding of the cells to be 0% growth ratio. The concentration of the test compound was varied on several levels, the cell growth ratio for each case was determined, and the concentration of the test compound necessary for inhibiting 50% of cell growth ratio (IC₅₀) was calculated. The results were as shown in Table 4.

**Table 4**

| IC₅₀(nM) | | IC₅₀(nM) | |
|---|---|---|---|
| Compound No. | MOLM13 | Compound No. | MOLM13 |
| 1 | 1.0 | 40 | 2.0 |
| 2 | 770.0 | 41 | 75.0 |
| 3 | 3.0 | 42 | 273.0 |
| 4 | 653,0 | 43 | 22.0 |
| 5 | 36.0 | 44 | 3.0 |
| 6 | 21.0 | 45 | 140.0 |
| 7 | 593.0 | 46 | 123.0 |
| 8 | 3.0 | 47 | 60.0 |
| 9 | 24.0 | 38 | 222.0 |
| 10 | 2.0 | 49 | 224.0 |
| 11 | 416.0 | 50 | 243.0 |
| 12 | 6.0 | 51 | 216.0 |
| 13 | 21.0 | 52 | 188.0 |
| 14 | 3.0 | 53 | 13.0 |
| 15 | 22.0 | 54 | 10.0 |
| 16 | 7.0 | 55 | 25.0 |
| 17 | 27.0 | 56 | 26.0 |
| 18 | 2.0 | 57 | 29.0 |
| 19 | 10.0 | 58 | 17.0 |
| 20 | 39.0 | 59 | 4.0 |
| 21 | 22.0 | 60 | 59.0 |
| 22 | 7.0 | 61 | 138.0 |
| 23 | 4.0 | 62 | 7.0 |
| 24 | 24.0 | 63 | 22.0 |
| 25 | 258.0 | 64 | 19.0 |
| 26 | 18.0 | 65 | 9.0 |
| 27 | 45.0 | 66 | <1 |
| 28 | 26.0 | 67 | 4.0 |
| 29 | 3.0 | 68 | 1.0 |
| 30 | 8.0 | 69 | 8.0 |
| 31 | 2.0 | 70 | 3.0 |
| 32 | 314.0 | 71 | 27.0 |
| 33 | 4.0 | 72 | 8.0 |
| 34 | 2.0 | 73 | 20.0 |
| 35 | 3.0 | 74 | 244.0 |
| 36 | 2.0 | 75 | <1 |
| 37 | 38.0 | | |
| 39 | 3.0 | | |

### Pharmacological Test Example 5: Tumor growth inhibitory activities in nude mice xenograft model inoculated with human leukemia cells (MOLM13)

Human leukemia cells MOLM13 (subdivided from Hayashibara Biochemical Laboratories, Inc.) were subcutaneously transplanted into nude mice. When the tumor volume became about 100 mm³, the mice were grouped so that the groups each consisted of four mice and had an even average tumor volume. The test compound suspended or dissolved in 0.5% methylcellulose was orally administered at a dose of 0.1 ml per 10 g body weight once a day for 9 days. For the control group, only vehicle was administered.

The tumor growth inhibition rate (TGIR) was calculated as follows:

The tumor growth inhibition rate (TGIR) = (1 - TX/CX) × 100 wherein CX represents the volume of tumor at day X for the control group when the tumor volume at the day of the start of the administration (day 1) was presumed to be 1; and TX represents the volume of tumor at day X for test compound administration groups. The results are shown in Table 5.

**Table 5**

| Compound No. | Dose | TGIR (%) (measurement date) |
|---|---|---|
| 1 | 50 mg/kg | 53.8% (day 11) |
| 10 | 50 mg/kg | 84.8% (day 10) |
| 12 | 50 mg/kg | 93.7% (day 10) |
| 13 | 50 mg/kg | 84.8% (day 10) |
| 14 | 50 mg/kg | 96.1% (day 10) |
| 15 | 50 mg/kg | 89.4% (day 10) |
| 43 | 50 mg/kg | 98.1% (day 10) |
| 44 | 25 mg/kg | 99.8% (day 9) |
| 44 | 12.5 mg/kg | 99.6% (day 9) |
| 75 | 50 mg/kg | 94.1% (day 9) |
| 76 | 50 mg/kg | 34.3% (day 9) |
| 77 | 50 mg/kg | 70.4% (day 9) |

## Claims

1. A pharmaceutical composition for use in the treatment or prevention of diseases where the inhibition of autophosphorylation of FMS-like tyrosine kinase 3 (Flt3) and/or its somatic cell variant (Flt3-ITD) is therapeutically or prophylactically effective, which comprises a compound represented by formula (I) or a pharmaceutically acceptable salt or solvate thereof: 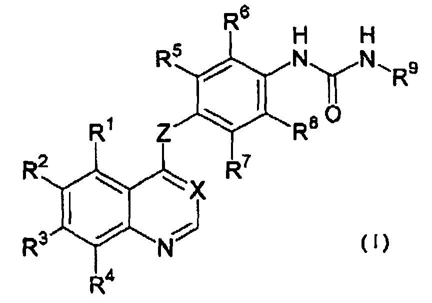 wherein
X represents CH or N,
Z represents O or S,
R¹, R², and R³, which may be the same or different, represent
a hydrogen atom,
hydroxyl,
a halogen atom,
nitro,
cyano,
amino,
C₁₋₆ alkyl,
C₂₋₆ alkenyl,
C₂₋₆ alkynyl,
C₁₋₆ alkoxy,
-(C=O)OR^{c} wherein R^{c} represents a hydrogen atom or C₁₋₄ alkyl, or
-(C=O)NR^{d}R^{e} wherein R^{d} and R^{e}, which may be the same or different, represent a hydrogen atom or C₁₋₄ alkyl,
the C₁₋₆ alkyl, C₂₋₆ alkenyl, C₂₋₆ alkynyl, and C₁₋₆ alkoxy groups, which may be represented by R¹, R², and R³, are optionally substituted by hydroxyl; a halogen atom; C₁₋₆ alkoxy; C₁₋₆ alkylcarbonyl; carboxyl; C₁₋₆ alkoxycarbonyl; -(C=O)-NR¹⁰R¹¹ wherein R¹⁰ and R¹¹, which may be the same or different, represent a hydrogen atom or C₁₋₄ alkyl optionally substituted by hydroxyl, or R¹⁰ and R¹¹ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, a halogen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic group;
one or two hydrogen atoms on the amino group, which may be represented by R¹, R², and R³, are optionally substituted by C₁₋₆ alkyl which is further optionally substituted by hydroxyl or C₁₋₆ alkoxy;
R⁴ represents a hydrogen atom;
all of R⁵, R⁶, R⁷, and R⁸ represent a hydrogen atom, or any one or two of R⁵, R⁶, R⁷, and R⁸ represent a halogen atom, C₁₋₄ alkyl, C₁₋₄ alkoxy, nitro, amino, or hydroxyl with all the remaining groups representing a hydrogen atom, and
R⁹ represents C₁₋₄ alkyl substituted by a substituent selected from the group consisting of a saturated three- to nine-membered carbocyclic group optionally substituted by C₁₋₄ alkyl, C₁₋₄ alkoxy, or hydroxyl; i-propyl optionally substituted by C₁₋₄ alkyl, C₁₋₄ alkoxy, or hydroxyl; t-butyl optionally substituted by C₁₋₄ alkyl, C₁₋₄ alkoxy, or hydroxyl; C₁₋₄ alkoxy; and -NR^{a}R^{b} wherein R^{a} and R^{b}, which may be the same or different, represent a hydrogen atom or C₁₋₄ alkyl optionally substituted by hydroxyl, or R^{a} and R^{b} may combine with a nitrogen atom attached thereto to form a saturated five- or six-membered heterocyclic group, or R⁹ represents a saturated three- to nine-membered carbocyclic group optionally substituted by one to three C₁₋₄ alkyl groups.

2. The pharmaceutical composition according to claim 1, wherein the disease where the inhibition of autophosphorylation of Flt3 and/or Flt3-ITD is therapeutically or prophylactically effective is hematopoietic malignancy.

3. The pharmaceutical composition according to claim 2, wherein the hematopoietic malignancy is acute myelocytic leukemia or myelodysplastic syndrome.

4. The pharmaceutical composition according to claim 1, wherein the disease where the inhibition of autophosphorylation of Flt3 and/or Flt3-ITD is therapeutically or prophylactically effective is an immunological disease caused by abnormal proliferation of B cells, dendritic cells, or natural killer cells.

5. The pharmaceutical composition according to claim 1, which is used in the treatment or prevention of diseases where the inhibition of autophosphorylation of Flt3 is therapeutically or prophylactically effective.

6. The pharmaceutical composition according to claim 5, wherein the disease where the inhibition of autophosphorylation of Flt3 is therapeutically or prophylactically effective is hematopoietic malignancy.

7. The pharmaceutical composition according to claim 6, wherein the hematopoietic malignancy is acute myelocytic leukemia or myelodysplastic syndrome.

8. The pharmaceutical composition according to claim 5, wherein the disease where the inhibition of autophosphorylation of Flt3 is therapeutically or prophylactically effective is an immunological disease caused by abnormal proliferation of B cells, dendritic cells, or natural killer cells.

9. The pharmaceutical composition according to claim 1, which is used in the treatment or prevention of diseases where the inhibition of autophosphorylation of Flt3-ITD is therapeutically or prophylactically effective.

10. The pharmaceutical composition according to claim 9, wherein the disease where the inhibition of autophosphorylation of Flt3-ITD is therapeutically or prophylactically effective is hematopoietic malignancy.

11. The pharmaceutical composition according to claim 10, wherein the hematopoietic malignancy is acute myelocytic leukemia or myelodysplastic syndrome.

12. The pharmaceutical composition according to claim 9, wherein the disease where the inhibition of autophosphorylation of Flt3-ITD is therapeutically or prophylactically effective is an immunological disease caused by abnormal proliferation of B cells, dendritic cells, or natural killer cells.

13. The pharmaceutical composition according to any one of claims 1 to 12, wherein X represents CH and Z represents O.

14. The pharmaceutical composition according to any one of claims 1 to 13, wherein R¹ represents a hydrogen atom and R² and R³, which may be the same or different, represent optionally substituted C₁₋₆ alkoxy.

15. The pharmaceutical composition according to any one of claims 1 to 14, wherein R¹ represents a hydrogen atom, R² and R³, which may be the same or different, represent -O-(CH₂)p-R¹² wherein p is an integer of 0 to 6, -(CH₂)p- is optionally substituted by C₁₋₆ alkyl, hydroxyl, or a halogen atom, and R¹² represents a hydrogen atom; hydroxyl; a halogen atom; C₁₋₆ alkoxy; C₁₋₆ alkylcarbonyl; carboxyl; C₁₋₆ alkoxycarbonyl; -(C=O)-NR¹³R¹⁴ wherein R¹³ and R¹⁴, which may be the same or different, represent a hydrogen atom or C₁₋₄ alkyl optionally substituted by hydroxyl, or R¹³ and R¹⁴ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic ring.

16. The pharmaceutical composition according to any one of claims 1 to 15, wherein all of R⁵, R⁶, R⁷, and R⁸ represent a hydrogen atom; or R⁶ represents a fluorine atom, and R⁵, R⁷, and R⁸ represent a hydrogen atom; or R⁵ represents a halogen atom, C₁₋₄ alkyl, C₁₋₄ alkoxy, nitro, or amino, and R⁶, R⁷, and R⁸ represent a hydrogen atom; or R⁵ and R⁷ represent a halogen atom, C₁₋₄ alkyl, C₁₋₄ alkoxy, nitro, or amino, and R⁶ and R⁸ represent a hydrogen atom.

17. The pharmaceutical composition according to any one of claims 1 to 16, wherein R⁹ represents -(CH₂)s-R⁵¹ wherein s is an integer of 1 to 4, and R⁵¹ represents a saturated three- to seven-membered carbocyclic group; i-propyl optionally substituted by hydroxyl; t-butyl optionally substituted by hydroxyl; C₁₋₄ alkoxy; or -NR⁵²R⁵³ wherein R⁵² and R⁵³, which may be the same or different, represent a hydrogen atom, or C₁₋₄ alkyl optionally substituted by hydroxyl, or R⁵² and R⁵³ may combine with a nitrogen atom attached thereto to form a saturated five- or six-membered heterocyclic group, or R⁹ represents a saturated five- to seven-membered carbocyclic group optionally substituted by one to three C₁₋₄ alkyl groups.

18. The pharmaceutical composition according to claim 1, wherein
X represents CH or N,
Z represents O or S,
R¹, R², and R³, which may be the same or different, represent
a hydrogen atom,
hydroxyl,
a halogen atom,
nitro,
amino,
C₁₋₆ alkyl,
C₂₋₆ alkenyl,
C₂₋₆ alkynyl, or
C₁₋₆ alkoxy,
the C₁₋₆ alkyl, C₂₋₆ alkenyl, C₂₋₆ alkynyl, and C₁₋₆ alkoxy groups, which may be represented by R¹, R², and R³, are optionally substituted by hydroxyl; a halogen atom; C₁₋₆ alkoxy; C₁₋₆ alkylcarbonyl; carboxyl; C₁₋₆ alkoxycarbonyl; -(C=O)-NR¹⁰R¹¹ wherein R¹⁰ and R¹¹, which may be the same or different, represent a hydrogen atom or C₁₋₄ alkyl optionally substituted by hydroxyl, or R¹⁰ and R¹¹ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic ring;
one or two hydrogen atoms on the amino group, which may be represented by R¹, R², and R³, are optionally substituted by C₁₋₆ alkyl which is further optionally substituted by hydroxyl or C₁₋₆ alkoxy;
R⁴ represents a hydrogen atom;
all of R⁵, R⁶, R⁷, and R⁸ represent a hydrogen atom, or any one or two of R⁵, R⁶, R⁷, and R⁸ represent a halogen atom, C₁₋₄ alkyl, C₁₋₄ alkoxy, nitro, or amino with all the remaining groups representing a hydrogen atom, and
R⁹ represents C₁₋₄ alkyl substituted by a substituent selected from the group consisting of a saturated three- to seven-membered carbocyclic group; i-propyl optionally substituted by hydroxyl; t-butyl optionally substituted by hydroxyl; C₁₋₄ alkoxy; and -NR^{a}R^{b} wherein R^{a} and R^{b}, which may be the same or different, represent a hydrogen atom or C₁₋₄ alkyl optionally substituted by hydroxyl, or R^{a} and R^{b} may combine with a nitrogen atom attached thereto to form a saturated five- or six-membered heterocyclic group, or R⁹ represents a saturated five- to seven-membered carbocyclic group optionally substituted by one to three C₁₋₄ alkyl groups.

19. The pharmaceutical composition according to claim 1, wherein said compound represented by formula (I) is represented by formula (Ia): 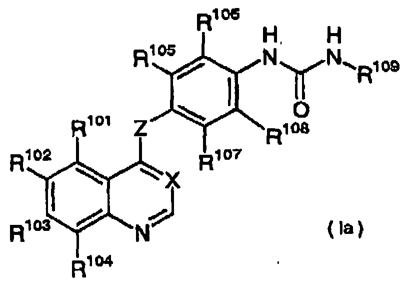 wherein
X represents CH or N,
Z represents O or S,
R¹⁰¹ and R¹⁰⁴ represent a hydrogen atom,
R¹⁰² and R¹⁰³, which may be the same or different, represent
a hydrogen atom,
hydroxyl,
a halogen atom,
nitro,
cyano,
-NR¹¹¹R¹¹² wherein R¹¹¹ and R¹¹², which may be the same or different, represent a hydrogen atom or C₁₋₄ alkyl,
-(C=O)OR¹¹³ wherein R¹¹³ represents a hydrogen atom or C₁₋₄ alkyl,
-(C=O)NR¹¹⁴R¹¹⁵ wherein R¹¹⁴ and R¹¹⁵, which may be the same or different, represent a hydrogen atom or C₁₋₄ alkyl,
C₁₋₆ alkoxy,
C₁₋₆ alkyl,
C₁₋₆ alkenyl, or
C₁₋₆ alkynyl,
the C₁₋₆ alkoxy, C₁₋₆ alkyl, C₁₋₆ alkenyl, or C₁₋₆ alkynyl are optionally substituted by hydroxyl; a halogen atom; C₁₋₄ alkoxy; -NR¹¹⁶R¹¹⁷ wherein R¹¹⁶ and R¹¹⁷, which may be the same or different, represent a hydrogen atom or C₁₋₄ alkyl and the alkyl group is further optionally substituted by hydroxyl or C₁₋₄ alkoxy; or a saturated or unsaturated three- to eight-membered carbocylic or heterocyclic group in which the cyclic group is optionally substituted by hydroxyl, a halogen atom, C₁₋₄ alkyl, or C₁₋₄ alkoxy,
all of R¹⁰⁵, R¹⁰⁶, R¹⁰⁷, and R¹⁰⁸ represent a hydrogen atom, or any one or two of R¹⁰⁵, R¹⁰⁶, R¹⁰⁷, and R¹⁰⁸ represent hydroxyl, C₁₋₄ alkyl, C₁₋₄ alkoxy, amino, nitro, or a halogen atom with all the remaining groups representing a hydrogen atom,
R¹⁰⁹ represents -(CH₂)n-R¹¹⁰ wherein n is 2, 3, or 4, and R¹¹⁰ represents i-propyl optionally substituted by C₁₋₄ alkyl, C₁₋₄ alkoxy, or hydroxyl; t-butyl optionally substituted by C₁₋₄ alkyl, C₁₋₄ alkoxy, or hydroxyl; or a three- to nine-membered saturated carbocyclic group optionally substituted by C₁₋₄ alkyl, C₁₋₄ alkoxy, or hydroxyl.

20. The pharmaceutical composition according to claim 19, wherein R¹⁰² and R¹⁰³, which may be the same or different, represent C₁₋₆ alkoxy and the C₁₋₆ alkoxy is optionally substituted by hydroxyl; a halogen atom; C₁₋₄ alkoxy; -NR¹¹⁶R¹¹⁷ wherein R¹¹⁶ and R¹¹⁷, which may be the same or different, represent a hydrogen atom or C₁₋₄ alkyl and the alkyl group is further optionally substituted by hydroxyl or C₁₋₄ alkoxy; or a saturated or unsaturated three- to eight-membered carbocylic or heterocyclic group in which the cyclic group is optionally substituted by hydroxyl, halogen atom, C₁₋₄ alkyl, or C₁₋₄ alkoxy.

21. The pharmaceutical composition according to claim 20, wherein R¹⁰² and R¹⁰³, which may be the same or different, represent C₁₋₆ alkoxy in which the alkoxy group is optionally substituted by a saturated or unsaturated three- to eight-membered carbocylic or heterocyclic group and the cyclic group is further optionally substituted by hydroxyl, a halogen atom, C₁₋₄ alkyl, or C₁₋₄ alkoxy.

22. The pharmaceutical composition according to claim 21, wherein R¹⁰² and R¹⁰³, which may be the same or different, represent C₁₋₄ alkoxy in which the alkoxy group is optionally substituted by a saturated five- to seven-membered heterocyclic group and the cyclic group is further optionally substituted by C₁₋₄ alkyl.

23. The pharmaceutical composition according to claim 22, wherein said substituted C₁₋₄ alkoxy group is a group represented by

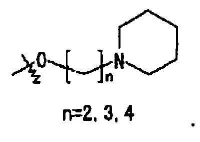

24. The pharmaceutical composition according to claim 23, wherein n is 2.

25. The pharmaceutical composition according to claim 22, wherein said substituted C₁₋₄ alkoxy group is a group represented by

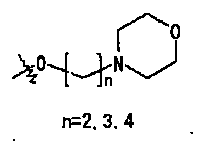

26. The pharmaceutical composition according to claim 25, wherein n is 2.

27. The pharmaceutical composition according to any one of claims 19 to 26, wherein one of R¹⁰² and R¹⁰³ represents unsubstituted C₁₋₆ alkoxy and the other represents substituted C₁₋₆ alkoxy.

28. The pharmaceutical composition according to claim 27, wherein R¹⁰² represents unsubstituted C₁₋₆ alkoxy and R¹⁰³ represents substituted C₁₋₆ alkoxy.

29. The pharmaceutical composition according to claim 28, wherein R¹⁰² represents methoxy.

30. The pharmaceutical composition according to any one of claims 19 to 29, wherein X represents CH.

31. The pharmaceutical composition according to any one of claims 19 to 30, wherein Z represents O.

32. The pharmaceutical composition according to any one of claims 19 to 31, wherein all of R¹⁰⁵, R¹⁰⁶, R¹⁰⁷, and R¹⁰⁸ represent a hydrogen atom, or any one or two of R¹⁰⁵, R¹⁰⁶, R¹⁰⁷, and R¹⁰⁸ represent C₁₋₄ alkyl, C₁₋₄ alkoxy, or a halogen atom with all the remaining groups representing a hydrogen atom.

33. The pharmaceutical composition according to claim 32, wherein R¹⁰⁵ represents methoxy and R¹⁰⁶, R¹⁰⁷, and R¹⁰⁸ represent a hydrogen atom.

34. The pharmaceutical composition according to claim 32, wherein R¹⁰⁵ represents methyl and R¹⁰⁶, R¹⁰⁷, and R¹⁰⁸ represent a hydrogen atom.

35. The pharmaceutical composition according to claim 32, wherein R¹⁰⁵ represents a halogen atom and R¹⁰⁶, R¹⁰⁷, and R¹⁰⁸ represent a hydrogen atom.

36. The pharmaceutical composition according to claim 35, wherein the halogen atom represents a chlorine or fluorine atom.

37. The pharmaceutical composition according to claim 35, wherein the halogen atom represents a fluorine atom.

38. The pharmaceutical composition according to claim 32, wherein all of R¹⁰⁵, R¹⁰⁶, R¹⁰⁷, and R¹⁰⁸ represent a hydrogen atom.

39. The pharmaceutical composition according to any one of claims 19 to 38, wherein R¹⁰⁹ is a group represented by

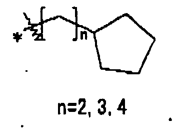

40. The pharmaceutical composition according to claim 39, wherein n is 2.

41. The pharmaceutical composition according to any one of claims19 to 38, wherein R¹⁰⁹ is a group represented by

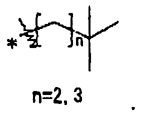

42. The pharmaceutical composition according to claim 41, wherein n is 2.

43. The pharmaceutical composition according to claim 19, wherein the compound represented by formula (Ia) is 1-(3,3-dimethylbutyl)-3-{3-fluoro-4-[6-methoxy-7-(2-piperidin-1-yl-ethoxy)-quinolin-4-yloxy]-phenyl}-urea.

44. The pharmaceutical composition according to claim 19, wherein the compound represented by formula (Ia) is 1-(2-cyclopentylethyl)-3-{3-fluoro-4-[6-methoxy-7-(2-piperidin-1-yl-ethoxy)-quinolin-4-yloxy]-phenyl}-urea.

45. The pharmaceutical composition according to claim 19, wherein the compound represented by formula (Ia) is 1-(2-cyclopentylethyl)-3-{2-fluoro-4-[6-methoxy-7-(2-piperidin-1-yl-ethoxy)-quinolin-4-yloxy]-phenyl}-urea.

46. The pharmaceutical composition according to claim 1, wherein the compound represented by formula (I) is represented by formula (II): 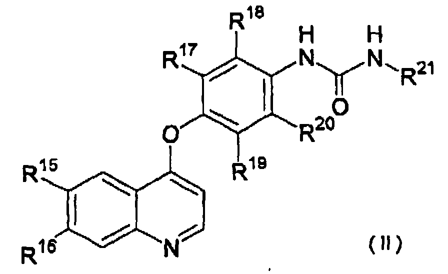 wherein
R¹⁵ and R¹⁶, which may be the same or different, represent -O-(CH₂)r-R²² wherein r is an integer of 0 to 6, -(CH₂)r- is optionally substituted by C₁₋₆ alkyl, hydroxyl, or a halogen atom, and R²² represents a hydrogen atom; hydroxyl; a halogen atom; C₁₋₆ alkoxy; C₁₋₆ alkylcarbonyl; carboxyl; C₁₋₆ alkoxycarbonyl; -(C=O)-NR²³R²⁴ wherein R²³ and R²⁴, which may be the same or different, represent a hydrogen atom or C₁₋₄ alkyl optionally substituted by hydroxyl, or R²³ and R²⁴ may combine with a nitrogen atom attached thereto to form a saturated five- or six-membered heterocyclic group; amino in which one or two hydrogen atoms on the amino group are optionally substituted by C₁₋₆ alkyl or a saturated or unsaturated three- to eight-membered carbocyclic or heterocyclic group, and the C₁₋₆ alkyl group is further optionally substituted by hydroxyl, C₁₋₆ alkoxy, or a saturated or unsaturated three- to eight-membered carbocyclic or heterocyclic group; or a saturated or unsaturated three- to eight-membered carbocyclic or heterocyclic group in which the carbocyclic or heterocyclic group is optionally substituted by hydroxyl, an oxygen atom, C₁₋₆ alkyl, C₂₋₆ alkenyl, C₂₋₆ alkynyl, C₁₋₆ alkoxy, C₁₋₆ alkoxycarbonyl, or a saturated or unsaturated three- to eight-membered carbocyclic or heterocyclic group, the C₁₋₆ alkyl, C₂₋₆ alkenyl, and C₂₋₆ alkynyl groups are further optionally substituted by hydroxyl, C₁₋₆ alkoxy, or a saturated or unsaturated three- to eight-membered carbocyclic or heterocyclic group, and, when the carbocyclic or heterocyclic group is substituted by two C₁₋₆ alkyl groups, the two alkyl groups may combine together to form an alkylene chain, or the carbocyclic or heterocyclic group may be a bicyclic group condensed with another saturated or unsaturated five- to seven-membered carbocyclic or heterocyclic ring,
all of R¹⁷, R¹⁸, R¹⁹, and R²⁰ represent a hydrogen atom, or any one or two of R¹⁷, R¹⁸, R¹⁹, and R²⁰ represent a halogen atom, C₁₋₄ alkyl, C₁₋₄ alkoxy, nitro, or amino with all the remaining groups representing a hydrogen atom, and
R²¹ represents -(CH₂)t-R⁶¹ wherein t is an integer of 1 to 4 and R⁶¹ represents a saturated three- to seven-membered carbocyclic group; i-propyl optionally substituted by hydroxyl; t-butyl optionally substituted by hydroxyl; C₁₋₄ alkoxy; or -NR⁶²R⁶³ wherein R⁶² and R⁶³, which may be the same or different, represent a hydrogen atom, or C₁₋₄ alkyl optionally substituted by hydroxyl, or R⁶² and R⁶³ may combine with a nitrogen atom attached thereto to form a saturated five- or six-membered heterocyclic group, or R²¹ represents a saturated five- to seven-membered carbocyclic group optionally substituted by one to three C₁₋₄ alkyl groups.

47. The pharmaceutical composition according to claim 46, wherein R¹⁵ and R¹⁶ represent -O-(CH₂)r-H wherein r is an integer of 1 to 4 and the -(CH₂)r- part is unsubstituted, or any one of R¹⁵ and R¹⁶ repersents -O-(CH₂)r-H wherein r is an integer of 1 to 4 and the -(CH₂)r- part is unsubstituted with the other representing -O-(CH₂)r-R²² wherein r is an integer of 1 to 4, the -(CH₂)r- part is unsubstituted, and R²² represents optionally substituted amino or an optionally substituted saturated three- to eight-membered heterocyclic group,
all of R¹⁷, R¹⁸, R¹⁹, and R²⁰ represent a hydrogen atom, or any one or two of R¹⁷, R¹⁸, R¹⁹, and R²⁰ represent a halogen atom, C₁₋₄ alkyl, C₁₋₄ alkoxy, nitro, or amino with all the remaining groups representing a hydrogen atom, and
R²¹ represents -(CH₂)t-R⁶¹, wherein t is an integer of 1 to 4 and R⁶¹ represents a saturated five- to seven-membered carbocyclic group; i-propyl; t-butyl optionally substituted by hydroxyl; C₁₋₄ alkoxy; or -NR⁶²R⁶³ wherein R⁶² and R⁶³, which may be the same or different, represent C₁₋₄ alkyl, or R²¹ represents a five- to seven-membered carbocyclic group optionally substituted by 1 to 3 C₁₋₄ alkyl groups.

48. The pharmaceutical composition according to claim 46, wherein R¹⁵ and R¹⁶ represent -O-(CH₂)r-H wherein r is an integer of 1 to 4 and the -(CH₂)r- part is unsubstituted, or any one of R¹⁵ and R¹⁶ represents -O-(CH₂)r-H wherein r is an integer of 1 to 4 and the -(CH₂)r- part is unsubstituted with the other representing -O-(CH₂)r-R²² wherein r is an integer of 1 to 4, the -(CH₂)r- part is unsubstituted, and R²² represents optionally substituted amino or an optionally substituted saturated three- to eight-membered heterocyclic group,
all of R¹⁷, R¹⁸, R¹⁹, and R²⁰ represent a hydrogen atom; or R¹⁸ represents a fluorine atom, and R¹⁷, R¹⁹, and R²⁰ represent a hydrogen atom; or R¹⁷ represents a halogen atom, C₁₋₄ alkyl, or C₁₋₄ alkoxy, and R¹⁸, R¹⁹, and R²⁰ represent a hydrogen atom; or R¹⁷ and R¹⁹ represent a halogen atom, C₁₋₄ alkyl, or C₁₋₄ alkoxy, and R¹⁸ and R²⁰ represent a hydrogen atom, and
R²¹ represents -(CH₂)t-R⁶¹, wherein t is an integer of 2 or 3 and R⁶¹ represents a saturated five- to seven-membered carbocyclic group or t-butyl, or R²¹ represents a five- to seven-membered carbocyclic group optionally substituted by one to three C₁₋₄ alkyl groups.

49. A method for treating or preventing a disease where the inhibition of autophosphorylation of Flt3 and/or Flt3-ITD is therapeutically or prophylactically effective, which comprises the step of administering a compound or a pharmaceutically acceptable salt or solvate thereof according to any one of claims 1 to 48 together with a pharmaceutically acceptable carrier, to a mammal.

50. Use of a compound or a pharmaceutically acceptable salt or solvate thereof according to any one of claims 1 to 48, for the manufacture of a medicament used in the treatment or prevention of diseases where the inhibition of autophosphorylation of Flt3 and/or Flt3-ITD is therapeutically or prophylactically effective.

51. A compound represented by formula (Ia) or a pharmaceutically acceptable salt or solvate thereof: 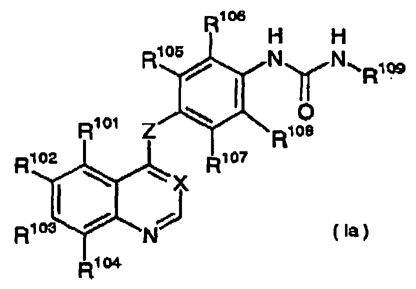 wherein X, Z, R¹⁰¹, R¹⁰², R¹⁰³, R¹⁰⁴, R¹⁰⁵, R¹⁰⁶, R¹⁰⁷, R¹⁰⁸, and R¹⁰⁹ are as defined in claim 19.

52. A compound represented by formula (II) or a pharmaceutically acceptable salt or solvate thereof: 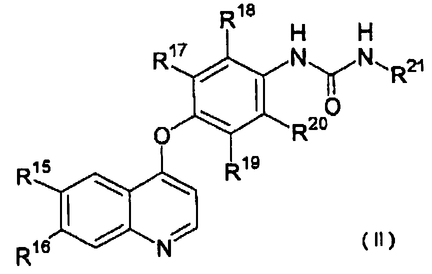 wherein R¹⁵, R¹⁶, R¹⁷, R¹⁸, R¹⁹, R²⁰, and R²¹ are as defined in claim 46.

53. A pharmaceutical composition comprising a compound according to claim 51 or 52 or a pharmaceutically acceptable salt or solvate thereof.
